# EUROPEAN PATENT APPLICATION

(11) **EP 2 711 018 A1**
(43) Date of publication of application: **26.03.2014**
(21) Application number: 13182099.5
(22) Date of filing: 21.06.2010
(51) Int. Cl.: A61K 39/00, C12P 21/08, A61K 47/48, C07K 16/00

(54) **Engineered Fc regions for site-specific conjugation**

(30) Priority: 22.06.2009 US 219225 P
(62) Divisional of application: 10797547.6
(71) Applicant: MedImmune, LLC, Gaithersburg, MD 20878 (US)
(72) Inventor: Dimasi, Nazzareno, Gaithersburg, MD 20878 (US); Gao, Changshou, Potomac, MD 20854 (US)
(74) Representative: Winter, Christopher Spencer

(57) **Abstract**

Fc regions useful for site-specific conjugation to a variety of agents are provided. Methods for the design, preparation, screening, selection and use of such Fc regions are also provided.

## Description

### 1. CROSS REFERENCE TO RELATED APPLICATIONS

This application claims priority to U.S. Provisional Application No. 61/219,225, filed June 22, 2009, which is incorporated by reference in its entirety.

### 2. REFERENCE TO THE SEQUENCE LISTING

This application incorporates by reference a Sequence Listing submitted with this application as text file entitled "MED0455.PCT_ST25" created on June 14, 2010 and having a size of 69 kilobytes.

### 3. FIELD OF THE DISCLOSURE

The disclosure relates to Fc regions which result in groups for conjugation reactions. Also provided are methods of design, modification, production, and use of such Fc regions.

### 4. BACKGROUND OF THE DISCLOSURE

### 4.1 CANCER AND CANCER THERAPIES

More than 1.2 million Americans develop cancer each year. Cancer is the second leading case of death in the United States and if current trends continue, cancer is expected to be the leading cause of the death by the year 2010. Lung and prostate cancer are the top cancer killers for men in the United States. Lung and breast cancer are the top cancer killers for women in the United States. One in two men in the United States will be diagnosed with cancer at some time during his lifetime. One in three women in the United States will be diagnosed with cancer at some time during her lifetime. Current treatment options, such as surgery, chemotherapy and radiation treatment, are often either ineffective or present serious side effects.

One barrier to the development of anti-metastasis agents has been the assay systems that are used to design and evaluate these drugs. Most conventional cancer therapies target rapidly growing cells. However, cancer cells do not necessarily grow more rapidly but instead survive and grow under conditions that are non-permissive to normal cells (Lawrence and Steeg, 1996, World J. Urol. 14:124-130). These fundamental differences between the behaviors of normal and malignant cells provide opportunities for therapeutic targeting. The paradigm that micrometastatic tumors have already disseminated throughout the body emphasizes the need to evaluate potential chemotherapeutic drugs in the context of a foreign and three-dimensional microenvironment. Many standard cancer drug assays measure tumor cell growth or survival under typical cell culture conditions (i.e., monolayer growth). However, cell behavior in two-dimensional assays often does not reliably predict tumor cell behavior in vivo.

Currently, cancer therapy may involve surgery, chemotherapy, hormonal therapy and/or radiation treatment to eradicate neoplastic cells in a patient (see, for example, Stockdale, 1998, "Principles of Cancer Patient Management", in Scientific American: Medicine, vol. 3, Rubenstein and Federman, eds., Chapter 12, Section IV). All of these approaches pose significant drawbacks for the patient. Surgery, for example, may be contraindicated due to the health of the patient or may be unacceptable to the patient. Additionally, surgery may not completely remove the neoplastic tissue. Radiation therapy is only effective when the neoplastic tissue exhibits a higher sensitivity to radiation than normal tissue, and radiation therapy can also often elicit serious side effects. Hormonal therapy is rarely given as a single agent and although can be effective, is often used to prevent or delay recurrence of cancer after other treatments have removed the majority of the cancer cells.

With respect to chemotherapy, there are a variety of chemotherapeutic agents available for treatment of cancer. A significant majority of cancer chemotherapeutics act by inhibiting DNA synthesis (see, for example, Gilman et al., Goodman and Gilman's: The Pharmacological Basis of Therapeutics, Eighth Ed. (Pergamon Press, New York, 1990)). As such, chemotherapy agents are inherently nonspecific. In addition almost all chemotherapeutic agents are toxic, and chemotherapy causes significant, and often dangerous, side effects, including severe nausea, bone marrow depression, immunosuppression, etc. (see, for example, Stockdale, 1998, "Principles Of Cancer Patient Management" in Scientific American Medicine, vol. 3, Rubenstein and Federman, eds., ch. 12, sect. 10). Furthermore, even with administration of combinations of chemotherapeutic agents, many tumor cells are resistant or develop resistance to the chemotherapeutic agents.

Cancer therapy can now also involve biological therapy or immunotherapy. Biological therapies/immunotherapies are limited in number and although more specific then chemotherapeutic agents many still target both healthy and cancerous cells. In addition, such therapies may produce side effects such as rashes or swellings, flu-like symptoms, including fever, chills and fatigue, digestive tract problems or allergic reactions.

### 4.2 ANTIBODIES FOR THE TREATMENT OF CANCER

Antibodies are immunological proteins that bind a specific antigen. In most mammals, including humans and mice, antibodies are constructed from paired heavy and light polypeptide chains. Each chain is made up of two distinct regions, referred to as the variable (Fv) and constant (Fc) regions. The light and heavy chain Fv regions contain the antigen binding determinants of the molecule and are responsible for binding the target antigen. The Fc regions define the class (or isotype) of antibody (IgG for example) and are responsible for binding a number of natural proteins to elicit important biochemical events.

The Fc region of an antibody interacts with a number of ligands including Fc receptors and other ligands, imparting an array of important functional capabilities referred to as effector functions. An important family of Fc receptors for the IgG class are the Fc gamma receptors (FcγRs). These receptors mediate communication between antibodies and the cellular arm of the immune system (Raghavan et al., 1996, Annu Rev Cell Dev Biol 12:181-220; Ravetch et al., 2001, Annu Rev Immunol 19:275-290). In humans this protein family includes FcγRI (CID64), including isoforms FcγRIA, FcγRIB, and FcγRIC; FcγRII (CD32), including isoforms FcγRIIA, FcγRIIB, and FcγRIIC; and FcγRIII (CD16), including isoforms FcγRIIIA and FcγRIIB (Jefferis et al., 2002, Immunol Lett 82:57-65). These receptors typically have an extracellular domain that mediates binding to Fc, a membrane spanning region, and an intracellular domain that may mediate some signaling event within the cell. These different FcγR subtypes are expressed on different cell types (reviewed in Ravetch et al., 1991, Annu Rev Immunol 9:457-492). For example, in humans, FcγRIIIB is found only on neutrophils, whereas FcγRIIIA is found on macrophages, monocytes, natural killer (NK) cells, and a subpopulation of T-cells.

Formation of the Fc/FcγR complex recruits effector cells to sites of bound antigen, typically resulting in signaling events within the cells and important subsequent immune responses such as release of inflammation mediators, B cell activation, endocytosis, phagocytosis, and cytotoxic attack. The ability to mediate cytotoxic and phagocytic effector functions is a potential mechanism by which antibodies destroy targeted cells. The cell-mediated reaction wherein nonspecific cytotoxic cells that express FcγRs recognize bound antibody on a target cell and subsequently cause lysis of the target cell is referred to as antibody dependent cell-mediated cytotoxicity (ADCC) (Raghavan et al., 1996, Annu Rev Cell Dev Biol 12:181-220; Ghetie et al., 2000, Annu Rev Immunol 18:739-766; Ravetch et al., 2001, Annu Rev Immunol 19:275-290). Notably, the primary cells for mediating ADCC, NK cells, express only FcγRIIIA, whereas monocytes express FcγRI, FcγRII and FcγRIII (Ravetch et al., 1991, supra).

Another important Fc ligand is the complement protein Clq. Fc binding to Clq mediates a process called complement dependent cytotoxicity (CDC) (reviewed in Ward et al., 1995, Ther Immunol 2:77-94). Clq is capable of binding six antibodies, although binding to two IgGs is sufficient to activate the complement cascade. Clq forms a complex with the Clr and Cls serine proteases to form the C1 complex of the complement pathway.

Several key features of antibodies including but not limited to, specificity for target, ability to mediate immune effector mechanisms, and long half-life in serum, make antibodies and related immunoglobulin molecules powerful therapeutics. Numerous monoclonal antibodies are currently in development or are being used therapeutically for the treatment of a variety of conditions including cancer. Examples of these include Herceptin® (Genentech), a humanized anti-Her2/neu antibody approved to treat breast cancer (e.g., U.S. 5,677,171), CNTO 95 (Centocor), a human Integrin αv antibody (PCT publication WO 02/12501), Rituxan® (IDEC/Genentech/Roche), a chimeric anti-CD20 antibody approved to treat Non-Hodgkin's lymphoma (e.g., U.S. 5,736,137) and Erbitux® (ImClone), a chimeric anti-EGFR antibody (e.g., U.S. 4,943,533).

There are a number of possible mechanisms by which antibodies destroy tumor cells, including anti-proliferation via blockage of needed growth pathways, intracellular signaling leading to apoptosis, enhanced down regulation and/or turnover of receptors, ADCC, CDC, and promotion of an adaptive immune response (Cragg et al., 1999, Curr Opin Immunol 11:541-547; Glennie et al., 2000, Immunol Today 21:403-410). However, despite widespread use, antibodies are not yet fully optimized for clinical use and many have suboptimal anticancer potency. Thus, there is a significant need to enhance the capacity of antibodies to destroy targeted cancer cells.

### 4.3 ANTIBODY CONJUGATES

The use of antibody conjugates, i.e. immunoconjugates, for the local delivery of cytotoxic or cytostatic agents, i.e. drugs to kill or inhibit tumor cells in the treatment of cancer (Lambert, J. (2005) Curr. Opinion in Pharmacology 5:543-549; Wu et al.(2005) Nature Biotechnology 23(9):1137-1146; Payne, G. (2003) Cancer Cell 3:207-212; Syrigos and Epenetos (1999) Anticancer Research 19:605-614; Niculescu-Duvaz and Springer (1997) Adv. Drug Del. Rev. 26:151-172; U.S. Pat. No. 4,975,278) may allow targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al (ed.s), pp. 475-506). Maximal efficacy with minimal toxicity is sought thereby. Efforts to design and refine antibody conjugates have focused on the selectivity of monoclonal antibodies (mAbs) as well as drug-linking and drug-releasing properties (Lambert, J. (2005) Curr. Opinion in Pharmacology 5:543-549). Both polyclonal antibodies and monoclonal antibodies have been reported as useful in these strategies (Rowland et al (1986) Cancer Immunol. Immunother., 21:183-87). Drugs used in these methods include daunomycin, doxorubicin, methotrexate, and vindesine (Rowland et al (1986)). Toxins used in antibody-toxin conjugates include bacterial toxins such as diphtheria toxin, plant toxins such as ricin, and gelonin, small molecule toxins such as geldanamycin (Mandler et al (2000) J. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may affect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

Several antibody conjugates have been approved by the FDA or are in clinical trials. For instance, ZEVALIN® (ibritumomab tiuxetan, Biogen/Idec) is composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman et al (2000) Eur. J. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN® has activity against B-cell non-Hodgkin's Lymphoma (NHL), administration results in severe and prolonged cytopenias in most patients. MYLOTARG® (gemtuzumab ozogamicin, Wyeth Pharmaceuticals), an antibody-drug conjugate composed of a human CD33 antibody linked to calicheamicin, was also approved in 2000 for the treatment of acute myeloid leukemia by injection (Drugs of the Future (2000) 25(7):686; U.S. Pat. Nos. 4,970,198; 5,079,233; 5,585,089; 5,606,040; 5,693,762; 5,739,116; 5,767,285; 5,773,001).

The auristatin peptides, auristatin E (AE) and monomethylauristatin (MMAE), synthetic analogs of dolastatin (WO 02/088172), have been conjugated to: (i) chimeric monoclonal antibodies cBR96 (specific to Lewis Y on carcinomas); (ii) cAC10 which is specific to CD30 on hematological malignancies (Klussman, et al (2004), Bioconjugate Chemistry 15(4):765-773; Doronina et al (2003) Nature Biotechnology 21(7):778-784; Francisco et al (2003) Blood 102(4):1458-1465; US 2004/0018194; (iii) anti-CD20 antibodies such as RITUXAN®(WO 04/032828) for the treatment of CD20-expressing cancers and immune disorders; (iv) anti-EphB2R antibodies 2H9 and anti-IL-8 for treatment of colorectal cancer (Mao et al (2004) Cancer Research 64(3):781-788); (v) E-selectin antibody (Bhaskar et al (2003) Cancer Res. 63:6387-6394); and (vi) other anti-CD30 antibodies (WO 03/043583). Variants of auristatin E are disclosed in U.S. Pat. No. 5,767,237 and U.S. Pat. No. 6,124,431. Monomethyl auristatin E conjugated to monoclonal antibodies are disclosed in Senter et al, Proceedings of the American Association for Cancer Research, Volume 45, Abstract Number 623, presented Mar. 28, 2004. Auristatin analogs MMAE and MMAF have been conjugated to various antibodies (WO 2005/081711).

Conventional means of attaching, i.e. linking through covalent bonds, a drug moiety to an antibody may lead to heterogeneous mixture of molecules where the drug moieties are attached at a number of sites on the antibody. For example, cytotoxic drugs have typically been conjugated to antibodies through the often-numerous lysine or cysteine residues of an antibody, generating a heterogeneous antibody-drug conjugate mixture. Depending on reaction conditions, the heterogeneous mixture typically contains a distribution of antibodies with from 0 to about 8, or more, attached drug moieties. In addition, within each subgroup of conjugates with a particular integer ratio of drug moieties to antibody, is a potentially heterogeneous mixture where the drug moiety is attached at various sites on the antibody.

Cysteine thiols are reactive at neutral pH, unlike most amines which are protonated and less nucleophilic near pH 7. Since thiol (R-SH, sulfhydryl) groups are relatively reactive, proteins with cysteine residues often exist in their oxidized form as disulfide-linked oligomers or have internally bridged disulfide groups. Extracellular proteins generally do not have free thiols (Garman, 1997, Non-Radioactive Labelling: A Practical Approach, Academic Press, London, at page 55). The amount of free thiol in a protein may be estimated by the standard Ellman's assay. IgM is an example of a disulfide-linked pentamer, while IgG is an example of a protein with internal disulfide bridges bonding the subunits together. In proteins such as this, reduction of the disulfide bonds with a reagent such as dithiothreitol (DTT), selenol (Singh et al (2002) Anal. Biochem. 304:147-156), and tris-(2-carboxyethyl)phosphine (TCEP) could be used generate the reactive thiol.

Antibody cysteine thiol groups are generally more reactive, i.e. more nucleophilic, towards electrophilic conjugation reagents than antibody amine or hydroxyl groups. Cysteine residues have been introduced into proteins by genetic engineering techniques to form covalent attachments to ligands or to form new intramolecular disulfide bonds (Better et al (1994) J. Biol. Chem. 13:9644-9650; Bernhard et al (1994) Bioconjugate Chem. 5:126-132; Greenwood et al (1994) Therapeutic Immunology 1:247-255; Tu et al (1999) Proc. Natl. Acad. Sci USA 96:4862-4867; Kanno et al (2000) J. of Biotechnology, 76:207-214; Chmura et al (2001) Proc. Nat. Acad. Sci. USA 98(15):8480-8484; U.S. Pat. No. 6,248,564). However, designing in cysteine thiol groups by the mutation of various amino acid residues of a protein to cysteine amino acids is potentially problematic, particularly in the case of unpaired (free Cys) residues or those which are relatively accessible for reaction or oxidation. In concentrated solutions of the protein, whether in the periplasm of E. *coli*, culture supernatants, or partially or completely purified protein, unpaired Cys residues on the surface of the protein can pair and oxidize to form intermolecular disulfides, and hence protein dimers or multimers. Disulfide dimer formation renders the new Cys unreactive for conjugation to a drug, ligand, or other label. Furthermore, if the protein oxidatively forms an intramolecular disulfide bond between the newly engineered Cys and an existing Cys residue, both Cys groups are unavailable for active site participation and interactions. Also, the protein may be rendered inactive or non-specific, by misfolding or loss of tertiary structure (Zhang et al (2002) Anal. Biochem. 311:1-9).\

Cysteine is not the only amino acid that could be used for conjugation and other amino acids such as lysine, tyrosine, histidine, selenocysteine, selenomethionine, and other amino acids are possible.

Previous attempts to engineer conjugation sites into antibodies have been attempted. US Patent No. 5,219,916 describes the modification of "surface pocket" residues such as Ser 156 or Thr 173 (according to Kabat et al., Sequences of Immunological Interest, 4th ed., US Dept. of Health and Human Services, 1987). In the related study, the researchers determined that only residues on "surface pockets" were capable of supporting the substitution of cysteine in an effort to engineer a conjugation site (Lyons et al. (1990) Protein Eng. 3:8 pg 703-708).

Thus, there is a need to develop stable engineered antibodies which provide reactive groups capable of conjugation to various agents, including engineered antibodies which provide thiol groups capable of conjugation to various agents.

Citation or discussion of a reference herein shall not be construed as an admission that such is prior art to the present disclosure.

### 5. SUMMARY

The present disclosure provides Fc regions that contain non-naturally occurring (i.e., those not normally present at that position in the protein) amino acid residues (which may be natural and/or synthetic) capable of conjugation to various agents. One example of such a substitution is the use of cysteine, however other substitutions, such as lysine, tyrosine, histidine, selenocysteine, selenomethionine, and other amino acids are possible, as described below.

Fc regions of the disclosure comprise one or more amino acids from the Fc region of the heavy chain of an antibody substituted with one or more non-naturally occurring amino acids thereby providing a group capable for conjugation. In one aspect of the disclosure, the substitution is with cysteine whereby the substituted cysteine amino acid residue provides a thiol group for conjugation.

In one embodiment, the Fc regions of the disclosure comprise 1, 2, 3, 4, 5, 6, 7, 8, 9, 10, 11, 12, 13, 14, 15, 16, 17, 18, 19, 20, or more substituted amino acids. In other embodiments, the Fc regions of the disclosure include Fc regions where 1, 2, 3, 4, 5, 6, 7, 8, 9, 10, 11, 12, 13, 14, 15, 16, 17, 18, 19, 20, or more amino acids chosen from positions 239, 282, 289, 297, 312, 324, 330, 335, 337, 339, 356, 359, 361, 383, 384, 398, 400, 440, 422, and 442 of the antibody heavy chain wherein the numbering system of the constant region is that of the EU index as set forth in Kabat et al. (1991, NIH Publication 91-3242, National Technical Information Service, Springfield, VA, hereinafter "Kabat") of a parent or native antibody or alternatively, are substituted with a non-naturally occurring amino acid. The "EU index as set forth in Kabat" refers to the residue numbering of the human IgG1 EU antibody as described in Kabat et al. *supra.*

In one embodiment, the Fc regions of the disclosure comprise antibodies.

In one embodiment, the disclosure provides Fc regions comprising the amino acid sequences of SEQ ID NOs: 1-24. In another embodiment, the disclosure provides Fc regions comprising any of the substitutions exemplified by SEQ ID NOs: 1-24 in any combination.

In one embodiment, the disclosure provides antibodies comprising the amino acid sequences of SEQ ID NOs: 1-24. In another embodiment, the disclosure provides Fc regions comprising any of the substitutions exemplified by SEQ ID NOs: 1-24 in any combination.

Another aspect of the disclosure provides nucleic acids, vectors and host cells for the generation of Fc regions.

Another aspect of the disclosure provides Fc region conjugates and methods of making such conjugates comprising the Fc regions of the disclosure coupled to one or more additional substances. In one embodiment, the substance is a drug where the drug is chosen from cytotoxic agent, chemotherapeutic agent, peptide, peptidomimetic, protein scaffold, enzyme, toxin, radionuclide, DNA, RNA, siRNA, microRNA, peptidonucleic acid, fluorescent tag, or biotin.

Another aspect of the disclosure provides antibodies comprising the Fc regions of the disclosure, wherein the antibodies are capable of internalizing when bound to cell surface receptors. In such aspects, antibodies of the disclosure are useful for intracellular delivery of cargo molecules and/or agents.

Another aspect of the disclosure provides methods of treating, detecting, and diagnosing cancer, autoimmune, inflammatory, or infectious diseases with the antibody conjugates of the disclosure.

Another aspect of the disclosure provides compositions comprising the Fc regions of the disclosure.

### 6. BRIEF DESCRIPTION OF THE FIGURES

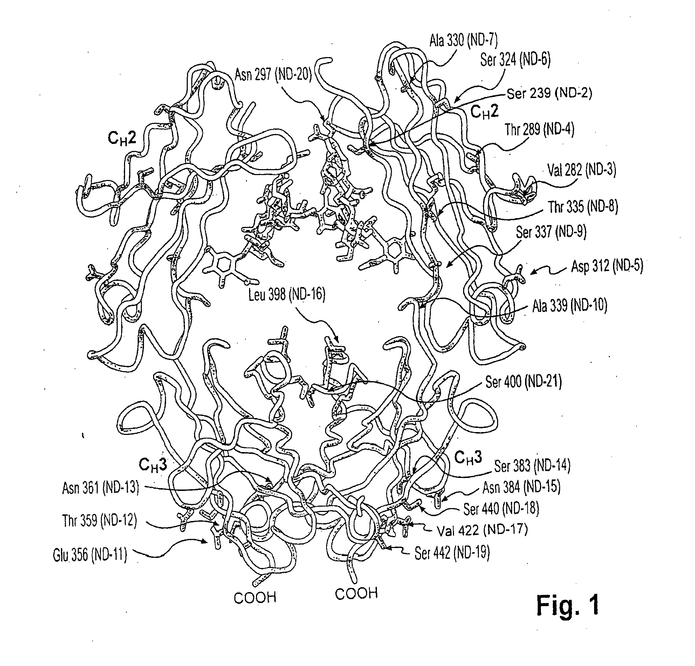

**Fig.1****. Fc region surface cysteine engineering.** This panel is a ribbon representation of an IgG1 Fc domain with surface site-specific mutations schematically labeled and shown in stick form. In this ribbon representation the CH2 and CH3 domains are schematically labeled. The sugar chains are shown as stick representations. Twenty cysteine engineering mutations ND2 to ND21 are shown as stick representations and schematically labeled with the respective clone names in parentheses, with ND1 representing the native antibody. Their numeration is based on the EU index as set forth in Kabat.

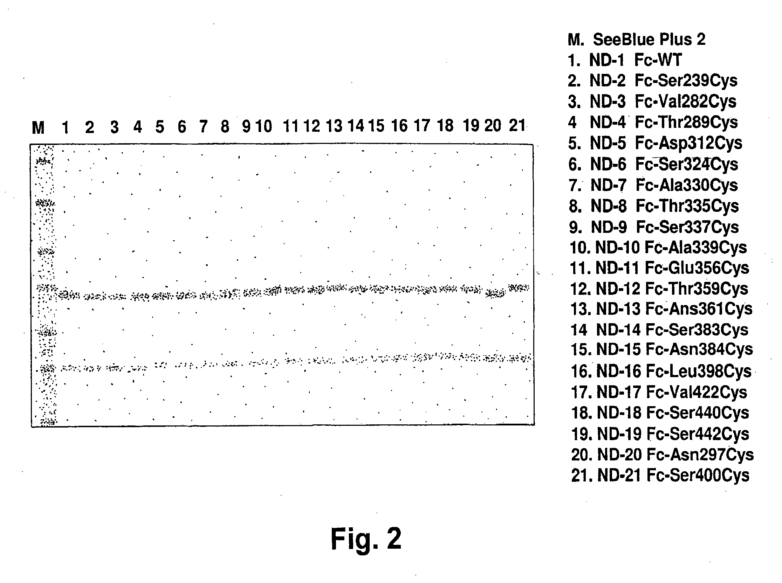

**Fig. 2****. SDS-PAGE of protein A purified Fc-cysteine mutants in reducing conditions.** All mutant and wild-type proteins were purified by Protein A, dialyzed into PBS 1X, 10 mM EDTA, pH 7.2, and analyzed on a 10% homogeneous SDS-PAGE under reducing and non-reducing conditions. SeeBlue Plus2 (Invitrogen) molecular weight standards (in kDa as schematically indicated) were used. 3 µg of each sample were loaded and the gel was stained using SimplyBlue™ SafeStain (Invitrogen). The samples are schematically labeled on the Figure and correspond to the clone names presented in Figure 1. This SDS-PAGE analysis confirms that the recombinant proteins retain their expected molecular weight.

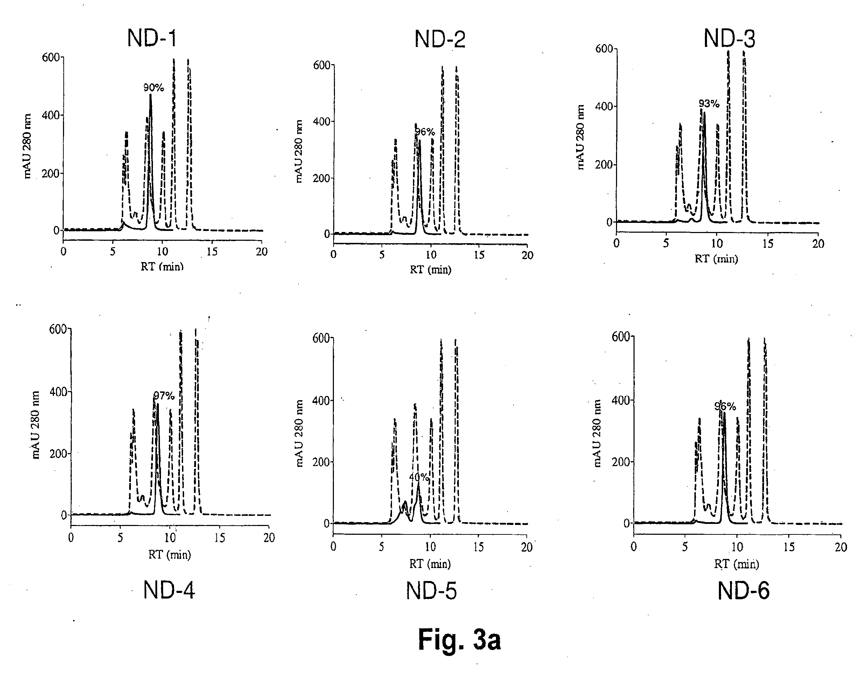

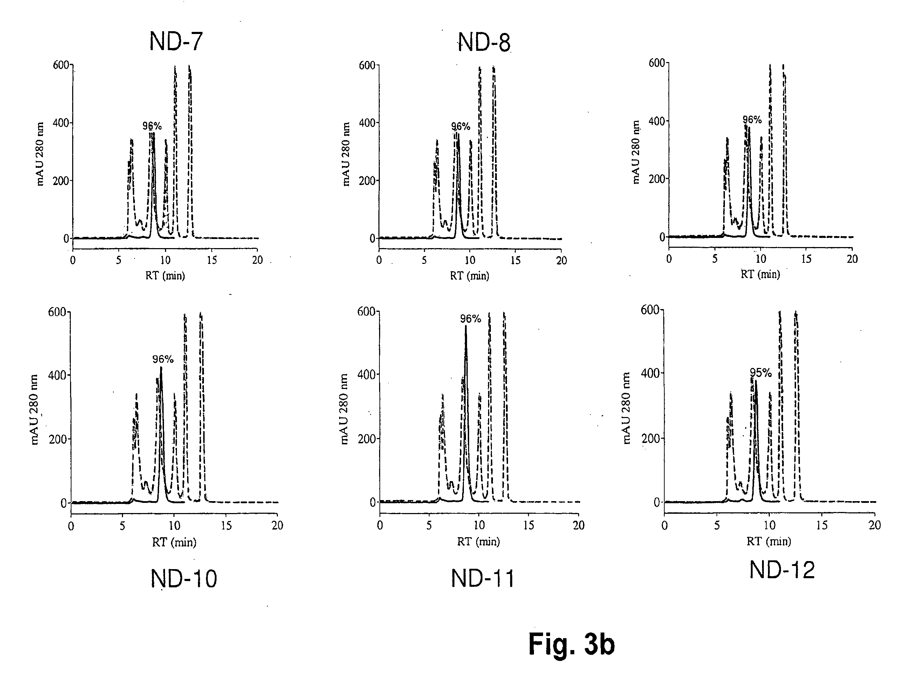

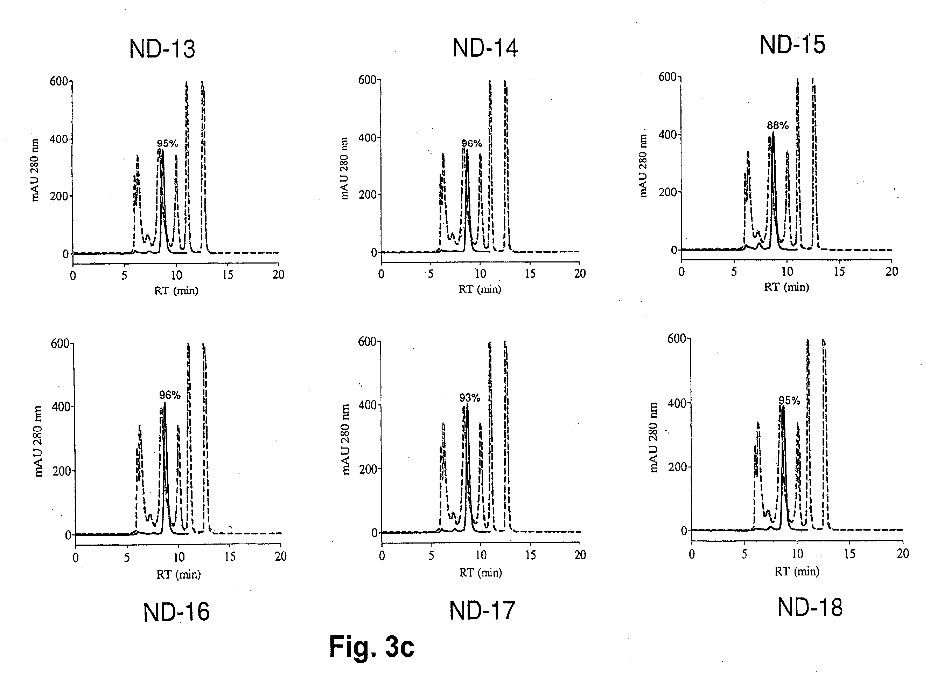

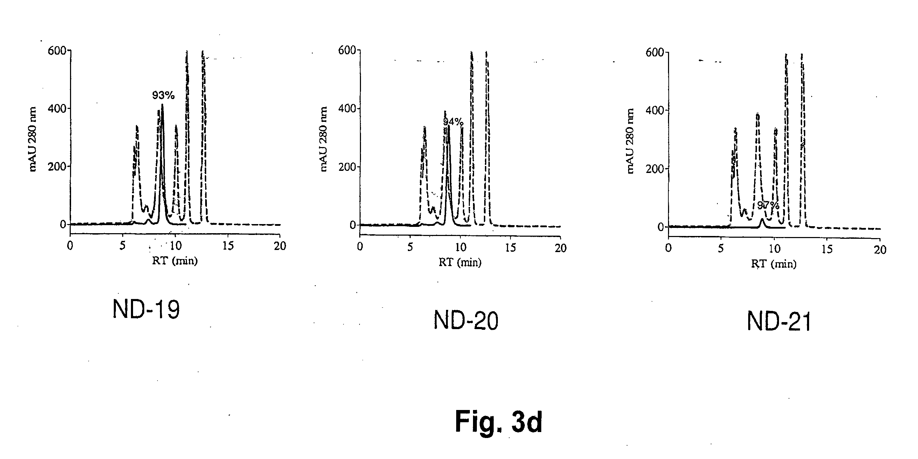

**Fig. 3****(a-d). Size-Exclusion chromatography of antibody mutants.** All mutant proteins were purified by protein A, dialyzed into PBS 1X, 10 mM EDTA, pH 7.2, and analyzed on SEC-HPLC. The identity of the samples correspond to the clone names presented in Figure 1. Peak position of standard proteins (dotted chromatogram) from right to left are thyroglobulin 670 KDa; 2, bovine gamma-globulin 158 KDa; 3, chicken ovalbumin 44 KDa; 4, equine myoglobin 17 KDa; 5, vitamin B12 13.5 KDa. The mutant antibodies retention time is about 8.6 minutes, which correspond to a molecular weight of about 150 KDa. The percentage of monomeric content for each mutant is also shown. This analysis confirms that the recombinant proteins in addition of retaining their expected molecular weight, do not form disulfide-linked homodimers due to the introduced cysteines.

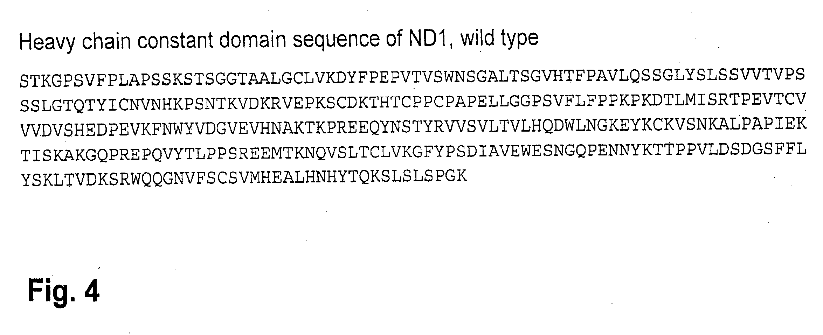

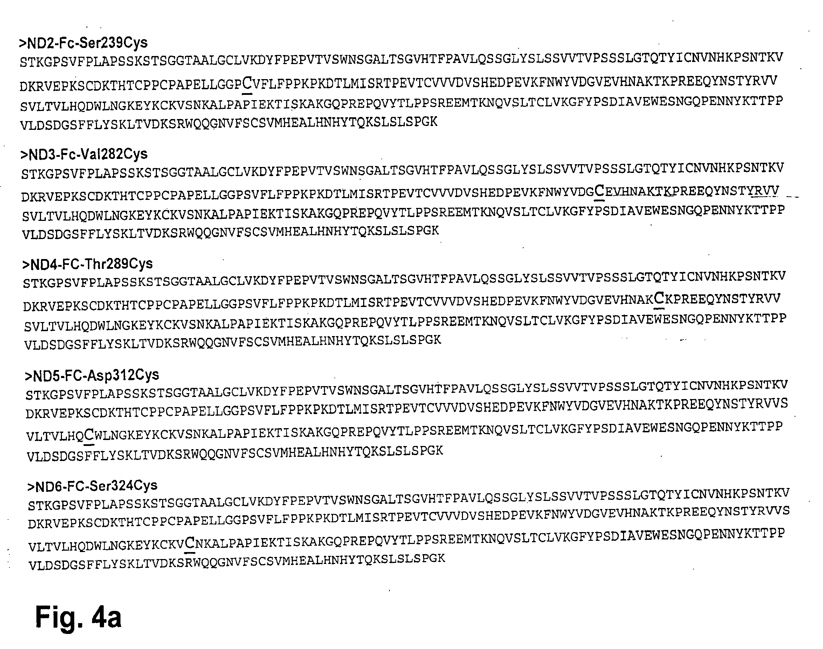

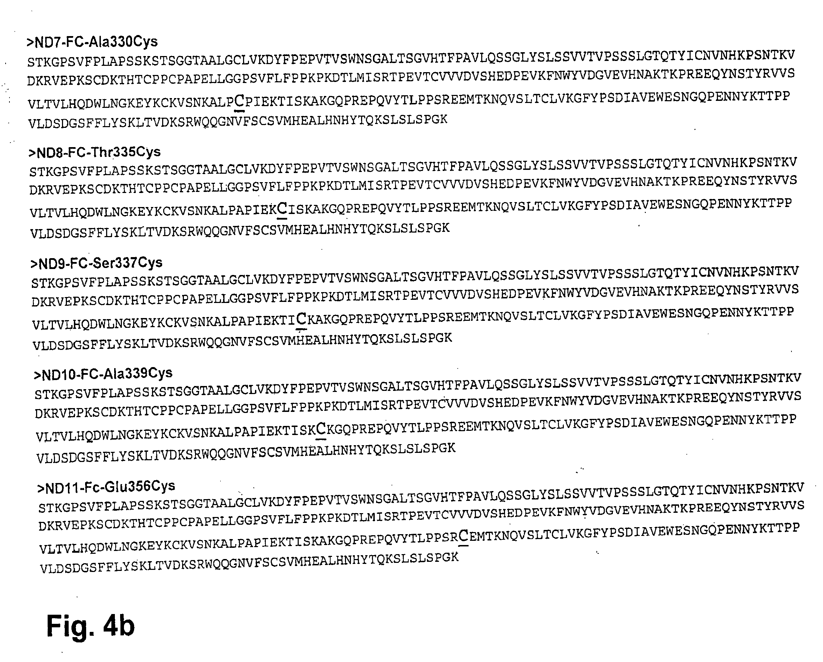

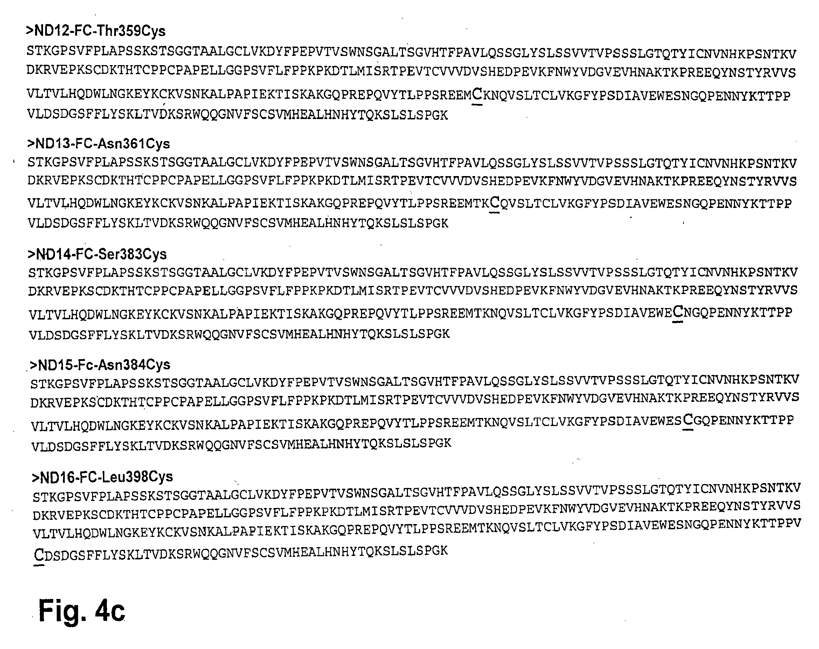

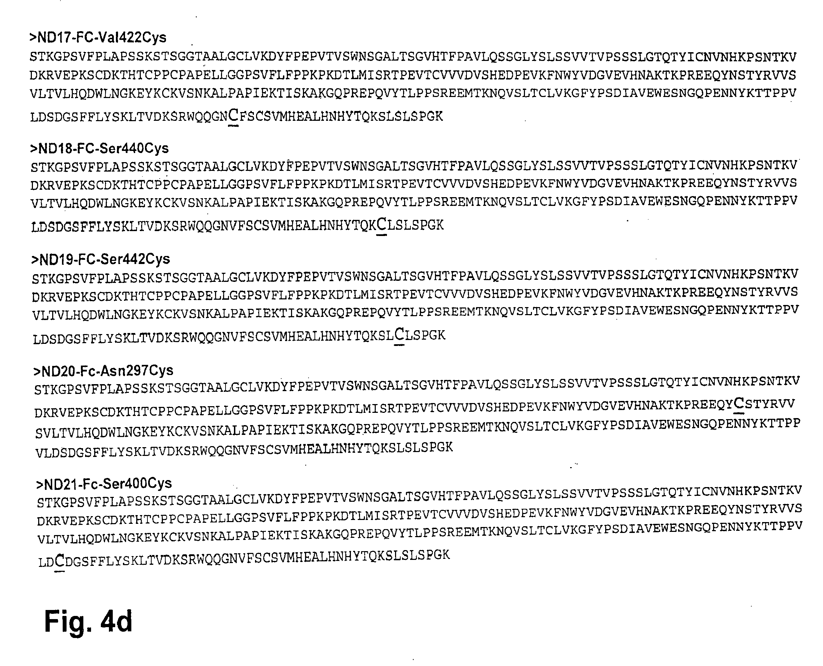

**Figs. 4** **(a-c). Heavy chain constant domain sequences.** Figure 4 shows the sequence of the heavy chain constant domain sequence of ND1 (native). Fig. 4a shows the sequences of variants ND2 through ND6. Fig. 4b shows the sequences of variants ND7 through ND 11. Fig. 4c shows the sequences of variants ND12 through ND16. Fig. 4d shows the sequences of variants ND17 through ND21. The introduced point mutations of ND2 to ND21 are underlined and bold.

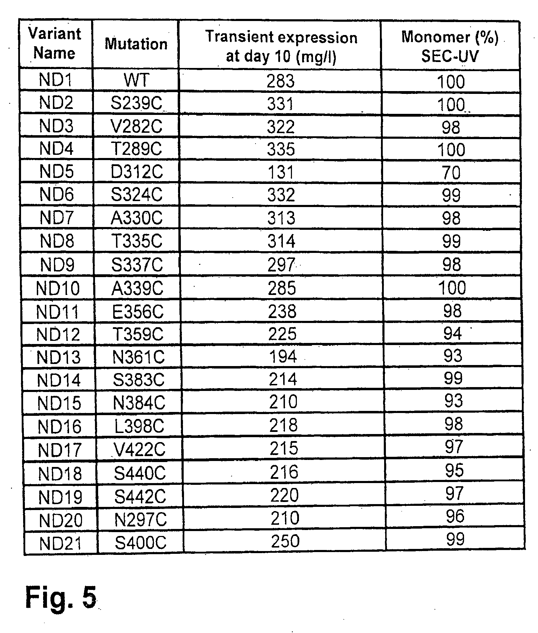

**Fig. 5****. Expression of Fc domain variants.** Recombinant expression of Fc cysteine variants is shown. Each variant had an expression level comparable to the native ND1. All variants except variant ND5 also were expressed at or greater than 93% monomer (determined by SEC-HPLC).

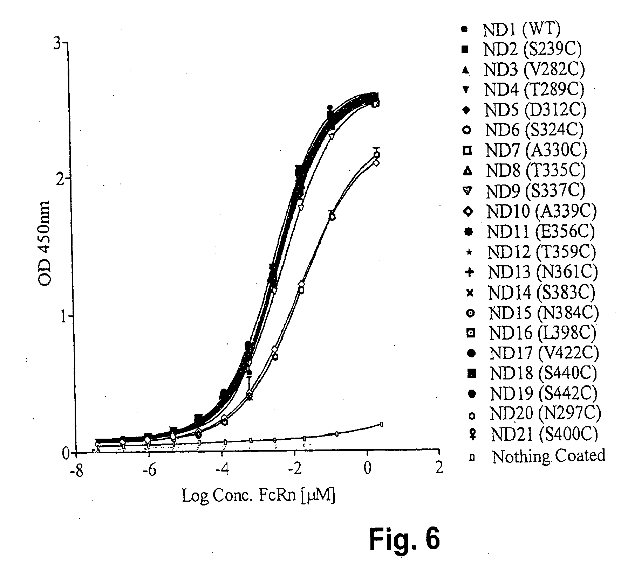

**Fig. 6****. Binding of Fc domain variants to FcRn.** FcRn binding of the cysteine Fc-engineered variants was determined by enzyme-linked immunoabsorbent assay (ELISA). Except variant ND5 (D312C) that is 70 % monomeric, and the non-glycosylated variant ND20 (N297C), all variants retain binding signal to FcRn similar to the native antibody ND1.

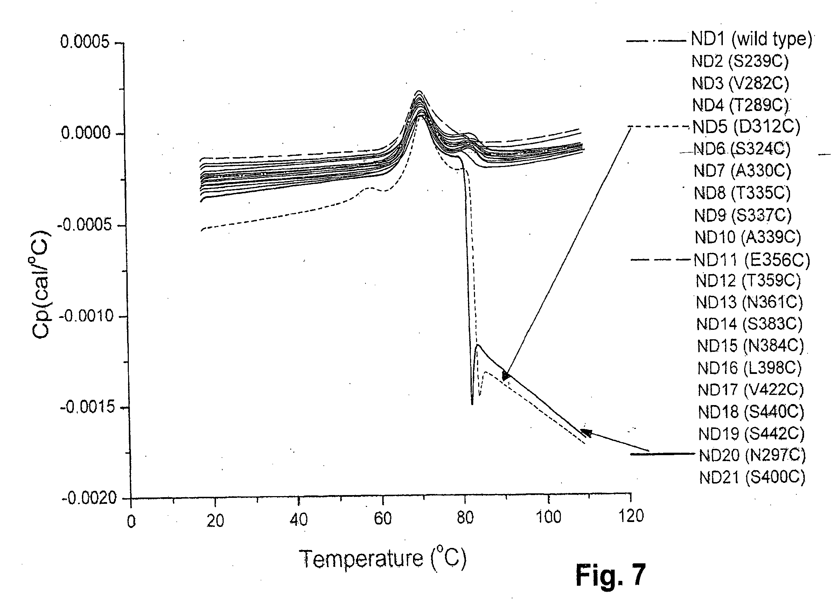

**Fig. 7****. Differential scanning calorimetry (DSC) analysis.** DSC analysis of ND1-21 was conducted using a buffer of 25 mM His, pH 6.0, a range from 15°C to 110°C, and a rate of 1°C / min. Except variants ND5 (D312C), ND11 (E356C), and ND20 (N297C), the variants have similar DSC profiles to the native ND1.

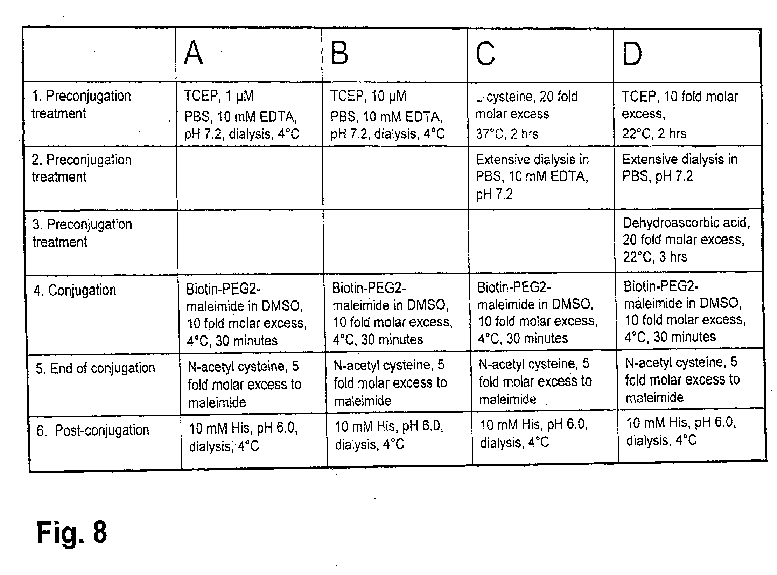

**Fig. 8****. Conjugation protocols.** Various protocols for the conjugation of Biotin-PEG2-maleimide to the antibody Fc cysteine are shown for D1-21 at 1 mg scale. Indicated molar excess is with respect to moles antibody unless specified otherwise.

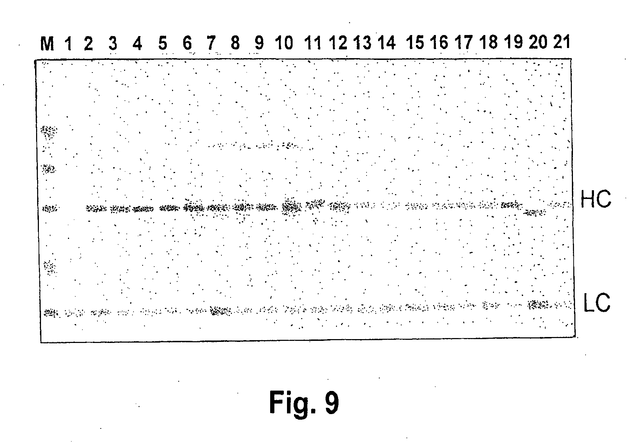

**Fig. 9****. Western blot of reducing protein gels on samples from conjugation protocol D.** The variant and native proteins were purified by Protein A and dialyzed into PBS 1X, 10 mM EDTA, pH 7.2. The numbers 1-21 correspond to ND1-21, respectively. M is a molecular weight marker. The samples were first resolved by SDS-PAGE on NuPage 10% gels. The blots with transferred protein were blocked in TBST 2% BSA, incubated with avidin-HRP, and visualized with a chromogenic substrate for HRP. HC are the protein bands corresponding to the antibody heavy chains, and LC are the protein bands corresponding to the antibody light chains. There is pronounced conjugation on the heavy chain of a number of variants (especially ND2, 3, 4, 6, 7, 8, 9, 10, 11, 12, 18, 19), while there is no detectable conjugation on the HC of ND1 (native). There is some conjugation on the LC of all variants, including ND1.

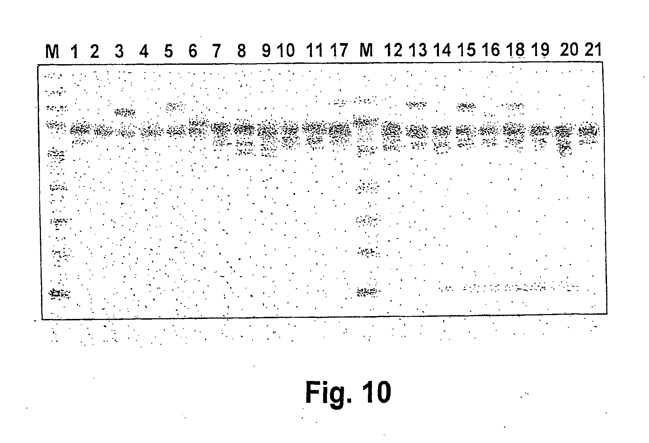

**Fig. 10****. Coomassie-stained non-reducing protein gels on samples from conjugation protocol D.** The variant and native proteins were purified by Protein A, dialyzed into PBS 1X, 10 mM EDTA, pH 7.2, and analyzed on a 10% homogeneous SDS-PAGE under non-reducing conditions. SeeBlue Plus2 (Invitrogen) molecular weight standards were used. 3 µg of each sample were loaded and the gel was stained using SimplyBlue™ SafeStain (Invitrogen). The numbers 1-21 correspond to ND1-21, respectively. M is the molecular weight marker. Most conjugated variants show electrophoretic mobility patterns similar to native ND1. Several conjugated variants contain some apparent dimer (ND 3, 5, 13, 15, 17, 18, 19), and one variant shows some apparent fragmentation (ND 20).

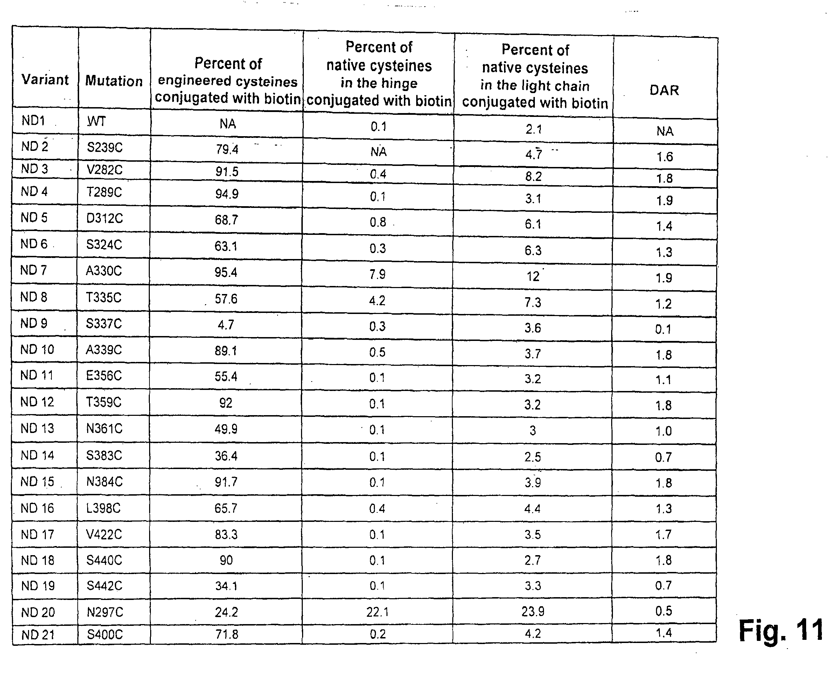

**Fig. 11****. Intact mass and peptide mapping data summaries of ND variants, conjugated by protocol D.** Variants ND4, 7, 10, 12, 18 have close to or greater than 90% conjugation at the engineered cysteines, and show no or little oligomerization. DAR is Drug to Antibody molar Ratio of the conjugated sample.

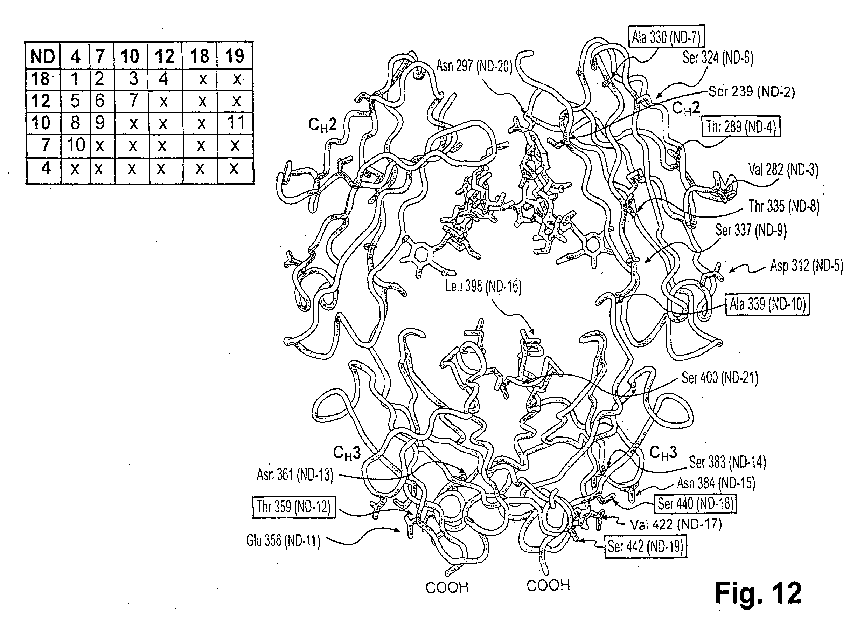

**Fig. 12****. Double variants.** Eleven double variants were designed based on select single variants. The grid in the upper left of the figure shows the combination of single variants used to generate the double variants.

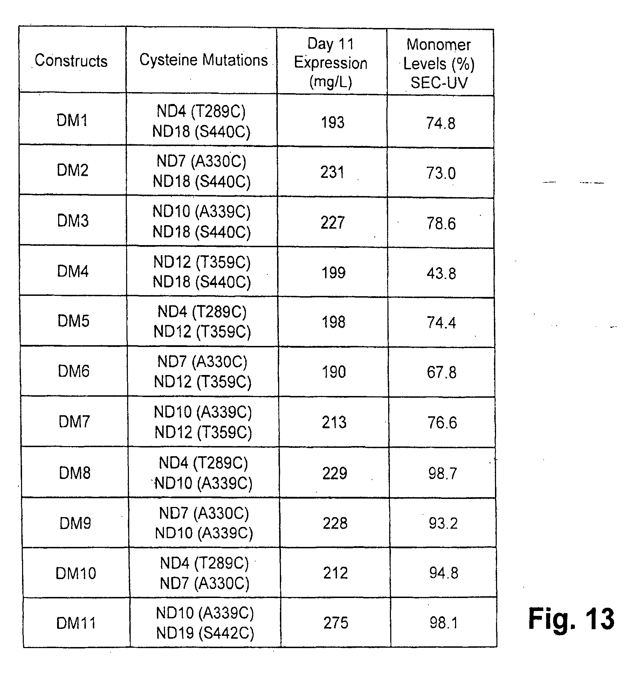

**Fig. 13****. Expression of double variants.** A set of 11 double ND variants was made based on select single variants, ND4, ND7, ND10, ND12, ND18, and another double variant based on ND10 and ND19. All double variants express well by transient transfection. Only four of these double variants, DM8, DM9, DM10, DM11 are expressed and purified at greater than 93% monomer levels (determined by size exclusion chromatography with ultraviolet detection (SEC-UV)).

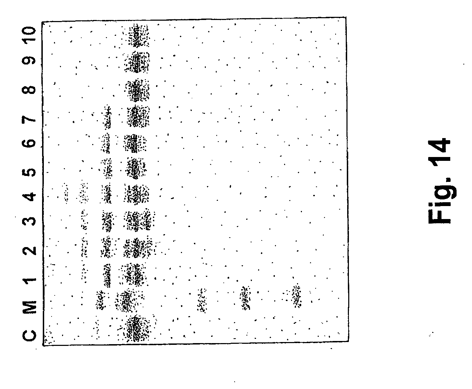

**Fig.** 14. **Comparison of double ND variants on non-reducing SDS-PAGE.** "C" is native ND1 used as a control. "M" is a molecular weight standard. The numbers 1-10 correspond to DM1-10. The SDS-PAGE results are consistent with the SEC-UV results. Variant DM4 has the least amount of monomer, and DM8 contains the largest percent of monomer.

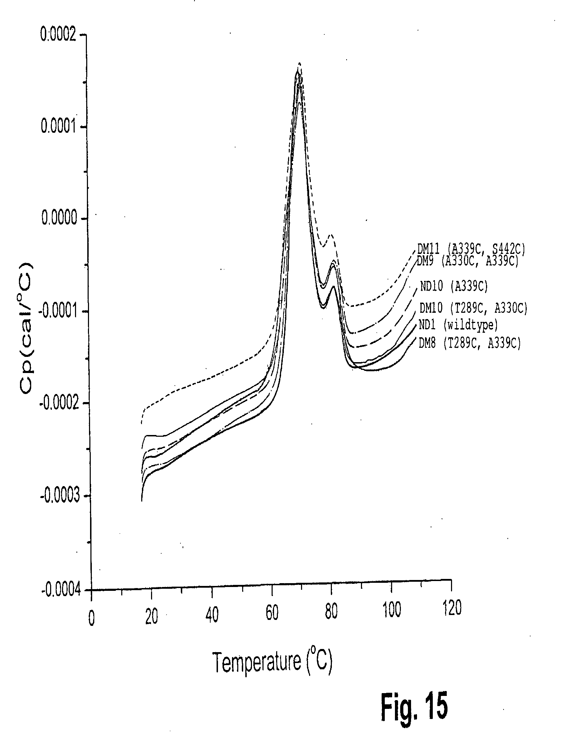

**Fig. 15****. DSC analysis of double variants.** The four mostly monomeric (>93%) double variants show DSC profiles very similar to native and to the single variant ND10.

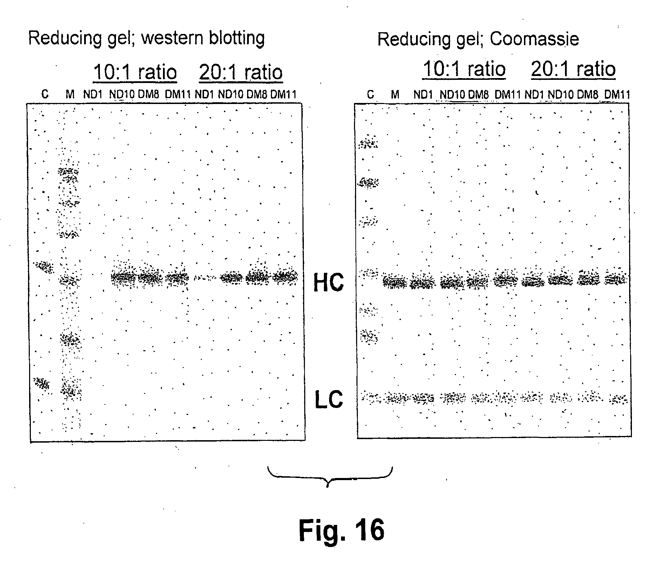

**Fig. 16****. Conjugation of double variants.** Conjugation of double variants DM8 (ND 4 and ND10) and DM11 (ND10 and ND19) using protocol D (see Figure 8) 10:1 biotin-maleimide:antibody ratio and 20:1 biotin-maleimide:antibody ratio. Reducing gels subjected to Western blotting and coomassie staining are shown.

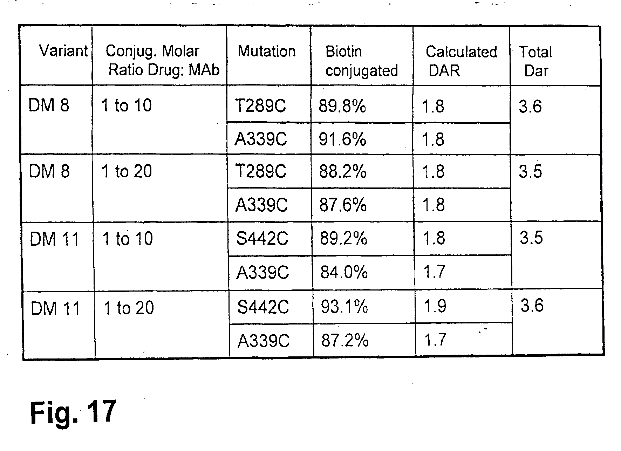

**Fig. 17****. Intact mass and peptide mapping data of double variants, conjugated as described in** **Fig. 16****.** DM8 (ND4 and ND10) and DM11 (ND10 and ND19) have close to or greater than 90% conjugation at the engineered cysteines with either 10:1 biotin-maleimide:antibody ratio or 20:1 biotin-maleimide:antibody ratio.. DAR is Drug to Antibody molar Ratio of the conjugated sample.

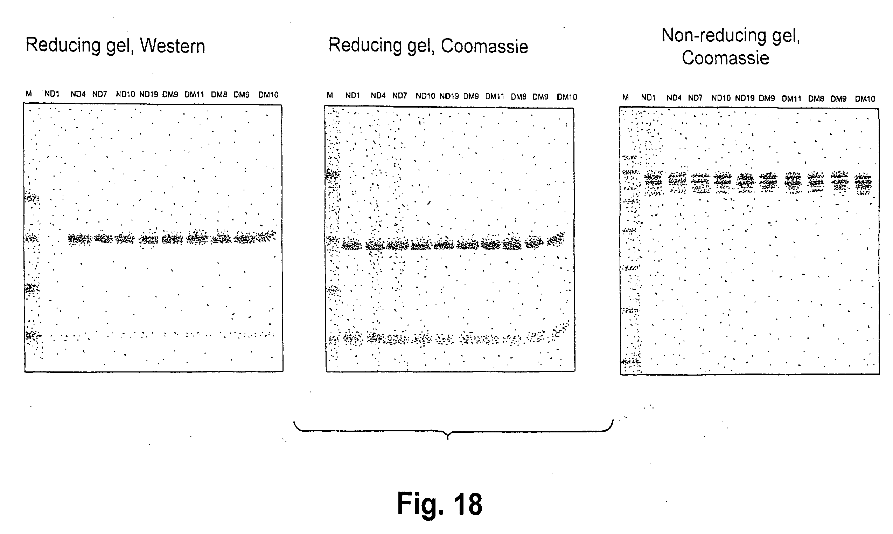

**Fig. 18****. Conjugation of single and double ND variants.** Conjugation protocol D (Figure 8) was used except for the employment of a more extensive overnight dialysis after TCEP treatment, and conjugation for 1 hr at room temperature. The drug:mAb molar ratio was 10:1 for single variants and 20:1 for double variants. Reducing gels subjected to Western blotting and coomassie staining are shown, as well as a non-reducing gel stained with coomassie.

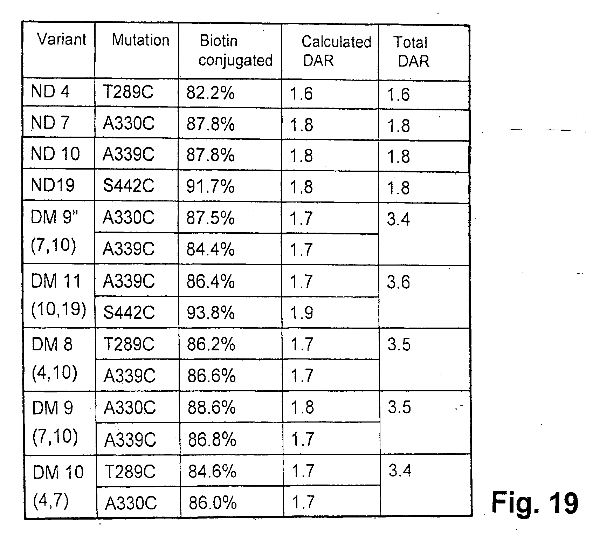

**Fig. 19****. Peptide mapping of single and double variants.** The conjugated samples shown on the previous Figure 18 were analyzed by peptide mapping analysis. This table summarizes the conjugation efficiencies at the engineered cysteines, as well as the overall drug-to-antibody ratio (DAR). DM9" is a second preparation of DM9 using the same mutations.

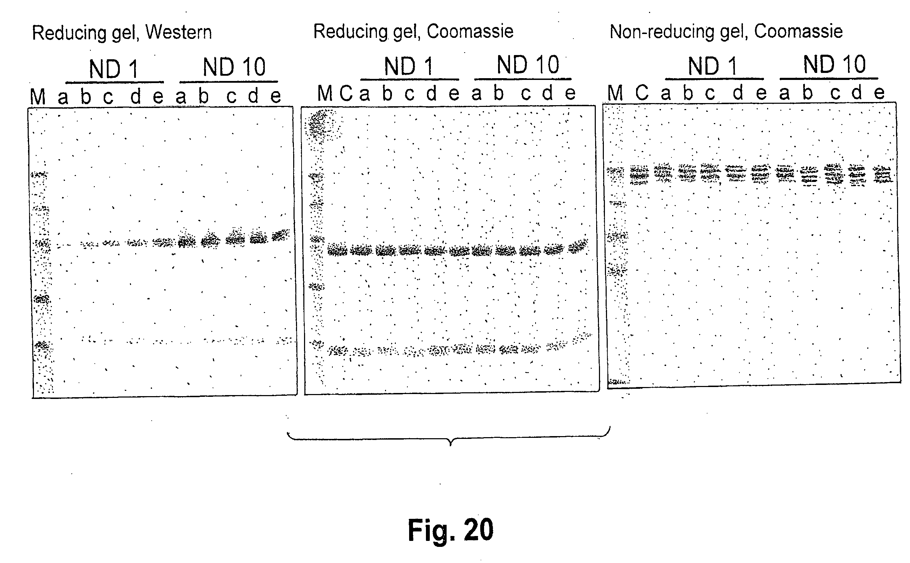

**Fig. 20****. Scale-up conjugation experiment.** Conjugation was performed at (a) 2.5, (b) 5, (c) 10, (d) 20, and (e) 40 mg/ml of antibody. The conjugation protocol as described for Fig. 18 was used. The drug:mab molar ratio was 10:1. In the protein gels, C is native ND1. Reducing gels subjected to Western blotting and coomassie staining are shown, as well as a non-reducing gel stained with coomassie.

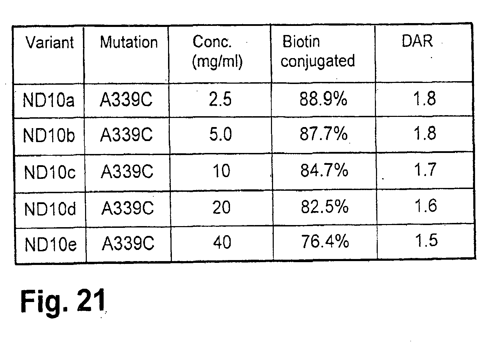

**Fig. 21****. Peptide mapping of scaled-up conjugation.** The conjugated samples from the previous Figure 20 were analyzed by peptide mapping analysis. The conjugation efficiency results are shown.

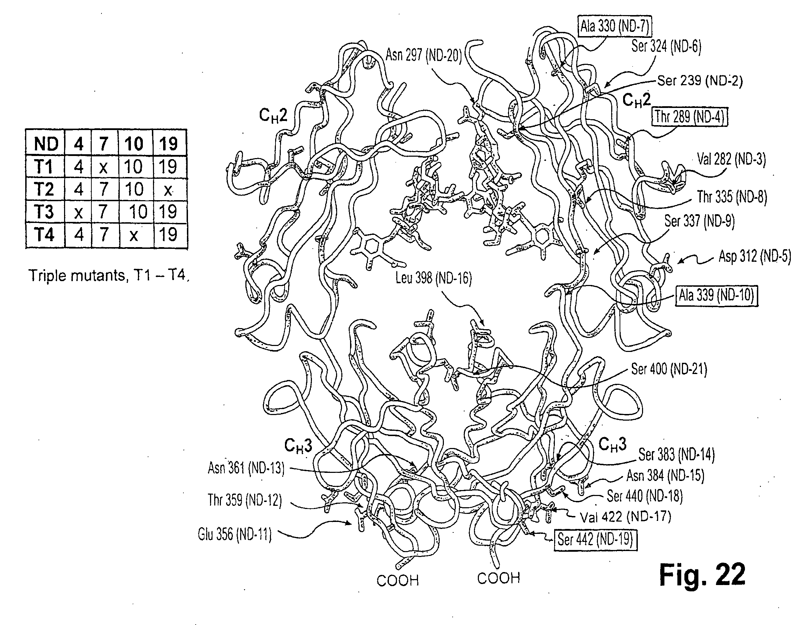

**Fig. 22** **Triple variants.** This illustrates the residues selected for mutation in four triple variants based on single variants. The grid in the upper left of the figure shows the combination of variants used to generate the triple variants.

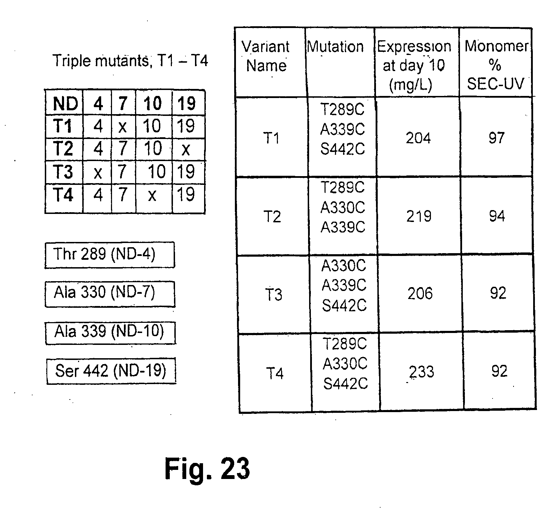

**Fig. 23****. Expression and monomer levels of the four triple variants.** Protein was purified from 0.5 L culture. Monomer levels were determined on SEC-HPLC. For comparison, native antibody ND1 is 99% monomeric.

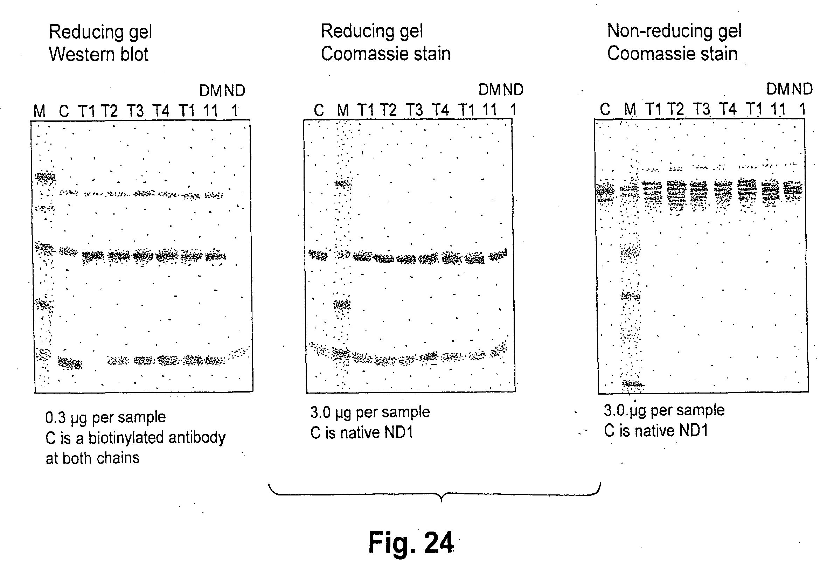

**Fig. 24****. Conjugation of triple variants with biotin maleimide.** Protocol D was used with conjugation for 1 hr at room temperature and 1:20 antibody:drug molar ratio. Two different protein preps of T1 were conjugated. The native ND1 and the double variant DM11 were included as controls for conjugation specificity and efficiency. Reducing gels subjected to Western blotting and coomassie staining are shown, as well as a non-reducing gel stained with coomassie.

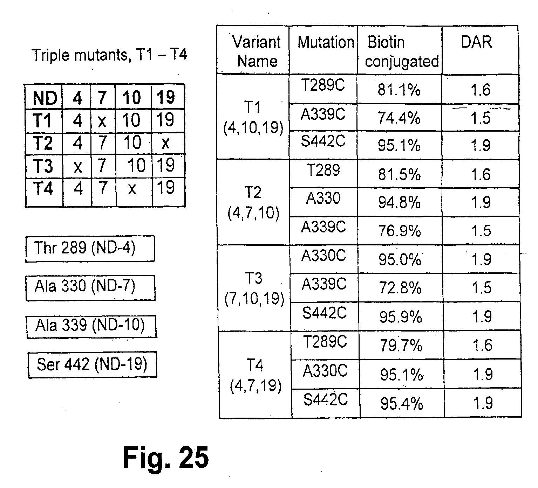

**Fig. 25****. Peptide mapping of triple variants.** The conjugated triple variant samples from the previous Figure 24 were analyzed by peptide mapping analysis. The conjugation efficiencies at the engineered cysteines are presented.

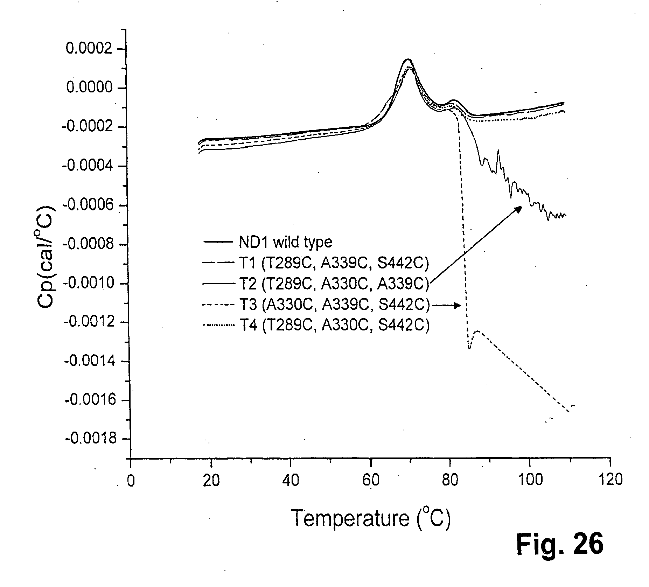

**Fig. 26****. DSC analysis of triple variants.** T1 shows a few degrees lower melting temperature for the first transition, CH2. T2 shows some precipitation at 85°C. T3 precipitates at high temperatures. T4 is nearly identical to native.

### 7. DETAILED DESCRIPTION

The disclosure is based on the finding that residues present on the surface of the CH2 or CH3 domain of antibodies (see Figure 1) are suitable for the substitution of the naturally-occurring amino acid with, for example, cysteine, and useful to engineer a site capable of conjugation to various agents.

Other amino acids besides cysteine, including natural and/or non-natural amino acids, may be used in the substitution to allow for conjugation of various agents. Such other amino acids include lysine (described in Benhar et al., (1994) Bioconjug. Chem. 55, 321-326), tyrosine (described in Byers & Baldwin, (1988) Immunology. 65, 329-335), histidine (described in Waibel et al. (1999) Nature Biotechnol. 17: 897-901), selenocysteine, selenomethionine, and/or non-natural amino acids. Thus, where one or more cysteine substitutions are described herein, one of ordinary skill in the art may optionally employ one or more of these natural and/or non-natural amino acids instead of cysteine. One of ordinary skill in the art may also use any combination of amino acids in the substitution, such as substituting with cysteine and lysine to produce a variant antibody with cysteines substituted at some positions and lysines at others.

The Fc regions of the disclosure include engineered antibodies where 1, 2, 3, 4, 5, 6, 7, 8, 9, 10, 11, 12, 13, 14, 15, 16, 17, 18, 19, 20, or more amino acids chosen from positions: 239, 282, 289, 297, 312, 324, 330, 335, 337, 339, 356, 359, 361, 383, 384, 398, 400, 440, 422, and 442 of the antibody heavy chain wherein the numbering system of the constant region is that of the EU index as set forth in Kabat et al. (1991, NIH Publication 91-3242, National Technical Information Service, Springfield, VA, hereinafter "Kabat") of a parent, native, or wild type antibody are substituted with another amino acid (including natural and synthetic amino acids). It should be noted that a single substitution, for example of a cysteine residue, normally results in the display of two corresponding residues in the resultant antibody due to the homodimeric nature of IgG molecules. The resultant engineered antibodies of the disclosure may display at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or more reactive groups for the purpose of conjugation to a drug or compound. In an embodiment, one or more of the substitutions is with a cysteine residue, and the resulting engineered antibodies may display at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or more thiol groups for the purpose of conjugation to a drug or compound.

In some embodiments, the engineered antibodies of the disclosure comprise at least one substitution at positions selected from: 239, 282, 289, 297, 312, 324, 330, 335, 337, 339, 356, 359, 361, 383, 384, 398, 400, 440, 422, and 442 of the heavy chain of an antibody, wherein the numbering system of the constant region is that of the EU index as set forth in Kabat et al. (*supra*). In other embodiments, the engineered antibodies of the disclosure comprise at least two substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least three substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least four substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least five substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least six substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least seven substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least eight substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least nine substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least ten substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least eleven substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least twelve substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least thirteen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least fourteen substitutions selected from the positions 239, 282, 289, 297, 312, 324, 330, 335, 337, 339, 356, 359, 361, 383, 384, 398, 400, 440, 422, and 442 of the heavy chain of an antibody. In other embodiments, the engineered antibodies of the disclosure comprise at least fifteen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least sixteen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least seventeen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least eighteen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise at least nineteen substitutions selected from the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure comprise substitutions at each of the positions 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat.

In one embodiment, the engineered antibodies comprise following pairs of substitutions:
a) 289 and 440;
b) 330 and 440;
c) 339 and 440;
d) 359 and 440;
e) 289 and 359;
f) 330 and 359;
g) 339 and 359;
h) 289 and 339;
i) 330 and 339;
j) 289 and 330; and
k) 339 and 442.

In another embodiment, the engineered antibodies comprise one or more of the following groups of substitutions:
a) 289, 339, and 442;
b) 289, 330, and 339;
c) 330, 339, and 442; and
d) 289, 330, and 442.

The substitutions described above correspond to the positions in SEQ ID NO: 1 as follows, and it is intended that the number with reference to Kabat throughout the disclosure may be used interchangeably the the position of the substitutions in SEQ ID NO: 1 to describe the compositions of the disclosure:

| **ND** | **Kabat position** | **Position in SEQ ID NO: 1** |
|---|---|---|
| 2 | 239 | 121 |
| 3 | 282 | 164 |
| 4 | 289 | 171 |
| 5 | 312 | 194 |
| 6 | 324 | 206 |
| 7 | 330 | 212 |
| 8 | 335 | 217 |
| 9 | 337 | 219 |
| 10 | 339 | 221 |
| 11 | 356 | 238 |
| 12 | 359 | 241 |
| 13 | 361 | 243 |
| 14 | 383 | 265 |
| 15 | 384 | 266 |
| 16 | 398 | 280 |
| 17 | 422 | 304 |
| 18 | 440 | 322 |
| 19 | 442 | 324 |
| 20 | 297 | 179 |
| 21 | 400 | 282 |

In other embodiments, the engineered antibodies of the disclosure comprise a substitution of at least one naturally occurring amino acid chosen from: Ser239, Val282, Thr289, Asn297, Asp312, Ser324, Ala330, Thr335, Ser337, Ala339, Glu356, Thr359, Asn361, Ser383, Asn384, Leu398, Ser400, Ser440, Val422, and Ser442 of the heavy chain of an antibody wherein the numbering system of the constant region is that of the EU index as set forth in Kabat.

In some embodiments, the engineered antibodies of the disclosure do not comprise a substitution at a position or positions selected from: Ser239, Val282, Thr289, Asn297, Asp312, Ser324, Ala330, Thr335, Ser337, Ala339, Glu356, Thr359, Asn361, Ser383, Asn384, Leu398, Ser400, Ser440, Val422, and Ser442 of the heavy chain of an antibody wherein the numbering system of the constant region is that of the EU index as set forth in Kabat.

In other embodiments, the engineered antibodies of the disclosure comprise a substitution of the naturally occurring amino acid at position 297 wherein said substitution reduces and/or ablates glycosylation at position 297. In specific embodiments, antibodies of the disclosure comprise a substitution of cysteine for asparagine at position 297 of the heavy chain of the antibody. In yet other embodiments, the disclosure provides antibodies lacking glycosylation at position 297 of the heavy chain of the antibody. In each of these, the numbering system of the constant region is that of the EU index as set forth in Kabat.

In one embodiment, the engineered antibodies of the disclosure include an IgG1 having a naturally occurring amino acid substituted (for example, with a cysteine) at a position chosen from: 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. In other embodiments, the engineered antibodies of the disclosure are derived from an IgG1, IgG2, IgG3 or an IgG4 format. In yet other embodiments, the engineered antibodies of the disclosure are derived from non-IgG formats such as IgA1, IgA2 IgM, IgD, or IgE. In other embodiments, antibodies of the disclosure comprise engineering of surface residues of the CH2 and/or CH3 region of an IgG1 molecule or equivalents thereof by substitution of a naturally-occurring residue for cysteine and/or other amino acids.

One of ordinary skill in the art can readily select a suitable amino acid to use in the substitution. It may be desirable to select a residue that is similar to the non-naturally occurring residue in order to minimize changes to the protein structure. For example, for cysteine substitutions, it can be desirable to substitute cysteine for a naturally occurring alanine or serine.

In the case of substitutions in IgG2, IgG3, and IgG4, one of ordinary skill in the art can use sequence alignment with IgG1 to determine which residues of the desired isoform correspond to the above-described positions of 239, 282, 289, 297, 312, 324, 330, 335, 337, 339, 356, 359, 361, 383, 384, 398, 400, 440, 422, and 442 of the heavy chain of an antibody wherein the numbering system of the constant region is that of the EU index as set forth in Kabat. The heavy chain constant domains (HC Fc) of IgG2, IgG3, and IgG4 are disclosed herein as SEQ ID NOs: 22, 23, and 24, respectively.

In other embodiments, the disclosure comprises the expression of an isolated Fc region comprising engineered residues. Such isolated Fc regions may be useful as scaffolds for display purposes or as dimerization domains alone, or when combined with another agent.

In other embodiments, the disclosure provides fusion proteins comprising Fc regions that contain at least one or more substitutions at positions selected from: 239, 282, 289, 297, 312, 324, 330, 335, 337, 339, 356, 359, 361, 383, 384, 398, 400, 440, 422, and 442 wherein the numbering system of the constant region is that of the EU index as set forth in Kabat, fused to another protein.

In other embodiments, engineered antibodies of the disclosure may further comprise at least one or more non-naturally occurring cysteine amino acids in the 131-139 region of the CH1 domain of an antibody. In some embodiments, the engineered antibodies of the disclosure comprise at least one substitution at positions selected from: 131, 132, 133, 134, 135, 136, 137, 138, and 139 of the CH1 domain of an antibody, wherein the numbering system of the constant region is that of the EU index as set forth in Kabat. Further embodiments can be found in "Cysteine engineered antibodies for site-specific conjugation" corresponding to PCT application PCT/US09/31294, filed January 16, 2009 and U.S. Provisional Application 61/022,073 filed January 18, 2008, each of which is incorporated by reference in its entirety for all purposes.

As used herein, the terms "antibody" and "antibodies", also known as immunoglobulins, encompass any of the following antibodies that comprise an Fc region, including monoclonal antibodies (including full-length monoclonal antibodies), polyclonal antibodies, multispecific antibodies formed from at least two different epitope binding fragments (e.g., bispecific antibodies), human antibodies, humanized antibodies, camelised antibodies, chimeric antibodies, single-chain Fvs (scFv), single-chain antibodies, single domain antibodies, domain antibodies, Fab fragments, F(ab')2 fragments, antibody fragments that exhibit the desired biological activity (e.g. the antigen binding portion), disulfide-linked Fvs (dsFv), and anti-idiotypic (anti-Id) antibodies (including, e.g., anti-Id antibodies to antibodies of the invention), intrabodies, and epitope-binding fragments of any of the above. In particular, antibodies include immunoglobulin molecules and immunologically active fragments of immunoglobulin molecules, i.e., molecules that contain at least one antigen-binding site. Immunoglobulin molecules can be of any isotype (e.g., IgG, IgE, IgM, IgD, IgA and IgY), subisotype (e.g., IgG1, IgG2, IgG3, IgG4, IgA1 and IgA2) or allotype (e.g., Gm, e.g., G1m(f, z, a or x), G2m(n), G3m(g, b, or c), Am, Em, and Km(1, 2 or 3)). Antibodies may be derived from any mammal, including, but not limited to, humans, monkeys, pigs, horses, rabbits, dogs, cats, mice, etc., or other animals such as birds (e.g. chickens). In one embodiment, the antibodies of the disclosure comprise the bispecific antibodies disclosed in U.S. Patent Application Publication No. 20090144275 A1 having any or all of the substitutions disclosed herein. U.S. Patent Application Publication No. 20090144275 A1 in incorporated by reference in its entirety.

### Antibody Affinity

Engineered antibodies of the disclosure retain the antigen binding capability of their native counterpart. In one embodiment, the engineered antibodies of the disclosure exhibit essentially the same affinity as compared to an antibody prior to engineering. In another embodiment, engineered antibodies of the disclosure exhibit a reduced affinity as compared to an antibody prior to engineering. In another embodiment, engineered antibodies of the disclosure exhibit an enhanced affinity as compared to an antibody prior to engineering.

Antibodies of the disclosure may have a high binding affinity to one or more of its cognate antigens. For example, an antibody described herein may have an association rate constant or kₒₙ rate (antibody (Ab) + antigen->Ab-Ag) of at least 2 X 10⁵ M⁻¹s⁻¹, at least 5 X 10⁵ M⁻¹ s⁻¹, at least 10⁶ M⁻¹s⁻¹, at least 5 X 10⁶ M⁻¹s⁻¹, at least 10⁷ M⁻¹s⁻¹, at least 5 X 10⁷ M¹s⁻¹, or at least 10⁸ M⁻¹s⁻¹.

In another embodiment, an antibody of the disclosure may have a k_{off} rate (Ab-Ag -> Ab + Ag) of less than 5x10⁻¹s⁻¹, less than 10⁻¹s⁻¹, less than 5x10⁻²s⁻¹, less than 10⁻²s⁻¹, less than 5x10⁻³s⁻¹, less than 10⁻³s⁻¹, less than 5x10⁻⁴s⁻¹, or less than 10⁻⁴s⁻¹. In a another embodiment, an antibody of the disclosure has a k_{off} of less than 5x10⁻⁵s⁻¹, less than 10⁻⁵s⁻¹, less than 5x10⁻⁶s⁻¹, less than 10⁻⁶s⁻¹, less than 5x10⁻⁷s⁻¹, less than 10⁻⁷s⁻¹, less than 5x10⁻⁸s⁻¹, less than 10⁻⁸s⁻¹, less than 5x10⁻⁹s⁻¹, less than 10⁻⁹s⁻¹, or less than 10⁻¹⁰s⁻¹.

In another embodiment, an antibody of the disclosure may have an affinity constant or Kₐ (kₒₙ/k_{off}) of at least 10² M⁻¹, at least 5 X 10² M⁻¹, at least 10³ M⁻¹, at least 5 X 10³ M⁻¹, at least 10⁴ M⁻¹, at least 5 X 10⁴ M⁻¹, at least 10⁵ M⁻¹, at least 5 X 10⁵ M⁻¹, at least 10⁶ M⁻¹, at least 5 X 10⁶ M⁻¹, at least 10⁷ M⁻¹, at least 5 X 10⁷ M⁻¹, at least 10⁸ M⁻¹, at least 5 X 10⁸ M⁻¹, at least 10⁹ M⁻¹, at least 5 X 10⁹ M⁻¹, at least 10¹⁰ M⁻¹, at least 5 X 10¹⁰ M⁻¹, at least 10¹¹ M⁻¹, at least 5 X 10¹¹ M⁻¹, at least 10¹² M⁻¹, at least 5 X 10¹² M⁻¹, at least 10¹³ M⁻¹, at least 5 X 10¹³ M⁻¹, at least 10¹⁴ M⁻¹, at least 5 X 10¹⁴ M⁻¹, at least 10¹⁵ M⁻¹, or at least 5 X 10¹⁵ M⁻¹. In yet another embodiment, an antibody of the disclosure may have a dissociation constant or K_{d} (k_{off}/kₒₙ) of less than 5x10⁻² M, less than 10⁻² M, less than 5x10⁻³ M, less than 10⁻³ M, less than 5x10⁻⁴ M, less than 10⁻⁴ M, less than 5x10⁻⁵ M, less than 10⁻⁵ M, less than 5x10⁻⁶ M, less than 10⁻⁶ M, less than 5x10⁻⁷ M, less than 10⁻⁷ M, less than 5x10⁻⁸ M, less than 10⁻⁸ M, less than 5x10⁻⁹ M, less than 10⁻⁹ M, less than 5x10⁻¹⁰ M, less than 10⁻¹⁰ M, less than 5x10⁻¹¹ M, less than 10⁻¹¹ M, less than 5x10⁻¹² M, less than 10⁻¹² M, less than 5x10⁻¹³ M, less than 10⁻¹³ M, less than 5x10⁻¹⁴ M, less than 10⁻¹⁴ M, less than 5x10⁻¹⁵ M, or less than 10⁻¹⁵ M.

An antibody used in accordance with a method described herein may have a dissociation constant (K_{d}) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM as assessed using a method described herein or known to one of skill in the art (e.g., a BIAcore assay, ELISA) (Biacore International AB, Uppsala, Sweden).

### Antibody Specificity

In some embodiments, engineered antibodies of the disclosure comprise an antibody that comprises an epitope binding domain (for example, but not limited to, an antibody variable region having all 6 CDRs, or an equivalent region that is at least 90% identical to an antibody variable region) chosen from: abagovomab, abatacept (also know as ORENCIA®), abciximab (also known as REOPRO®, c7E3 Fab), adalimumab (also known as HUMIRA®), adecatumumab, alemtuzumab (also known as CAMPATH®, MabCampath or Campath-1H), altumomab, afelimomab, anatumomab mafenatox, anetumumab, anrukizumab, apolizumab, arcitumomab, aselizumab, atlizumab, atorolimumab, bapineuzumab, basiliximab (also known as SIMULECT®), bavituximab, bectumomab (also known as LYMPHOSCAN®), belimumab (also known as LYMPHO-STAT-B®), bertilimumab, besilesomab, bevacizumab (also known as AVASTIN®), biciromab brallobarbital, bivatuzumab mertansine, campath, canakinumab (also known as ACZ885), cantuzumab mertansine, capromab (also known as PROSTASCINT®), catumaxomab (also known as REMOVAB®), cedelizumab (also known as CIMZIA®), certolizumab pegol, cetuximab (also known as ERBITUX®), clenoliximab, dacetuzumab, dacliximab, daclizumab (also known as ZENAPAX®), denosumab (also known as AMG 162), detumomab, dorlimomab aritox, dorlixizumab, duntumumab, durimulumab, durmulumab, ecromeximab, eculizumab (also known as SOLIRIS®), edobacomab, edrecolomab (also known as Mab17-1A, PANOREX®), efalizumab (also known as RAPTIVA®), efungumab (also known as MYCOGRAB®), elsilimomab, enlimomab pegol, epitumomab cituxetan, efalizumab, epitumomab, epratuzumab, erlizumab, ertumaxomab (also known as REXOMUN®), etanercept (also known as ENBREL®), etaracizumab (also known as etaratuzumab, VITAXIN®, ABEGRIN™), exbivirumab, fanolesomab (also known as NEUTROSPEC®), faralimomab, felvizumab, fontolizumab (also known as HUZAF®), galiximab, gantenerumab, gavilimomab (also known as ABX-CBL®), gemtuzumab ozogamicin (also known as MYLOTARG®), golimumab (also known as CNTO 148), gomiliximab, ibalizumab (also known as TNX-355), ibritumomab tiuxetan (also known as ZEVALIN®), igovomab, imciromab, infliximab (also known as REMICADE®), inolimomab, inotuzumab ozogamicin, ipilimumab (also known as MDX-010, MDX-101), iratumumab, keliximab, labetuzumab, lemalesomab, lebrilizumab, lerdelimumab, lexatumumab (also known as, HGS-ETR2, ETR2-ST01), lexitumumab, libivirumab, lintuzumab, lucatumumab, lumiliximab, mapatumumab (also known as HGS-ETR1, TRM-1), maslimomab, matuzumab (also known as EMD72000), mepolizumab (also known as BOSATRIA®), metelimumab, milatuzumab, minretumomab, mitumomab, morolimumab, motavizumab (also known as NUMAX™), muromonab (also known as OKT3), nacolomab tafenatox, naptumomab estafenatox, natalizumab (also known as TYSABRI®, ANTEGREN®), nebacumab, nerelimomab, nimotuzumab (also known as THERACIM hR3®, THERA-CIM-hR3®, THERALOC®), nofetumomab merpentan (also known as VERLUMA®), ocrelizumab, odulimomab, ofatumumab, omalizumab (also known as XOLAIR®), oregovomab (also known as OVAREX®), otelixizumab, pagibaximab, palivizumab (also known as SYNAGIS®), panitumumab (also known as ABX-EGF, VECTIBIX®), pascolizumab, pemtumomab (also known as THERAGYN®), pertuzumab (also known as 2C4, OMNITARG®), pexelizumab, pintumomab, priliximab, pritumumab, ranibizumab (also known as LUCENTIS®), raxibacumab, regavirumab, reslizumab, rituximab (also known as RITUXAN®, MabTHERA®), rovelizumab, ruplizumab, satumomab, sevirumab, sibrotuzumab, siplizumab (also known as MEDI-507), sontuzumab, stamulumab (also known as MYO-029), sulesomab (also known as LEUKOSCAN®), tacatuzumab tetraxetan, tadocizumab, talizumab, taplitumomab paptox, tefibazumab (also known as AUREXIS®), telimomab aritox, teneliximab, teplizumab, ticilimumab, tocilizumab (also known as ACTEMRA®), toralizumab, tositumomab, trastuzumab (also known as HERCEPTIN®), tremelimumab (also known as CP-675,206), tucotuzumab celmoleukin, tuvirumab, urtoxazumab, ustekinumab (also known as CNTO 1275), vapaliximab, veltuzumab, vepalimomab, visilizumab (also known as NUVION®), volociximab (also known as M200), votumumab (also known as HUMASPECT®), zalutumumab, zanolimumab (also known as HuMAX-CD4), ziralimumab, or zolimomab aritox.

In other embodiments, antibodies of the disclosure comprise a heavy and light chain variable domain having six CDRs, and competes for binding with an antibody selected from the preceding list. In other embodiments, engineered antibodies of the disclosure comprise a heavy and light chain variable domain having six total CDRs, and binds to the same antigen as the antibodies in the proceeding list.

In other embodiments, engineered antibodies of the disclosure comprise a heavy and light chain variable domain having 6 total CDRs, and specifically binds to an antigen selected from: PDGFRalpha, PDGFRbeta, PDGF, VEGF, VEGF-A, VEGF-B, VEGF-C. VEGF-D, VEGF-E, VEGF-F, VEGFR-1, VEGFR-2, VEGFR-3, FGF, FGF2, HGF, KDR, flt-1, FLK-1 Ang-2, Ang-1, PLGF, CEA, CXCL13, Baff, IL-21, CCL21, TNF-alpha, CXCL12, SDF-1, bFGF, MAC-1, IL23p19, FPR, IGFBP4, CXCR3, TLR4, CXCR2, EphA2, EphA4, EphrinB2, EGFR(ErbB1), HER2(ErbB2 or p185neu), HER3(ErbB3), HER4 ErbB4 or tyro2), SC1, LRP5, LRP6, RAGE, Nav1.7, GLP1, RSV, RSV F protein, Influenza HA protein, Influenza NA protein, HMGB1, CD16, CD19, CD20, CD21, CD28, CD32, CD32b, CD64, CD79, CD22, ICAM-1, FGFR1, FGFR2, HDGF, EphB4, GITR, β-amyloid, hMPV, PIV-1, PIV-2, OX40L, IGFBP3, cMet, PD-1, PLGF, Neprolysin, CTD, IL-18, IL-6, CXCL-13, IL-1R1, IL-15, IL-4R, IgE, PAI-1, NGF, EphA2, uPARt, DLL-4, αvβ6, α5β1, interferon receptor type I and type II. CD19, ICOS, IL-17, Factor II, Hsp90, IGF, IGF-I, IGF-II, CD19, GM-CSFR, PIV-3, CMV, IL-13, IL-9, and EBV.

In other embodiments, the engineered antibodies of the disclosure specifically bind to a member (receptor or ligand) of the TNF superfamily. Various molecules include, but are not limited to Tumor Necrosis Factor-alpha ("TNF-alpha"), Tumor Necrosis Factor-beta ("TNF-beta"), Lymphotoxin-alpha ("LT-alpha"), CD30 ligand, CD27 ligand, CD40 ligand, 4-1 BB ligand, Apo-1 ligand (also referred to as Fas ligand or CD95 ligand), Apo-2 ligand (also referred to as TRAIL), Apo-3 ligand (also referred to as TWEAK), osteoprotegerin (OPG), APRIL, RANK ligand (also referred to as TRANCE), TALL-1 (also referred to as BlyS, BAFF or THANK), DR4, DR5 (also known as Apo-2, TRAIL-R2, TR6, Tango-63, hAPO8, TRICK2, or KILLER), DR6, DcR1,DcR2, DcR3 (also known as TR6 or M68), CAR1, HVEM (also known as ATAR or TR2), GITR, ZTNFR-5, NTR-1, TNFL1, CD30, LTBr, 4-1BB receptor and TR9.

In another embodiment the engineered antibodies of the disclosure are capable of binding one or more targets chosen from 5T4, ABL, ABCFI, ACVR1, ACVR1B, ACVR2, ACVR2B, ACVRL1, ADORA2A, Aggrecan, AGR2, AICDA, AIFI, AIG1, AKAP1, AKAP2, AMH, AMHR2, ANGPT1, ANGPT2, ANGPTL3, ANGPTL4, ANPEP, APC, APOC1, AR, aromatase, ATX, AX1, AZGP1 (zinc-a-glycoprotein), B7.1, B7.2, B7-H1, BAD, BAFF, BAG1, BAI1, BCR, BCL2, BCL6, BDNF, BLNK, BLR1 (MDR15), BIyS, BMP1, BMP2, BMP3B (GDFIO), BMP4, BMP6, BMP8, BMPR1A, BMPR1B, BMPR2, BPAG1 (plectin), BRCA1, C19orflO (IL27w), C3, C4A, C5, C5R1, CANT1, CASP1, CASP4, CAVI, CCBP2 (D6 / JAB61), CCL1 (1-309), CCLI 1 (eotaxin), CCL13 (MCP-4), CCL15 (MIP-Id), CCL16 (HCC-4), CCL 17 (TARC), CCL18 (PARC), CCL 19 (MIP-3b), CCL2 (MCP -1), MCAF, CCL20 (MIP-3a), CCL21 (MEP-2), SLC, exodus-2, CCL22(MDC / STC-I), CCL23 (MPIF-I), CCL24 (MPIF-2 / eotaxin-2), CCL25 (TECK), CCL26(eotaxin-3), CCL27 (CTACK / ILC), CCL28, CCL3 (MIP-Ia), CCL4 (MIP-Ib), CCL5(RANTES), CCL7 (MCP-3), CCL8 (mcp-2), CCNA1, CCNA2, CCNDI, CCNE1, CCNE2, CCR1 (CKR1 / HM145), CCR2 (mcp-1RB / RA),CCR3 (CKR3 / CMKBR3), CCR4, CCR5(CMKBR5 / ChemR13), CCR6 (CMKBR6 / CKR-L3 / STRL22 / DRY6), CCR7 (CKR7 / EBI1),CCR8 (CMKBR8 / TER1 / CKR-Ll), CCR9 (GPR-9-6), CCRL1 (VSHK1), CCRL2 (L-CCR),CD164, CD19, CD1C, CD20, CD200, CD-22, CD24, CD28, CD3, CD33, CD35, CD37, CD38, CD3E, CD3G,CD3Z, CD4, CD40, CD40L, CD44, CD45RB, CD52, CD69, CD72, CD74, CD79A, CD79B, CD8, CD80, CD81, CD83, CD86, CD137, CDH1 (E-cadherin), CDH1O, CDH12, CDH13, CDH18,CDH19, CDH20, CDH5, CDH7, CDH8, CDH9, CDK2, CDK3, CDK4, CDK5, CDK6, CDK7,CDK9, CDKN1A (p21Wap1/Cip1), CDKN1B (p27Kip1), CDKN1C, CDKN2A (p16INK4a), CDKN2B, CDKN2C, CDKN3, CEBPB, CER1, CHGA, CHGB, Chitinase, CHSTlO, CKLFSF2,CKLFSF3, CKLFSF4, CKLFSF5, CKLFSF6, CKLFSF7, CKLFSF8, CLDN3, CLDN7 (claudin- 7), CLN3, CLU (clusterin), CMKLR1, CMKOR1 (RDC1), CNR1, COL18A1, COL1A1, COL4A3, COL6A1, CR2, Cripto, CRP, CSF1 (M-CSF), CSF2 (GM-CSF), CSF3 (GCSF), CTLA4, CTL8, CTNNB1 (b-catenin), CTSB (cathepsin B), CX3CL1 (SCYD1), CX3CR1 (V28), CXCL1(GRO1), CXCLlO (IP-IO), CXCLI1 (I-TAC / IP-9), CXCL12 (SDFI), CXCL13, CXCL14,CXCL16, CXCL2 (GRO2), CXCL3 (GRO3), CXCL5 (ENA-78 / LIX), CXCL6 (GCP-2), CXCL9 (MIG), CXCR3 (GPR9/CKR-L2), CXCR4, CXCR6 (TYMSTR /STRL33 / Bonzo),CYB5, CYC1, CYSLTR1, DAB2IP, DES, DKFZp451J0118, DNCL1, DPP4, E2F1, ECGF1,EDG1, EFNA1, EFNA3, EFNB2, EGF, EGFR, ELAC2, ENG, ENO1, ENO2, ENO3, EPHA1, EPHA2, EPHA3, EPHA4, EPHA5, EPHA6, EPHA7, EPHA8, EPHA9, EPHA10, EPHB 1, EPHB2, EPHB3, EPHB4, EPHB5, EPHB6, EPHRIN-A1, EPHRIN-A2, EPHRIN-A3, EPHRIN-A4, EPHRIN-A5, EPHRIN-A6, EPHRIN-B1, EPHRIN-B2, EPHRIN-B3, EPHB4,EPG, ERBB2 (Her-2), EREG, ERK8, Estrogen receptor, ESR1, ESR2, F3 (TF), FADD, farnesyltransferase, FasL, FASNf, FCER1A,FCER2, FCGR3A, FGF, FGF1 (aFGF), FGFlO, FGFl1, FGF12, FGF12B, FGF13, FGF 14, FGF 16, FGF 17, FGF 18, FGF 19, FGF2 (bFGF), FGF20, FGF21 , FGF22, FGF23, FGF3 (int-2),FGF4 (HST), FGF5, FGF6 (HST-2), FGF7 (KGF), FGF8, FGF9, FGFR3, FIGF (VEGFD), FILI(EPSILON), FBL1 (ZETA), FLJ12584, FLJ25530, FLRTI (fibronectin), FLT1, FLT-3, FOS, FOSL1(FRA-I), FY (DARC), GABRP (GABAa), GAGEBI, GAGECI, GALNAC4S-6ST, GATA3, GD2, GDF5, GFIl, GGT1, GM-CSF, GNAS1, GNRHl, GPR2 (CCRlO), GPR31, GPR44, GPR81 (FKSG80), GRCClO (ClO), GRP, GSN (Gelsolin), GSTP1, HAVCR2, HDAC, HDAC4, HDAC5,HDAC7A, HDAC9, Hedgehog, HGF, HIFlA, HIP1, histamine and histamine receptors, HLA-A, HLA-DRA, HM74, HMOX1, HSP90, HUMCYT2A, ICEBERG, ICOSL, ID2, IFN-a, IFNA1, IFNA2, IFNA4,IFNA5, EFNA6, BFNA7, IFNBI, IFNgamma, IFNWI, IGBPI, IGF1, IGF1R, IGF2, IGFBP2,IGFBP3, IGFBP6, DL-I, ILlO, IL1ORA, IL1ORB, IL-1, IL1R1 (CD121a), IL1R2(CD121b), IL-1RA, IL-2, IL2RA (CD25), IL2RB(CD122), IL2RG(CD132), IL-4, IL-4R(CD123), IL-5, IL5RA(CD125), IL3RB(CD131), IL-6, IL6RA (CD126), IR6RB(CD130), IL-7, IL7RA(CD127), IL-8, CXCR1 (IL8RA), CXCR2 (IL8RB/CD128), IL-9, IL9R (CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, 1L16, IL17, IL17A, IL17B, IL17C, IL17R, IL18, IL18BP, IL18R1, IL18RAP, IL19, ILIA, IL1B, IL1F10, IL1F5, IL1F6, IL1F7, IL1F8, DL1F9, IL1HY1, IL1R1, IL1R2, IL1RAP, IL1RAPL1, IL1RAPL2, IL1RL1, IL1RL2, IL1RN, IL2, IL20, IL20RA, IL21R, IL22, IL22R, IL22RA2, IL23,DL24, IL25, IL26, IL27, IL28A, IL28B, IL29, IL2RA, IL2RB, IL2RG, IL3, IL30, IL3RA, IL4,IL4R, IL6ST (glycoprotein 130), ILK, INHA, INHBA, INSL3, INSL4, IRAKI, IRAK2, ITGAI, ITGA2,ITGA3, ITGA6 (α6 integrin), ITGAV, ITGB3, ITGB4 (β4 integrin), JAG1, JAK1, JAK3, JTB, JUN,K6HF, KAI1, KDR, KITLG, KLF5 (GC Box BP), KLF6, KLKlO, KLK12, KLK 13, KLK 14, KLK 15, KLK3, KLK4, KLK5, KLK6, KLK9, KRT1, KRT 19 (Keratin 19), KRT2A, KRTHB6(hair-specific type II keratin), LAMA5, LEP (leptin), Lingo-p75, Lingo-Troy, LPS, LTA (TNF- b), LTB, LTB4R (GPR16), LTB4R2, LTBR, MACMARCKS, MAG or Omgp, MAP2K7 (c-Jun), MCP-1, MDK, MIBI, midkine, MIF, MISRII, MJP-2,MK, MKI67 (Ki-67), MMP2, MMP9, MS4A1, MSMB,MT3 (metallothionectin-UI), mTOR, MTSS1, MUCI (mucin), MYC, MYD88, NCK2, neurocan,NFKB1, NFKB2, NGFB (NGF), NGFR, NgR-Lingo, NgR-Nogo66 (Nogo), NgR-p75, NgR-Troy, NMEI (NM23A), NOTCH, NOTCH 1, NOX5, NPPB, NROB1, NR0B2, NR1D1, NR1D2, NR1H2, NR1H3, NR1H4, NR1I2, NR1I3, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4,ODZ1, OPRD1, P2RX7, PAP, PART1, PATE, PAWR, PCA3, PCDGF, PCNA, PDGFA, PDGFB, PDGFRA, PDGFRB, PECAM1, peg-asparaginase, PF4 (CXCL4), PGF, PGR, phosphacan, PIAS2, PI3 Kinase, PIK3CG, PLAU (uPA), PLG,PLXDC1, PKC, PKC-beta, PPBP (CXCL7), PPID, PRI, PRKCQ, PRKD1, PRL, PROC, PROK2, PSAP, PSCA, PTAFR, PTEN, PTGS2 (COX-2), PTN, RAC2 (P21Rac2), RANK, RANK ligand, RARB, RGS1, RGS13, RGS3,RNFIlO (ZNF144), Ron, ROBO2, RXR, S100A2, SCGB 1D2 (lipophilin B), SCGB2A1 (mammaglobin 2),SCGB2A2 (mammaglobin 1), SCYEI (endothelial Monocyte-activating cytokine), SDF2,SERPENA1, SERPINA3, SERPINB5 (maspin), SERPINE1 (PAI-I), SERPINFI, SHIP-1, SHIP-2, SHB1, SHB2, SHBG, SfcAZ,SLC2A2, SLC33A1, SLC43A1, SLIT2, SPPI, SPRR1B (Sprl), ST6GAL1, STAB1, STAT6, STEAP, STEAP2, TB4R2, TBX21, TCPlO, TDGFI, TEK, TGFA, TGFB1, TGFBlI1, TGFB2,TGFB3, TGFBI, TGFBR1, TGFBR2, TGFBR3, THlL, THBS1 (thrombospondin-1), THBS2,THBS4, THPO, TIE (Tie-1), TIMP3, tissue factor, TLRlO, TLR2, TLR3, TLR4, TLR5, TLR6,TLR7, TLR8, TLR9, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSFlO (TRAIL), TNFSFl 1 (TRANCE), TNFSF 12 (APO3L), TNFSF 13 (April), TNFSF 13B,TNFSF 14 (HVEM-L), TNFSF 15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Iia), TP53, TPM1, TPM2,TRADD, TRAF1, TRAF2, TRAF3, TRAF4, TRAF5, TRAF6, TRKA, TREM1, TREM2, TRPC6, TSLP, TWEAK, Tyrosinase, uPAR, VEGF, VEGFB, VEGFC, versican, VHL C5, VLA-4, Wnt-1, XCL1 (lymphotactin),XCL2 (SCM-Ib), XCR1 (GPR5 / CCXCR1), YY1, and ZFPM2.

### Further alteration of the Fc region

The disclosure also provides engineered antibodies with further altered Fc regions that can be combined with any or all of the variant Fc regions described above. It is known that variants of the Fc region (*e.g*., amino acid substitutions, insertions, and/or additions and/or deletions) enhance or diminish effector function (see Presta et al., 2002, Biochem Soc Trans 30:487-490; U.S. patents 5,624,821, 5,885,573 and PCT publication Nos. WO 00/42072, WO 99/58572 and WO 04/029207). In one embodiment, the variant Fc regions of antibodies exhibit a similar level of inducing effector function as compared to the native Fc. In another embodiment, the variant Fc region exhibits a higher induction of effector function as compared to the native Fc. In another embodiment, the variant Fc region exhibits lower induction of effector function as compared to the native Fc. In another embodiment, the variant Fc region exhibits higher induction of ADCC as compared to the native Fc. In another embodiment, the variant Fc region exhibits lower induction of ADCC as compared to the native Fc. In another embodiment, the variant Fc region exhibits higher induction of CDC as compared to the native Fc. In another embodiment, the variant Fc region exhibits lower induction of CDC as compared to the native Fc. Specific embodiments of variant Fc regions are detailed *infra.*

It is also known that the glycosylation of the Fc region can be modified to increase or decrease effector function (see for examples, Umana et al, 1999, Nat. Biotechnol 17:176-180; Davies et al., 2001, Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potillegent™ technology (Biowa, Inc. Princeton, N.J.); GlycoMAb™ glycosylation engineering technology (GLYCART biotechnology AG, Zurich, Switzerland).

Accordingly, in one embodiment, the Fc regions of antibodies of the disclosure comprise altered glycosylation of amino acid residues. In another embodiment, the altered glycosylation of the amino acid residues results in lowered effector function. In another embodiment, the altered glycosylation of the amino acid residues results in increased effector function. In a specific embodiment, the Fc region has reduced fucosylation. In another embodiment, the Fc region is afucosylated (see for examples, U.S. Patent Application Publication No.2005/0226867).

Recent research suggests that addition of sialic acid to the oligosaccharides on IgG molecules enhances their anti-inflammatory activity and alters their cytotoxicity (Keneko et al., Science 313, 670-673(2006), Scallon et al., Mol. Immuno. 2007 Mar;44(7):1524-34). Thus, the efficacy of antibody therapeutics may be optimized by selection of a glycoform that is suited to the intended application. The two oligosaccharide chains interposed between the two CH2 domains of antibodies are involved in the binding of the Fc region to its receptors. The studies referenced above demonstrate that IgG molecules with increased sialylation have anti-inflammatory properties whereas IgG molecules with reduced sialylation have increased immunostimulatory properties. Therefore, an antibody therapeutic can be "tailor-made" with an appropriate sialylation profile for a particular application. Methods for modulating the sialylation state of antibodies are presented in WO2007/005786 entitled "Methods And Compositions With Enhanced Therapeutic Activity", and W02007/117505 entitled "Polypeptides With Enhanced Anti-Inflammatory And Decreased Cytotoxic Properties And Related Methods" each of which are incorporated by reference in their entireties for all purposes.

In one embodiment, the Fc regions of antibodies of the disclosure comprise an altered sialylation profile compared to a reference unaltered Fc region. In one embodiment, the Fc regions of antibodies of the disclosure comprise an increased sialylation profile compared to a reference unaltered Fc region. In some embodiments the Fc regions of antibodies of the disclosure comprise an increase in sialylation of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150% or more as compared to a reference unaltered Fc region. In some embodiments the Fc regions of antibodies of the disclosure comprise an increase in sialylation of about 2 fold, about 3 fold, about 4 fold, about 5 fold, about 10 fold, about 20 fold, about 50 fold or more as compared to an unaltered reference Fc region.

In another embodiment, the Fc regions of antibodies of the disclosure comprise a decreased sialylation profile compared to a reference unaltered Fc region. In some embodiments, the Fc regions of antibodies of the disclosure comprise a decrease in sialylation of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of antibodies of the disclosure comprise a decrease in sialylation of about 2 fold, about 3 fold, about 4 fold, about 5 fold, about 10 fold, about 20 fold, about 50 fold or more as compared to an unaltered reference Fc region.

It is also known in the art that the Fc region can be modified to increase the half-lives of proteins. The increase in half-life allows for the reduction in amount of drug given to a patient as well as reducing the frequency of administration. Accordingly, antibodies of the disclosure with increased half-lives may be generated by modifying (for example, substituting, deleting, or adding) amino acid residues identified as involved in the interaction between the Fc and the FcRn receptor (see, for examples, PCT publication Nos. 97/34631 and 02/060919 each of which are incorporated by reference in their entireties). In addition, the half-life of antibodies of the disclosure may be increase by conjugation to PEG or Albumin by techniques widely utilized in the art. In some embodiments the Fc regions of antibodies of the disclosure comprise an increase in half-life of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150% or more as compared to a reference unaltered Fc region. In some embodiments the Fc regions of antibodies of the disclosure comprise an increase in half-life of about 2 fold, about 3 fold, about 4 fold, about 5 fold, about 10 fold, about 20 fold, about 50 fold or more as compared to an unaltered reference Fc region.

In an alternate embodiment, the Fc regions of antibodies of the disclosure comprise a decrease in half-life. In some embodiments the Fc regions of antibodies of the disclosure comprise a decrease in half-life of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150% or more as compared to a reference unaltered Fc region. In some embodiments the Fc regions of antibodies of the disclosure comprise a decrease in half-life of about 2 fold, about 3 fold, about 4 fold, about 5 fold, about 10 fold, about 20 fold, about 50 fold or more as compared to an unaltered reference Fc region.

The present disclosure encompasses Fc variant proteins which have altered binding properties for an Fc ligand (e.g., an Fc receptor, Clq) relative to a comparable molecule (e.g., a protein having the same amino acid sequence except having a native Fc region). Examples of binding properties include but are not limited to, binding specificity, equilibrium dissociation constant (K_{D}), dissociation and association rates (k_{off} and kₒₙ respectively), binding affinity and/or avidity. It is generally understood that a binding molecule (e.g., a Fc variant protein such as an antibody) with a low K_{D} may be preferable to a binding molecule with a high K_{D}. However, in some instances the value of the kₒₙ or k_{off} may be more relevant than the value of the K_{D}. One skilled in the art can determine which kinetic parameter is most important for a given antibody application.

The affinities and binding properties of an Fc region for its ligand may be determined by a variety of in vitro assay methods (biochemical or immunological based assays) known in the art for determining Fc-FcγR interactions, i.e., specific binding of an Fc region to an FcγR including but not limited to, equilibrium methods (e.g., enzyme-linked immunoabsorbent assay (ELISA), or radioimmunoassay (RIA)), or kinetics (e.g., BIACORE® analysis), and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions.

In one embodiment, the Fc variant protein has enhanced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. In a specific embodiment, the Fc variant protein has enhanced binding to an Fc receptor. In another specific embodiment, the Fc variant protein has enhanced binding to the Fc receptor FcγRIIIA. In a further specific embodiment, the Fc variant protein has enhanced binding to the Fc receptor FcγRIIB. In still another specific embodiment, the Fc variant protein has enhanced binding to the Fc receptor FcRn. In yet another specific embodiment, the Fc variant protein has enhanced binding to C1q relative to a comparable molecule.

The ability of any particular Fc variant protein to mediate lysis of the target cell by ADCC can be assayed. To assess ADCC activity an Fc variant protein of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985 79:277-282; Bruggemann et al., 1987, J Exp Med 166:1351-1361; Wilkinson et al., 2001, J Immunol Methods 258:183-191; Patel et al., 1995 J Immunol Methods 184:29-38. ADCC activity of the Fc variant protein of interest may also be assessed in vivo, e.g., in an animal model such as that disclosed in Clynes et al., 1998, Proc. Natl. Acad. Sci. USA 95:652-656.

In one embodiment, an Fc variant protein has enhanced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold greater than that of a comparable molecule. In another specific embodiment, an Fc variant protein has enhanced binding to the Fc receptor FcγRIIIA and has enhanced ADCC activity relative to a comparable molecule. In other embodiments, the Fc variant protein has both enhanced ADCC activity and enhanced serum half life relative to a comparable molecule.

In one embodiment, an Fc variant protein has reduced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold lower than that of a comparable molecule. In another specific embodiment, an Fc variant protein has reduced binding to the Fc receptor FcγRIIIA and has reduced ADCC activity relative to a comparable molecule. In other embodiments, the Fc variant protein has both reduced ADCC activity and enhanced serum half life relative to a comparable molecule.

In one embodiment, an Fc variant protein has enhanced CDC activity relative to a comparable molecule. In a specific embodiment, an Fc variant protein has CDC activity that is at least 2 fold, or at least 3 fold, or at least 5 fold or at least 10 fold or at least 50 fold or at least 100 fold greater than that of a comparable molecule. In other embodiments, the Fc variant protein has both enhanced CDC activity and enhanced serum half life relative to a comparable molecule. In one embodiment, the Fc variant protein has reduced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold lower than that of a comparable molecule. In a specific embodiment, the Fc variant protein has reduced binding to an Fc receptor. In another specific embodiment, the Fc variant protein has reduced binding to the Fc receptor FcγRIIIA. In a further specific embodiment, an Fc variant described herein has an affinity for the Fc receptor FcγRIIIA that is at least about 5 fold lower than that of a comparable molecule, wherein said Fc variant has an affinity for the Fc receptor FcγRIIB that is within about 2 fold of that of a comparable molecule. In still another specific embodiment, the Fc variant protein has reduced binding to the Fc receptor FcRn. In yet another specific embodiment, the Fc variant protein has reduced binding to C1q relative to a comparable molecule.

In one embodiment, the present disclosure provides Fc variants, wherein the Fc region further comprises a non naturally occurring amino acid residue (in addition to or other than, the substitutions disclosed above) at one or more positions chosen from 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 and 443 as numbered by the EU index as set forth in Kabat. Optionally, the Fc region may comprise a non naturally occurring amino acid residue at additional and/or alternative positions known to one skilled in the art (see, e.g., U.S. Patents 5,624,821; 6,277,375; 6,737,056; 7,217,797, U.S. Patent Publication No. US2007/0135620; PCT Patent Publications WO 01/58957; WO 02/06919; WO 04/016750; WO 04/029207; WO 04/035752; WO 04/074455; WO 04/099249; WO 04/063351; WO 05/070963; WO 05/040217, WO 05/092925 and WO 06/020114, each of which is incorporated by reference in its entirety.).

In a specific embodiment, the present disclosure provides an Fc variant, wherein the Fc region comprises at least one non naturally occurring amino acid residue chosen from 234D, 234E, 234N, 234Q, 234T, 234H, 234Y, 234I, 234V, 234F, 235A, 235D, 235R, 235W, 235P, 235S, 235N, 235Q, 235T, 235H, 235Y, 235I, 235V, 235F, 236E, 239D, 239E, 239N, 239Q, 239F, 239T, 239H, 239Y, 240I, 240A, 240T, 240M, 241W, 241 L, 241Y, 241E, 241R, 243W, 243L 243Y, 243R, 243Q, 244H, 245A, 247L, 247V, 247G, 251F, 252Y, 254T, 255L, 256E, 256M, 262I, 262A, 262T, 262E, 263I, 263A, 263T, 263M, 264L, 264I, 264W, 264T, 264R, 264F, 264M, 264Y, 264E, 265G, 265N, 265Q, 265Y, 265F, 265V, 265I, 265L, 265H, 265T, 266I, 266A, 266T, 266M, 267Q, 267L, 268E, 269H, 269Y, 269F, 269R, 270E, 280A, 284M, 292P, 292L, 296E, 296Q, 296D, 296N, 296S, 296T, 296L, 296I, 296H, 269G, 297S, 297D, 297E, 298H, 298I, 298T, 298F, 299I, 299L, 299A, 299S, 299V, 299H, 299F, 299E, 305I, 313F, 316D, 325Q, 325L, 325I, 325D, 325E, 325A, 325T, 325V, 325H, 327G, 327W, 327N, 327L, 328S, 328M, 328D, 328E, 328N, 328Q, 328F, 328I, 328V, 328T, 328H, 328A, 329F, 329H, 329Q, 330K, 330G, 330T, 330C, 330L, 330Y, 330V, 330I, 330F, 330R, 330H, 331G, 331A, 331L, 331M, 331F, 331W, 331K, 331Q, 331E, 331S, 331V, 331I, 331C, 331Y, 331H, 331R, 331N, 331D, 331T, 332D, 332S, 332W, 332F, 332E, 332N, 332Q, 332T, 332H, 332Y, 332A, 339T, 370E, 370N, 378D, 392T, 396L, 416G, 419H, 421K, 440Yand 443W as numbered by the EU index as set forth in Kabat. Optionally, the Fc region may comprise additional and/or alternative non naturally occurring amino acid residues known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

In a specific embodiment, the present disclosure provides an Fc variant antibody, wherein the Fc region comprises at least one modification e.g., amino acid substitutions, amino acid insertions, amino acid deletions, amino acid additions) at one or more positions chosen from 234, 235 and 331. In one embodiment, the non-naturally occurring amino acids are chosen from 234F, 235F, 235Y, and 331S. In another specific embodiment, the present disclosure provides an Fc variant, wherein the Fc region comprises at least one non-naturally occurring amino acid at one or more positions chosen from 239, 330 and 332. In one embodiment, the non-naturally occurring amino acids are selected from the group chosen from 239D, 330L and 332E.

In a specific embodiment, the present disclosure provides an Fc variant antibody, wherein the Fc region comprises at least one non-naturally occurring amino acid at one or more positions chosen from 252, 254, and 256. In one embodiment, the non-naturally occurring amino acids are selected from the group chosen from 252Y, 254T and 256E (referred to as the "YTE modification"), as described in Dall'Acqua et al., J. Biol. Chem., 281,23514-23524 (2006), and in U.S. Patent No. 7,083,784, both of which are incorporated herein by reference in their entireties.

### Methods of Producing Antibodies

The engineered antibodies of the disclosure may be produced by any method known in the art for the synthesis of antibodies, in particular, by chemical synthesis or preferably, by recombinant expression techniques.

Monoclonal antibodies can be prepared using a wide variety of techniques known in the art including the use of hybridoma, recombinant, and phage display technologies, or a combination thereof For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with a target antigen (either the full length protein or a domain thereof, *e.g.,* the extracellular domain or the ligand binding domain) and once an immune response is detected, *e.g.,* antibodies specific for the target antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. Hybridoma clones are then assayed by methods known in the art for cells that secrete antibodies capable of binding a polypeptide of the disclosure. Ascites fluid, which generally contains high levels of antibodies, can be generated by immunizing mice with positive hybridoma clones.

Accordingly, monoclonal antibodies can be generated by culturing a hybridoma cell secreting an antibody of the disclosure wherein, preferably, the hybridoma is generated by fusing splenocytes isolated from a mouse immunized with a target antigen with myeloma cells and then screening the hybridomas resulting from the fusion for hybridoma clones that secrete an antibody able to bind to a specific target antigen.

Antibody fragments which recognize specific target antigen epitopes may be generated by any technique known to those of skill in the art. For example, Fab and F(ab')2 fragments of the disclosure may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies of the present disclosure can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (*e.g.,* human or murine cDNA libraries of lymphoid tissues). The DNA encoding the VH and VL domains are recombined together with an scFv linker by PCR and cloned into a phagemid vector (*e.g.,* pCANTAB 6 or pComb 3 HSS). The vector is electroporated in E. *coli* and the *E. coli* is infected with helper phage. Phage used in these methods are typically filamentous phage including fd and M13 and the VH and VL domains are usually recombinantly fused to either the phage gene III or gene VIII. Phage expressing an antigen binding domain that binds to an epitope of interest can be selected or identified with antigen, *e.g.*, using labeled antigen or antigen bound or captured to a solid surface or bead. Examples of phage display methods that can be used to make the antibodies of the present disclosure include those disclosed in Brinkman et al., 1995, J. Immunol. Methods 182:41-50; Ames et al., 1995, J. Immunol. Methods 184:177; Kettleborough et al., 1994, Eur. J. Immunol. 24:952-958; Persic et al., 1997, Gene 187:9; Burton et al., 1994, Advances in Immunology 57:191-280; International Application No. PCT/GB91/01134; International Publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.,* as described below. Techniques to recombinantly produce Fab, Fab' and F(ab')2 fragments can also be employed using methods known in the art such as those disclosed in International Publication No. WO 92/22324; Mullinax et al., 1992, BioTechniques 12:864; Sawai et al., 1995, AJRI 34:26; and Better et al., 1988, Science 240:1041 (said references incorporated by reference in their entireties).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g.,* the human gamma 4 constant region, and the PCR amplified VL domains can be cloned into vectors expressing a VL constant region, *e.g.,* human kappa or lambda constant regions. Preferably, the vectors for expressing the VH or VL domains comprise an EF-1α promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, *e.g.,* IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Patent Nos. 4,444,887 and 4,716,111; and International Publication Nos. WO 98/46645, WO 98/50433, WO 98/24893, WO 98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the J_{H} region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, *e.g.*, all or a portion of a polypeptide of the disclosure. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* International Publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, which are incorporated by reference herein in their entirety. In addition, companies such as Medarex (Princeton, NJ) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human antibody and a human immunoglobulin constant region. Methods for producing chimeric antibodies are known in the art. See *e.g.,* Morrison, 1985, Science 229:1202; Oi et al., 1986, BioTechniques 4:214; Gillies et al., 1989, J. Immunol. Methods 125:191-202; and U.S. Patent Nos. 6,311,415, 5,807,715, 4,816,567, and 4,816,397, which are incorporated herein by reference in their entirety. Chimeric antibodies comprising one or more CDRs from a non-human species and framework regions from a human immunoglobulin molecule can be produced using a variety of techniques known in the art including, for example, CDR-grafting (EP 239,400; International Publication No. WO 91/09967; and U.S. Patent Nos. 5,225,539, 5,530,101, and 5,585,089), veneering or resurfacing (EP 592,106; EP 519,596; Padlan, 1991, Molecular Immunology 28(4/5):489-498; Studnicka et al., 1994, Protein Engineering 7:805; and Roguska et al., 1994, PNAS 91:969), and chain shuffling (U.S. Patent No. 5,565,332).

Often, framework residues in the framework regions will be substituted with the corresponding residue from the CDR donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (See, *e.g.,* U.S. Patent No. 5,585,089; and Riechmann et al., 1988, Nature 332:323, which are incorporated herein by reference in their entireties.).

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immunoglobulin. A humanized antibody comprises substantially all of at least one, and typically two, variable domains in which all or substantially all of the CDR regions correspond to those of a non-human immunoglobulin (*i.e*., donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including IgG₁, IgG₂, IgG₃ and IgG₄. Usually the constant domain is a complement fixing constant domain where it is desired that the humanized antibody exhibit cytotoxic activity, and the class is typically IgG₁. Where such cytotoxic activity is not desirable, the constant domain may be of the IgG₂ class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, *e.g.*, the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, or even greater than 95%.

Humanized antibodies can be produced using variety of techniques known in the art, including but not limited to, CDR-grafting (European Patent No. EP 239,400; International Publication No. WO 91/09967; and U.S. Patent Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973), chain shuffling (U.S. Patent No. 5,565,332), and techniques disclosed in, *e.g.,* U.S. Patent Nos. 6,407,213, 5,766,886, 5,585,089, International Publication No. WO 9317105, Tan et al., 2002, J. Immunol. 169:1119-25, Caldas et al., 2000, Protein Eng. 13:353-60, Morea et al., 2000, Methods 20:267-79, Baca et al., 1997, J. Biol. Chem. 272:10678-84, Roguska et al., 1996, Protein Eng. 9:895-904, Couto et al., 1995, Cancer Res. 55 (23 Supp):5973s-5977s, Couto et al., 1995, Cancer Res. 55:1717-22, Sandhu, 1994, Gene 150:409-10, Pedersen et al., 1994, J. Mol. Biol. 235:959-73, Jones et al., 1986, Nature 321:522-525, Riechmann et al., 1988, Nature 332:323, and Presta, 1992, Curr. Op. Struct. Biol. 2:593-596. Often, framework residues in the framework regions will be substituted with the corresponding residue from the CDR donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (*See, e.g.,* Queen et al., U.S. Patent No. 5,585,089; and Riechmann et al., 1988, Nature 332:323, which are incorporated herein by reference in their entireties.).

Further, the antibodies of the disclosure can, in turn, be utilized to generate anti-idiotype antibodies using techniques well known to those skilled in the art. (See, *e.g.,* Greenspan & Bona, 1989, FASEB J. 7:437-444; and Nissinoff, 1991, J. Immunol. 147:2429-2438). The disclosure provides methods employing the use of polynucleotides comprising a nucleotide sequence encoding an antibody of the disclosure or a fragment thereof.

Additionally, various publications describe methods for obtaining physiologically active molecules whose half-lives are modified either by introducing an FcRn-binding polypeptide into the molecules (WO 97/43316; U.S. Pat. No. 5,869,046; U.S. Pat. No. 5,747,035; WO 96/32478; WO 91/14438) or by fusing the molecules with antibodies whose FcRn-binding affinities are preserved but affinities for other Fc receptors have been greatly reduced (WO 99/43713) or fusing with FcRn binding domains of antibodies (WO 00/09560; U.S. Pat. No. 4,703,039). Specific techniques and methods of increasing half-life of physiologically active molecules can also be found in U.S. Patent No. 7,083,784 granted Aug 1, 2006 entitled "Antibodies with Increased Half-lives" which is hereby incorporated by reference for all purposes. Specifically, it is contemplated that the antibodies of the disclosure comprise an Fc region comprising amino acid residue mutations (as numbered by the EU index in Kabat): M252Y/S254T/T256E or H433K/N434F/Y436H.

### Polynucleotides Encoding an Antibody

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, *i.e.,* nucleotide codons known to encode particular amino acids are assembled in such a way to generate a nucleic acid that encodes the antibody or fragment thereof of the disclosure. Such a polynucleotide encoding the antibody may be assembled from chemically synthesized oligonucleotides (*e.g.,* as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody is known, a nucleic acid encoding the immunoglobulin may be chemically synthesized or obtained from a suitable source (*e.g*., an antibody cDNA library, or a cDNA library generated from, or nucleic acid, preferably poly A+ RNA, isolated from, any tissue or cells expressing the antibody by PCR amplification using synthetic primers hybridizable to the 3' and 5' ends of the sequence or by cloning using an oligonucleotide probe specific for the particular gene sequence to identify, *e.g.,* a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, *e.g.,* recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, *e.g.,* Chothia et al., 1998, J. Mol. Biol. 278: 457-479 for a listing of human framework regions). Preferably, the polynucleotide generated by the combination of the framework regions and CDRs encodes an antibody that specifically binds to EphA2 or EphA4. Preferably, as discussed *supra,* one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibodies lacking one or more intrachain disulfide bonds. Other alterations to the polynucleotide are encompassed by the present disclosure and within the skill of the art.

### Recombinant Expression of an Antibody

Recombinant expression of an antibody of the disclosure, derivative, analog or fragment thereof, requires construction of an expression vector containing a polynucleotide that encodes the antibody. Once a polynucleotide encoding an antibody or a heavy or light chain of an antibody, or portion thereof, of the disclosure has been obtained, the vector for the production of the antibody may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination.

The disclosure, thus, provides replicable vectors comprising a nucleotide sequence encoding an antibody of the disclosure, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody (see, *e.g.,* International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the disclosure. Thus, the disclosure includes host cells containing a polynucleotide encoding an antibody of the disclosure or fragments thereof, or a heavy or light chain thereof, or portion thereof, or a single chain antibody of the disclosure, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibodies of the disclosure (see, *e.g.,* U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody of the disclosure *in situ.* These include but are not limited to microorganisms such as bacteria (*e.g., E. coli* and *B. subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g.,* baculovirus) containing antibody coding sequences; plant cell systems infected with recombinant virus expression vectors (*e.g.,* cauliflower mosaic virus, CaMV; tobacco mosaic virus, TMV) or transformed with recombinant plasmid expression vectors (*e.g.,* Ti plasmid) containing antibody coding sequences; or mammalian cell systems (*e.g.,* COS, CHO, BHK, 293, NS0, and 3T3 cells) harboring recombinant expression constructs containing promoters derived from the genome of mammalian cells (*e.g.,* metallothionein promoter) or from mammalian viruses (*e.g.,* the adenovirus late promoter; the vaccinia virus 7.5K promoter). Preferably, bacterial cells such as *Escherichia coli,* and more preferably, eukaryotic cells, especially for the expression of whole recombinant antibody, are used for the expression of a recombinant antibody.

For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, BioTechnology 8:2).

In bacterial systems, a number of expression vectors may be advantageously selected depending upon the use intended for the antibody being expressed. For example, when a large quantity of such a protein is to be produced, for the generation of pharmaceutical compositions of an antibody, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. PGEX vectors may also be used to express foreign polypeptides as fusion proteins with glutathione 5-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions of the virus and placed under control of an AcNPV promoter.

In mammalian host cells, a number of viral-based expression systems may be utilized. In cases where an adenovirus is used as an expression vector, the antibody coding sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.,* region El or E3) will result in a recombinant virus that is viable and capable of expressing the antibody in infected hosts (*e.g*., see Logan & Shenk, 1984, PNAS 8 1:6355-6359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g.*, Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, BT483, Hs578T, HTB2, BT2O, NS1 and T47D, NS0 (a murine myeloma cell line that does not endogenously produce any immunoglobulin chains), CRL7O3O and HsS78Bst cells.

In one embodiment the antibodies of the disclosure are produced according to the methods disclosed in U.S. Patent No. 7,521,541 and U.S. Patent Application No. 12/399,241, which are incorporated by reference in their entireties.

For long-term, high-yield production of recombinant proteins, stable expression is preferred. For example, cell lines which stably express the antibody may be engineered. Rather than using expression vectors which contain viral origins of replication, host cells can be transformed with DNA controlled by appropriate expression control elements (*e.g.,* promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody. Such engineered cell lines may be particularly useful in screening and evaluation of compositions that interact directly or indirectly with the antibody.

A number of selection systems may be used, including but not limited to, the herpes simplex virus thymidine kinase (Wigler et al., 1977, Cell 11:223)*,* glutamine synthetase, hypoxanthine guanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, gs-, hgprt- or aprt- cells, respectively. Also, antimetabolite resistance can be used as the basis of selection for the following genes: *dhfr,* which confers resistance to methotrexate (Wigler et al., 1980, PNAS 77:357; O'Hare et al., 1981, PNAS 78:1527); *gpt,* which confers resistance to mycophenolic acid (Mulligan & Berg, 1981, PNAS 78:2072); neo, which confers resistance to the aminoglycoside G-418 (Wu and Wu, 1991, Biotherapy 3:87; Tolstoshev, 1993, Ann. Rev. Pharmacol. Toxicol. 32:573; Mulligan, 1993, Science 260:926; and Morgan and Anderson, 1993, Ann. Rev. Biochem. 62: 191; May, 1993, TIB TECH 11:155-); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1, which are incorporated by reference herein in their entireties.

The expression levels of an antibody can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the disclosure, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, PNAS 77:2197). The coding sequences for the heavy and light chains may comprise cDNA or genomic DNA.

Once a engineered antibody of the disclosure has been produced by recombinant expression, it may be purified by any method known in the art for purification of an immunoglobulin molecule, for example, by chromatography (*e.g.,* ion exchange, affinity, particularly by affinity for the specific antigen after Protein A, and sizing column chromatography), centrifugation, differential solubility, or by any other standard technique for the purification of proteins. Further, the antibodies of the present disclosure or fragments thereof may be fused to heterologous polypeptide sequences described herein or otherwise known in the art to facilitate purification.

### a. Scalable Production of engineered antibodies

In an effort to obtain large quantities of the engineered antibodies of the disclosure, they may be produced by a scalable process (hereinafter referred to as "scalable process of the disclosure"). In some embodiments, engineered antibodies may be produced by a scalable process of the disclosure in the research laboratory that may be scaled up to produce the proteins of the disclosure in analytical scale bioreactors (for example, but not limited to 5L, 10L, 15L, 30L, or 50L bioreactors) while maintaining the functional activity of the proteins. For instance, in one embodiment, proteins produced by scalable processes of the disclosure exhibit low to undetectable levels of aggregation as measured by HPSEC or rCGE, that is, no more than 5%, no more than 4%, no more than 3%, no more than 2%, no more than 1%, or no more than 0.5% aggregate by weight protein, and/or low to undetectable levels of fragmentation, that is, 80% or higher, 85% or higher, 90% or higher, 95% or higher, 98% or higher, or 99% or higher, or 99.5% or higher of the total peak area in the peak(s) representing intact engineered antibodies.

In other embodiments, the engineered antibodies may be produced by a scalable process of the disclosure in the research laboratory that may be scaled up to produce the proteins of the disclosure in production scale bioreactors (for example, but not limited to 75L, 100L, 150L, 300L, or 500L). In some embodiments, the scalable process of the disclosure results in little or no reduction in production efficiency as compared to the production process performed in the research laboratory. In other embodiments, the scalable process of the disclosure produces engineered antibodies at production efficiency of about 10 mg/L, about 20 m/L, about 30 mg/L, about 50 mg/L, about 75 mg/L, about 100 mg/ L, about 125 mg/L, about 150 mg/L, about 175 mg/L, about 200 mg/L, about 250 mg/L, about 300 mg/L or higher.

In other embodiments, the scalable process of the disclosure produces engineered antibodies at production efficiency of at least about 10 mg/L, at least about 20 mg/L, at least about 30 mg/L, at least about 50 mg/L, at least about 75 mg/L, at least about 100 mg/L, at least about 125 mg/L, at least about 150 mg/L, at least about 175 mg/L, at least about 200 mg/L, at least about 250 mg/L, at least about 300 mg/L or higher.

In other embodiments, the scalable process of the disclosure produces engineered antibodies at production efficiency from about 10 mg/L to about 300 mg/L, from about 10 mg/L to about 250 mg/L, from about 10 mg/L to about 200 mg/L, from about 10 mg/L to about 175 mg/L, from about 10 mg/L to about 150 mg/L, from about 10 mg/L to about 100 mg/L, from about 20 mg/L to about 300 mg/L, from about 20 mg/L to about 250 mg/L, from about 20 mg/L to about 200 mg/L, from 20 mg/L to about 175 mg/L, from about 20 mg/L to about 150 mg/L, from about 20 mg/L to about 125 mg/L, from about 20 mg/L to about 100 mg/L, from about 30 mg/L to about 300 mg/L, from about 30 mg/L to about 250 mg/L, from about 30 mg/L to about 200 mg/L, from about 30 mg/L to about 175 mg/L, from about 30 mg/L to about 150 mg/L, from about 30 mg/L to about 125 mg/L, from about 30 mg/L to about 100 mg/L, from about 50 mg/L to about 300 mg/L, from about 50 mg/L to about 250 mg/L, from about 50 mg/L to about 200 mg/L, from 50 mg/L to about 175 mg/L, from about 50 mg/L to about 150 mg/L, from about 50 mg/L to about 125 mg/L, from about 50 mg/L to about 100 mg/L.

To ensure the stability of the antibodies of the disclosure, suitable assays have been developed. In one embodiment, the stability of proteins of the disclosure is characterized by known techniques in the art. In other embodiments, the stability of the proteins of the disclosure can be assessed by aggregation and/or fragmentation rate or profile. To determine the level of aggregation or fragmentation, many techniques may be used. In one embodiment, the aggregation and/or fragmentation profile may be assessed by the use of analytical ultracentrifugation (AUC), size-exclusion chromatography (SEC), high-performance size-exclusion chromatography (HPSEC), melting temperature (Tₘ), polyacrylamide gel electrophoresis (PAGE), capillary gel electrophoresis (CGE), light scattering (SLS), Fourier Transform Infrared Spectroscopy (FTIR), circular dichroism (CD), urea-induced protein unfolding techniques, intrinsic tryptophan fluorescence, differential scanning calorimetry, or 1-anilino-8-naphthalenesulfonic acid (ANS) protein binding techniques. In another embodiment, the stability of proteins of the disclosure is characterized by polyacrylamide gel electrophoresis (PAGE) analysis. In another embodiment, the stability of the proteins of the disclosure is characterized by size exclusion chromatography (SEC) profile analysis.

### Antibody Conjugates and Fusion Proteins

The present disclosure encompasses the use of Fc regions, the the use of engineered antibodies, which are recombinantly fused or chemically conjugated (including both covalent and non-covalent conjugations) to a heterologous agent. Suitable substances for attachment to antibodies include, but are not limited to, an amino acid, a peptide, a protein, a polysaccharide, a nucleoside, a nucleotide, an oligonucleotide, a nucleic acid, a hapten, a drug, a hormone, a lipid, a lipid assembly, a synthetic polymer, a polymeric microparticle, a biological cell, a virus, a fluorophore, a chromophore, a dye, a toxin, a hapten, an enzyme, an antibody, an antibody fragment, a radioisotope, solid matrixes, semi-solid matrixes and combinations thereof. Methods for conjugation or covalently attaching another substance to an antibody are well known in the art. The fusion or conjugation does not necessarily need to be direct, but may occur through linker sequences. Antibodies fused or conjugated to heterologous agents may be used *in vivo* to detect, treat, manage, or monitor the progression of a disorder using methods known in the art. See, *e.g.,* International Publication WO 93/21232; EP 439,095; Naramura et al., 1994, Immunol. Lett. 39:91-99; U.S. Patent 5,474,981; Gillies et al., 1992, PNAS 89:1428-1432; and Fell et al., 1991, J. Immunol. 146:2446-2452, which are incorporated by reference in their entireties. In some embodiments, the disorder to be detected, treated, managed, or monitored is an autoimmune, inflammatory, infectious disease or cancer related disorder. Methods for fusing or conjugating polypeptides to antibody portions are known in the art. See, *e.g.,* U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341 (said references incorporated by reference in their entireties).

Additional fusion proteins may be generated through the techniques of gene-shuffling, motif-shuffling, exon-snuffling, and/or codon-shuffling (collectively referred to as "DNA shuffling"). DNA shuffling may be employed to alter the activities of engineered antibodies of the disclosure (*e.g.,* antibodies with higher affinities and lower dissociation rates). See, generally, U.S. Patent Nos. 5,605,793; 5,811,238; 5,830,721; 5,834,252; and 5,837,458, and Patten et al., 1997, Curr. Opinion Biotechnol. 8:724-33; Harayama, 1998, Trends Biotechnol. 16:76; Hansson, et al., 1999, J. Mol. Biol. 287:265; and Lorenzo and Blasco, 1998, BioTechniques 24:308 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment may be recombined with one or more components, motifs, sections, parts, domains, fragments, etc. of one or more heterologous agents.

In certain embodiments, the Fc regions or the antibodies of the disclosure are conjugated to a solid support. Antibodies may be conjugated to a solid support as part of the screening and/or purification and/or manufacturing process. Alternatively antibodies of the disclosure may be conjugated to a solid support as part of a diagnostic method or composition. A solid support suitable for use in the present disclosure is typically substantially insoluble in liquid phases. A large number of supports are available and are known to one of ordinary skill in the art. Thus, solid supports include solid and semi-solid matrixes, such as aerogels and hydrogels, resins, beads, biochips (including thin film coated biochips), microfluidic chip, a silicon chip, multi-well plates (also referred to as microtitre plates or microplates), membranes, conducting and nonconducting metals, glass (including microscope slides) and magnetic supports. More specific examples of solid supports include silica gels, polymeric membranes, particles, derivatized plastic films, glass beads, cotton, plastic beads, alumina gels, polysaccharides such as Sepharose, poly(acrylate), polystyrene, poly(acrylamide), polyol, agarose, agar, cellulose, dextran, starch, FICOLL, heparin, glycogen, amylopectin, mannan, inulin, nitrocellulose, diazocellulose, polyvinylchloride, polypropylene, polyethylene (including poly(ethylene glycol)), nylon, latex bead, magnetic bead, paramagnetic bead, superparamagnetic bead, starch and the like.

In some embodiments, the solid support may include a reactive functional group, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, etc., for attaching the antibodies of the disclosure.

A suitable solid phase support can be selected on the basis of desired end use and suitability for various synthetic protocols. For example, where amide bond formation is desirable to attach the antibodies of the disclosure to the solid support, resins generally useful in peptide synthesis may be employed, such as polystyrene (e.g., PAM-resin obtained from Bachem Inc., Peninsula Laboratories, etc.), POLYHIPE™ resin (obtained from Aminotech, Canada), polyamide resin (obtained from Peninsula Laboratories), polystyrene resin grafted with polyethylene glycol (TentaGel™, Rapp Polymere, Tubingen, Germany), polydimethyl-acrylamide resin (available from Milligen/Biosearch, California), or PEGA beads (obtained from Polymer Laboratories).

In one embodiment, engineered antibodies of the present disclosure or fragments or variants thereof are conjugated or fused to a marker sequence, such as a peptide, to facilitate purification. In certain embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, PNAS 86:821, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present disclosure thereof are conjugated or fused to a diagnostic or detectable agent. Such antibodies can be useful for monitoring or prognosing the development or progression of a disorder (such as, but not limited to cancer) as part of a clinical testing procedure, such as determining the efficacy of a particular therapy.

Such diagnosis and detection can accomplished by fusing or conjugating the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, bioluminescent materials, such as but not limited to, luciferase, luciferin, and aequorin; radioactive materials, such as but not limited to, bismuth (²¹³Bi), carbon (¹⁴C), chromium (⁵¹Cr), cobalt (¹⁷Co), fluorine (¹⁸F), gadolinium (¹⁵³Gd, ¹⁵⁹Gd), gallium (⁶⁸Ga, ⁶⁷Ga), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), zinc (⁶⁵Zn); positron emitting metals using various positron emission tomographies, and nonradioactive paramagnetic metal ions.

In other embodiments, engineered antibodies of the present disclosure are conjugated to a therapeutic agent such as a cytotoxin, *e.g.*, a cytostatic or cytocidal agent, a therapeutic agent or a radioactive metal ion, *e.g.,* alpha-emitters. A cytotoxin or cytotoxic agent includes any agent that is detrimental to cells. Examples include paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof. Therapeutic agents include, but are not limited to, antimetabolites (*e.g.,* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (*e.g.,* daunorubicin (formerly daunomycin) and doxorubicin), antibiotics (*e.g.,* dactinomycin (formerly actinomycin), bleomycin, mithramycin, and anthramycin (AMC)), and anti-mitotic agents (*e.g.,* vincristine and vinblastine). Chemical toxins can also be taken from the group chosen from duocarmycin (U.S. Patent Nos. 5,703,080; 4,923,990), methotrexate, doxorubicin, melphalan, chlorambucil, ARA-C, vindesine, mitomycin C, cis-platinum, etoposide, bleomycin and 5-fluorouracil. Examples of chemotherapeutic agents also include Adriamycin, Doxorubicin, 5-Fluorouracil, Cytosine arabinoside (Ara-C), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (U.S. Patent No. 4,675,187), Melphalan, and other related nitrogen mustards.

In one embodiment, the cytotoxic agent is chosen from an enediyne, a lexitropsin, a duocarmycin, a taxane, a puromycin, a dolastatin, a maytansinoid, and a vinca alkaloid. In other embodiments, the cytotoxic agent is paclitaxel, docetaxel, CC-1065, SN-38, topotecan, morpholino-doxorubicin, rhizoxin, cyanomorpholino-doxorubicin, dolastatin-10, echinomycin, combretastatin, calicheamicin, maytansine, DM-1, an auristatin or other dolastatin derivatives, such as auristatin E or auristatin F, AEB, AEVB, AEFP, MMAE (monomethylauristatin E), MMAF (monomethylauristatin F), eleutherobin or netropsin. The synthesis and structure of auristatin E, also known in the art as dolastatin-10, and its derivatives are described in U.S. Patent Application Publ. Nos. 2003/0083263 A1 and 2005/0009751 A1; in the International Patent Application No.: PCT/US02/13435, in U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414, all of which are incorporated by reference in their entireties herein.

In certain embodiments, the cytoxic agent is Maytansine or Maytansinoids, and derivatives thereof, wherein an antibodies (full length or fragments) of the disclosure are conjugated to one or more maytansinoid molecules. Maytansinoids are mitototic inhibitors which act by inhibiting tubulin polymerization. Maytansine was first isolated from the east African shrub Maytenus serrata (U.S. Pat. No. 3,896,111). Subsequently, it was discovered that certain microbes also produce maytansinoids, such as maytansinol and C-3 maytansinol esters (U.S. Pat. No. 4,151,042). Synthetic maytansinol and derivatives and analogues thereof are disclosed, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Pat. Nos. 5,208,020, 5,416,064 and European Patent EP 0 425 235 B1. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al. Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansonoid conjugate was tested in vitro on the human breast cancer cell line SK-BR-3, which expresses 3×105 HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice. Thus, the present disclosure contemplates antibodies conjugated to maytansinoid agents for therapeutic treatment of certain cancers.

In other embodiments, the cytotoxic agent of an antibody conjugate of the disclosure is an anti-tubulin agent. Anti-tubulin agents are a well established class of cancer therapy compounds. Examples of anti-tubulin agents include, but are not limited to, taxanes (e.g., Taxol® (paclitaxel), docetaxel), T67 (Tularik), vincas, and auristatins (e.g., auristatin E, AEB, AEVB, MMAE, MMAF, AEFP). Antitubulin agents included in this class are also: vinca alkaloids, including vincristine and vinblastine, vindesine and vinorelbine; taxanes such as paclitaxel and docetaxel and baccatin derivatives, epithilone A and B, nocodazole, 5-Fluorouracil and colcimid, estramustine, cryptophysins, cemadotin, maytansinoids, combretastatins, dolastatins, discodermolide and eleutherobin In more specific embodiments, the cytotoxic agent is chosen from a vinca alkaloid, a podophyllotoxin, a taxane, a baccatin derivative, a cryptophysin, a maytansinoid, a combretastatin, and a dolastatin. In more specific embodiments, the cytotoxic agent is vincristine, vinblastine, vindesine, vinorelbine, VP-16, camptothecin, paclitaxel, docetaxel, epithilone A, epithilone B, nocodazole, coichicine, colcimid, estramustine, cemadotin, discodermolide, maytansine, DM-1, an auristatin or other dolastatin derivatives, such as auristatin E or auristatin F, AEB, AEVB, AEFP, MMAE (monomethylauristatin E), MMAF (monomethylauristatin F), eleutherobin or netropsin.

In a specific embodiment, the drug is a maytansinoid, a group of anti-tubulin agents. In a more specific embodiment, the drug is maytansine. Further, in a specific embodiment, the cytotoxic or cytostatic agent is DM-1 (ImmunoGen, Inc.; see also Chari et al. 1992, Cancer Res 52:127-131). Maytansine, a natural product, inhibits tubulin polymerization resulting in a mitotic block and cell death. Thus, the mechanism of action of maytansine appears to be similar to that of vincristine and vinblastine. Maytansine, however, is about 200 to 1,000-fold more cytotoxic in vitro than these vinca alkaloids. In another specific embodiment, the drug is an AEFP.

In some embodiments, the antibodies may be conjugated or fused to other small molecule or protein toxins, such as, but not limited to abrin, brucine, cicutoxin, diphtheria toxin, batrachotoxin, botulism toxin, shiga toxin, endotoxin, Pseudomonas exotoxin, Pseudomonas endotoxin, tetanus toxin, pertussis toxin, anthrax toxin, cholera toxin, falcarinol, fumonisin B1, fumonisin B2, afla toxin, maurotoxin, agitoxin, charybdotoxin, margatoxin, slotoxin, scyllatoxin, hefutoxin, calciseptine, taicatoxin, calcicludine, geldanamycin, gelonin, lotaustralin, ocratoxin A, patulin, ricin, strychnine, trichothecene, zearlenone, and tetradotoxin. Enzymatically active toxins and fragments thereof which can be used include diphtheria A chain, non-binding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), Momordica charantia inhibitor, curcin, crotin, Sapaonaria officinalis inhibitor, gelonin, mitogellin, restrictocin, phenomycin, enomycin and the tricothecenes.

Further examples of toxins, spacers, linkers, stretchers and the like, and their structures can be found in U.S. Patent Application Publication Nos. 2006/0074008 A1, 2005/0238649 A1, 2005/0123536 A1, 2005/0180972 A1, 2005/0113308 A1, 2004/0157782 A1, U.S. Patent No. 6,884,869 B2, U.S. Patent No. 5,635,483, all of which are hereby incorporated by reference herein in their entirety.

As discussed herein, the compounds used for conjugation to the antibody conjugates of the present disclosure can include conventional chemotherapeutics, such as doxorubicin, paclitaxel, carboplatin, melphalan, vinca alkaloids, methotrexate, mitomycin C, etoposide, and others. In addition, potent agents such CC-1065 analogues, calichiamicin, maytansine, analogues of dolastatin 10, rhizoxin, and palytoxin can be linked to the antibodies using the conditionally stable linkers to form potent immunoconjugates.

In certain embodiments, the cytotoxic or cytostatic agent is a dolastatin. In more specific embodiments, the dolastatin is of the auristatin class. In a specific embodiment of the disclosure, the cytotoxic or cytostatic agent is MMAE. In another specific embodiment of the disclosure, the cytotoxic or cytostatic agent is AEFP. In another specific embodiment of the disclosure, the cytotoxic or cytostatic agent is MMAF.

In other embodiments, antibodies of the present disclosure are conjugated or fused to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, e.g., TNF-α, TNF-β, AIM I (see, International Publication No. WO 97/33899), AIM II (see, International Publication No. WO 97/34911), Fas Ligand (Takahashi et al., 1994, J. Immunol., 6:1567), and VEGf (see, International Publication No. WO 99/23105), a thrombotic agent or an anti-angiogenic agent, *e.g.,* angiostatin or endostatin; or, a biological response modifier such as, for example, a lymphokine (*e.g.,* interleukin-1 ("IL-1"), interleukin-2 ("IL-2"), interleukin-4 ("IL-4"), interleukin-6 ("IL-6"), interleukin-7 ("IL-7"), interleukin-9 ("IL-9"), interleukin-15 ("IL-15"), interleukin-12 ("IL-12"), granulocyte macrophage colony stimulating factor ("GM-CSF"), and granulocyte colony stimulating factor ("G-CSF")), or a growth factor (*e.g.,* growth hormone ("GH")).

In other embodiments, antibodies of the present disclosure are conjugated or fused to a polypeptide that comprises poly-arginine or poly-lysine residues. In some embodiments, said polypeptide comprises 5, 6, 7, 8, 9, 10, 11, 12, 13, 14, 15, or more amino acid residues. In some embodiments, the poly-arginine polypeptide may comprise at least 5, 6, 7, 8, 9, 10, 11, 12, 13, 14, 15, or more arginine residues. In other embodiments, the poly-lysine polypeptide may comprise at least 5, 6, 7, 8, 9, 10, 11, 12, 13, 14, 15, or more lysine residues. In other embodiments, the polypeptide may comprise any combination of arginine and lysine residues.

In other embodiments, antibodies of the present disclosure are conjugated to a therapeutic agent such as radioactive materials or macrocyclic chelators useful for conjugating radiometal ions (see above for examples of radioactive materials). In certain embodiments, the macrocyclic chelator is 1,4,7,10-tetraazacyclododecane-N,N',N",N"-tetraacetic acid (DOTA) which can be attached to the antibody via a linker molecule. Such linker molecules, further discussed herein below, are commonly known in the art and described in Denardo et al., 1998, Clin Cancer Res. 4:2483-90; Peterson et al., 1999, Bioconjug. Chem. 10:553; and Zimmerman et al., 1999, Nucl. Med. Biol. 26:943-50 each incorporated by reference in their entireties.

In other embodiments, antibodies of the present disclosure are conjugated to a nucleic acid. The nucleic acid may be chosen from DNA, RNA, short interfering RNA (siRNA), microRNA, hairpin or nucleic acid mimetics such as peptide nucleic acid. In some embodiments the conjugated nucleic acid is at least 10, at least 20, at least 30, at least 40, at least 50, at least 60 at least 100, at least 200, at least 500, at least 1000, at least 5000 or more base pairs. In some embodiments, the conjugated nucleic acid is single stranded. In alternative embodiments, the conjugated nucleic acid is double stranded.

In some embodiments, the conjugated nucleic acid encodes an open reading frame. In some embodiments, the open reading frame encoded by the conjugated nucleic acid corresponds to an apoptosis inducing protein, a viral protein, an enzyme, or a tumor suppressor protein. Techniques for delivery of such nucleic acids to cells may be found at Song et al. Nature Biotechnology, 2005, Vol23:6 p709-717 and also US Patent No. 6,333,396 which is incorporated by reference in its entirety.

Techniques for conjugating therapeutic moieties to antibodies are well known. Moieties can be conjugated to antibodies by any method known in the art, including, but not limited to aldehyde/Schiff linkage, sulphydryl linkage, acid-labile linkage, cis-aconityl linkage, hydrazone linkage, enzymatically degradable linkage (see generally Garnett, 2002, Adv. Drug Deliv. Rev. 53:171-216). Additional techniques for conjugating therapeutic moieties to antibodies are well known, see, *e.g.,* Arnon et al., "Monoclonal Antibodies For Immunotargeting Of Drugs In Cancer Therapy," in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review," in Monoclonal Antibodies '84: Biological And Clinical Applications, Pinchera et al. (eds.), pp. 475-506 (1985*);* "Analysis, Results, And Future Prospective Of The Therapeutic Use Of Radiolabeled Antibody In Cancer Therapy," in Monoclonal Antibodies For Cancer Detection And Therapy, Baldwin et al. (eds.), pp. 303-16 (Academic Press 1985), and Thorpe et al., 1982, Immunol. Rev. 62:119-58. Methods for fusing or conjugating antibodies to polypeptide moieties are known in the art. See, *e.g.,* U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341. The fusion of an antibody to a moiety does not necessarily need to be direct, but may occur through linker sequences. Such linker molecules are commonly known in the art and described in Denardo et al., 1998, Clin Cancer Res. 4:2483-90; Peterson et al., 1999, Bioconjug. Chem. 10:553; Zimmerman et al., 1999, Nucl. Med. Biol. 26:943-50; Garnett, 2002, Adv. Drug Deliv. Rev. 53:171-216, each of which is incorporated herein by reference in its entirety.

Two exemplary approaches may be taken to minimize drug activity outside the cells that are targeted by the antibody conjugates of the disclosure: first, an antibody that binds to a cell membrane receptor but not soluble receptor may be used, so that the drug, including drug produced by the actions of the prodrug converting enzyme, is concentrated at the cell surface of the cells, such as an activated lymphocyte; second, the drugs are conjugated in a manner that would reduce their activity unless they are hydrolyzed or cleaved off the antibody. Such methods would employ attaching the drug to the antibodies with linkers that are sensitive to the environment at the cell surface of the activated lymphocyte (e.g., the activity of a protease that is present at the cell surface of the activated lymphocyte) or to the environment inside the activated lymphocyte the conjugate encounters when it is taken up by the activated lymphocyte (e.g., in the endosomal or, for example by virtue of pH sensitivity or protease sensitivity, in the lysosomal environment). Examples of linkers that can be used in the present disclosure are disclosed in U.S. Patent Application Publication Nos. 2005/0123536 A1, 2005/0180972 A1, 2005/0113308 A1, 2004/0157782 A1, and U.S. Patent No. 6,884,869 B2, all of which are hereby incorporated by reference herein in their entirety.

In one embodiment, the linker is an acid-labile hydrazone or hydrazide group that is hydrolyzed in the lysosome (see, e.g., U.S. Pat. No. 5,622,929). In alternative embodiments, drugs can be conjugated to antibodies through other acid-labile linkers, such as cis-aconitic amides, orthoesters, acetals and ketals (Dubowchik and Walker, 1999, Pharm. Therapeutics 83:67-123; Neville et al., 1989, Biol. Chem. 264:14653-14661). Such linkers are relatively stable under neutral pH conditions, such as those in the blood, but are unstable at below pH 5, the approximate pH of the lysosome.

In other embodiments, drugs are attached to the antibodies of the disclosure using peptide spacers that are cleaved by intracellular proteases. Target enzymes include cathepsins B and D and plasmin, all of which are known to hydrolyze dipeptide drug derivatives resulting in the release of active drug inside target cells (Dubowchik and Walker, 1999, Pharm. Therapeutics 83:67-123). The advantage of using intracellular proteolytic drug release is that the drug is highly attenuated when conjugated and the serum stabilities of the conjugates can be extraordinarily high.

In yet other embodiments, the linker is a malonate linker (Johnson et al., 1995. Anticancer Res. 15:1387-93), a maleimidobeiizoyl linker (Lau et al., 1995, Bioorg-Med-Chem. 3(10):1299-1304), or a 3'-N-amide analog (Lau et al, 1995, Bioorg-Med-Chem. 3(103:1305-12).

Linking chemistry employing a maleimide group and a spacer (such as polyethylene glycol (PEG) or the like) is suitable for cysteine, lysine, selenocysteine, and selenomethaionine substitutions. For histidine substitutions, one may use a spacer coupled with a metal (such as copper, zinc, iron, nickel, etc.) for conjugation. For tyrosine, one may conjugate to a functional group present in a sugar or other hydroxyl compound. Details of these and other suitable conjugation techniques are known those of ordinary skill in the art and can be found in, for example, Bioconjugate Techniques, 2nd ed., by Greg T. Hermanson, Academic Press (2008).

As discussed above, antibody conjugates are generally made by conjugating a compound or a drug to an antibody through a linker. Any linker that is known in the art may be used in the conjugates of the present disclosure, e.g., bifunctional agents (such as dialdehydes or imidoesters) or branched hydrazone linkers (see, e.g., U.S. Pat. No. 5,824,805, which is incorporated by reference herein in its entirety).

In certain, non-limiting, embodiments of the disclosure, the linker region between the conjugate moiety and the antibody moiety is cleavable under certain conditions, wherein cleavage or hydrolysis of the linker releases the drug moiety from the antibody moiety. In some embodiments, the linker is sensitive to cleavage or hydrolysis under intracellular conditions.

In one embodiment, the linker region between the conjugate moiety and the antibody moiety is cleavable if the pH changes by a certain value or exceeds a certain value. In another embodiment of the disclosure, the linker is cleavable in the milieu of the lysosome, e.g., under acidic conditions (i.e., a pH of around 5-5.5 or less). In other embodiments, the linker is a peptidyl linker that is cleaved by a peptidase or protease enzyme, including but not limited to a lysosomal protease enzyme, a membrane-associated protease, an intracellular protease, or an endosomal protease. Typically, the linker is at least two amino acids long, more typically at least three amino acids long. For example, a peptidyl linker that is cleavable by cathepsin-B (e.g., a Gly-Phe-Leu-Gly linker), a thiol-dependent protease that is highly expressed in cancerous tissue, can be used. Other such linkers are described, e.g., in U.S. Pat. No. 6,214,345, which is incorporated by reference in its entirety herein.

In other, non-mutually exclusive embodiments of the disclosure, the linker by which the antibody and compound of an antibody conjugate of the disclosure are conjugated promotes cellular internalization. In certain embodiments, the linker-drug moiety promotes cellular internalization. In certain embodiments, the linker is chosen such that the structure of the entire antibody'conjugate promotes cellular internalization. In one embodiment, the linker is a thioether linker (see, e.g., U.S. Pat. No. 5,622,929 to Willner et al., which is incorporated by reference herein in its entirety). In another embodiment, the linker is a hydrazone linker (see, e.g., U.S. Pat. Nos. 5,122,368 to Greenfield et al. and 5,824,805 to King et al., which are incorporated by reference herein in their entireties).

In yet other embodiments, the linker is a disulfide linker. A variety of disulfide linkers are known in the art, including but not limited to those that can be formed using SATA (N-succinimidyl-S-acetylthioacetate), SPDP (N-succinimidyl-3-(2-pyridyldi-thio)propionate), SPDB (N-succinimidyl-3-(2-pyridyldithio)butyrate) and SMPT (N-succinimidyl-oxycarbonyl-alpha-methyl-alpha-(2-pyridyl-dithio)tol- uene). SPDB and SMPT (see, e.g., Thorpe et al., 1987, Cancer Res., 47:5924-5931; Wawrzynczak et al., 1987, In Immunoconjugates: Antibody Conjugates in Radioimagery and Therapy of Cancer, ed. C. W. Vogel, Oxford U. Press, pp. 28-55; see also U.S. Pat. No. 4,880,935 to Thorpe et al., which is incorporated by reference herein in its entirety).

A variety of linkers that can be used with the compositions and methods of the present disclosure are described in U.S. Patent Application Publication No. US 2004/0018194 A1, which is incorporated by reference in its entirety herein.

In yet other embodiments of the present disclosure, the linker unit of an antibody conjugate links the cytotoxic or cytostatic agent (drug unit; -D) and the antibody unit (-A). In certain embodiments, the linker unit has the general formula:
i. -Ta-Ww-Yy- wherein:
ii. -T- is a stretcher unit;
iii. a is 0 or 1;
iv. each -W-is independently an amino acid unit;
v. w is independently an integer ranging from 2 to 12;
vi. -Y-is a spacer unit; and
vii. y is 0, 1 or 2.

The stretcher unit (-T-), when present, links the antibody unit to an amino acid unit (--W--). Useful functional groups that can be present on an antibody, either naturally or via chemical manipulation include, but are not limited to, sulfhydryl, amino, hydroxyl, the anomeric hydroxyl group of a carbohydrate, and carboxyl. Engineered antibodies of the disclosure wherein a cysteine has been introduced present at least one sulfhydryl group for conjugation. Other methods of introducing sulfhydryl groups involve the reduction of the intramolecular disulfide bonds of an antibody. Alternatively, sulfhydryl groups can be generated by reaction of an amino group of a lysine moiety of an antibody (which is either native or that has been introduced) with 2-iminothiolane (Traut's reagent) or other sulfhydryl generating reagents.

The amino acid unit (--W--) links the stretcher unit (-T-) to the Spacer unit (-Y--) if the Spacer unit is present, and links the stretcher unit to the cytotoxic or cytostatic agent (Drug unit; D) if the spacer unit is absent.

In some embodiments, -W_{w}-is a dipeptide, tripeptide, tetrapeptide, pentapeptide, hexapeptide, heptapeptide, octapeptide, nonapeptide, decapeptide, undecapeptide or dodecapeptide unit. The amino acid unit of the linker unit can be enzymatically cleaved by an enzyme including, but not limited to, a tumor-associated protease to liberate the drug unit (-D) which is protonated in vivo upon release to provide a cytotoxic drug (D).

In a one embodiment, the amino acid unit is a phenylalanine-lysine dipeptide (phe-lys or FK linker). In another embodiment, the amino acid unit is a valine-citrulline dipeptide (val-cit or VC linker).

The spacer unit (--Y--), when present, links an amino acid unit to the drug unit. Spacer units are of two general types: self-immolative and non self-immolative. A non self-immolative spacer unit is one in which part or all of the spacer unit remains bound to the drug unit after enzymatic cleavage of an amino acid unit from the antibody-linker-drug conjugate or the drug-linker compound. Examples of a non self-immolative spacer unit include, but are not limited to a (glycine-glycine) spacer unit and a glycine spacer unit. When an antibody-linker-drug conjugate of the disclosure containing a glycine-glycine spacer unit or a glycine spacer unit undergoes enzymatic cleavage via a tumor-cell associated-protease, a cancer-cell-associated protease or a lymphocyte-associated protease, a glycine-glycine-drug moiety or a glycine-drug moiety is cleaved from A-T-W_{w}--. To liberate the drug, an independent hydrolysis reaction should take place within the target cell to cleave the glycine-drug unit bond.

Other examples of self-immolative spacers include, but are not limited to, aromatic compounds that are electronically equivalent to the PAB group such a 2-aminoimidazol-5-methanol derivatives (see Hay et al., Bioorg. Med. Chem. Lett., 1999, 9, 2237 for examples) and ortho or para-aminobenzylacetals. Spacers can be used that undergo facile cyclization upon amide bond hydrolysis, such as substituted and unsubstituted 4-aminobutyric acid amides (Rodrigues et al., Chemistry, Biology, 1995, 2, 223), appropriately substituted ring systems (Storm, et al., J. Amer. Chem. Soc., 1972, 94, 5815) and 2-aminophenylpropionic acid amides (Amsberry, et al., J. Org. Chem., 1990, 55, 5867). Elimination of amine-containing drugs that are substituted at the α-position of glycine (Kingsbury, et al., J. Med. Chem., 1984, 27, 1447) are also examples of self-immolative spacer strategies that can be applied to the antibody-linker-drug conjugates of the disclosure.

### Methods of conjugating a heterologous molecule to an antibody

Heterologous molecules, such as those described herein may be efficiently conjugated to antibodies or Fc regions of the disclosure through the reactive groups the engineered amino acid residues provide. In one aspect, the disclosure provides methods for efficiently conjugating heterologous molecules to cysteine engineered antibodies. In one embodiment, the conjugation of a heterologous molecule may occur at a reactive group provided by at least one engineered residue selected from the positions 239, 282, 289, 297, 312, 324, 330, 335, 337, 339, 356, 359, 361, 383, 384, 398, 400, 440, 422, and 442 of the Fc region or antibody, wherein the numbering system of the constant region is that of the EU index as set forth in Kabat. In a further aspect, the reactive group is a thiol, and the conjugation of a heterologous molecule may occur at a thiol group provided by at least one engineered cysteine residue selected from the positions 239, 282, 289, 297, 312, 324, 330, 335, 337, 339, 356, 359, 361, 383, 384, 398, 400, 440, 422, and 442 of the Fc region or an antibody, wherein the numbering system of the constant region is that of the EU index as set forth in Kabat.

The engineering of non-naturally occurring cysteine residues into antibodies may alter the disulfide pairing of the heavy and light chains such that a naturally occurring cysteine residue which was part of a disulfide bond is liberated and presents a thiol group capable of conjugation. In another embodiment, the method comprises the efficient conjugation of a heterologous molecule to a cysteine engineered antibody at a thiol group not provided by at least one engineered cysteine residue selected from the positions 239, 282, 289, 297, 312, 324, 330, 335, 337, 339, 356, 359, 361, 383, 384, 398, 400, 440, 422, and 442 of the Fc region of an antibody.

The presence of free thiol groups in antibodies may be determined by various art accepted techniques, such as those described in Example 1. The efficiency of conjugation of a heterologous molecule to an antibody may be determined by assessing the presence of free thiols remaining after the conjugation reaction. In one embodiment, the disclosure provides a method of efficiently conjugating a heterologous molecule to a cysteine engineered antibody. In one embodiment, the conjugation efficiency is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, at least 99%, or more as measured by the level of free thiol groups remaining after the conjugation reaction.

In another embodiment, the disclosure provides a method of conjugating a heterologous molecule to an antibody or Fc region wherein the antibody or Fc region comprises at least one amino acid substitution, such that 2 or more reactive groups are formed. In another embodiment, the method comprises an Fc region or antibody comprising at least one amino acid substitution, such that at least 2, at least 4, at least 6, at least 8, at least 10, at least 12, at least 14, at least 16, at least 18, at least 20, at least 22, at least 24, at least 26, at least 28, at least 30, at least 32, at least 34, at least 36, at least 38, at least 40, or more newly-introduced reactive groups are formed. In a further embodiment, at least one of the substitutions is with a cysteine, and the reactive groups are thiol groups.

Fc regions and antibodies of the disclosure capable of conjugation may contain cysteine residues that comprise sulfhydryl groups that are blocked or capped. Such caps include proteins, peptides, ions and other materials that interact with the sulfhydryl group and prevent or inhibit conjugate formation. In some embodiments, antibodies of the disclosure may require uncapping prior to a conjugation reaction. In specific embodiments, antibodies of the disclosure are uncapped and display a sulfhydryl group capable of conjugation. In other specific embodiments, antibodies of the disclosure are subjected to an uncapping reaction that does not disturb or rearrange the naturally occurring disulfide bonds. In other embodiments, antibodies of the disclosure are subjected to an uncapping reaction as presented in Examples 9 or 10 of PCT application PCT/US09/31294, filed Jan 16, 2009.

In some embodiments, antibodies of the disclosure may be subjected to conjugation reactions wherein the antibody to be conjugated is present at a concentration of at least 1mg/ml, at least 2 mg/ml, at least 3 mg/ml, at least 4 mg/ml, at least 5 mg/ml or higher.

### Methods of using antibody conjugates

It is contemplated that the conjugates of the present disclosure may be used to treat various diseases or disorders, *e.g.* those characterized by the overexpression of a tumor antigen. Exemplary conditions or hyperproliferative disorders include benign or malignant tumors, leukemia and lymphoid malignancies. Others include neuronal, glial, astrocytal, hypothalamic, glandular, macrophagal, epithelial, endothelial, and stromal malignancies. Other cancers or hyperproliferative disorders include: cancers of the head, neck, eye, mouth, throat, esophagus, chest, skin, bone, lung, colon, rectum, colorectal, stomach, spleen, kidney, skeletal muscle, subcutaneous tissue, metastatic melanoma, endometrial, prostate, breast, ovaries, testicles, thyroid, blood, lymph nodes, kidney, liver, pancreas, brain, or central nervous system. Examples of cancers that can be prevented, managed, treated or ameliorated in accordance with the methods of the disclosure include, but are not limited to, cancer of the head, neck, eye, mouth, throat, esophagus, chest, bone, lung, colon, rectum, stomach, prostate, breast, ovaries, kidney, liver, pancreas, and brain. Additional cancers include, but are not limited to, the following: leukemias such as but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple mycloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone cancer and connective tissue sarcomas such as but not limited to bone sarcoma, myeloma bone disease, multiple myeloma, cholesteatoma-induced bone osteosarcoma, Paget's disease of bone, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, and synovial sarcoma; brain tumors such as but not limited to, glioma, astrocytoma, brain stem glioma, ependymoma, oligodendroglioma, non-glial tumor, acoustic neurinoma, craniopharyngioma, medulloblastoma, meningioma, pineocytoma, pineoblastoma, and primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease (including juvenile Paget's disease) and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma, gallbladder cancers such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell cancer, adenocarcinoma, hypernephroma, fibrosarcoma, transitional cell cancer (renal pelvis and/or ureter); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesotheliorna, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J. B. Lippincott Co., Philadelphia and Murphy et al., 1997, Informed Decisions: The Complete Book of Cancer Diagnosis, Treatment, and Recovery, Viking Penguin, Penguin Books U.S.A., inc., United States of America). It is also contemplated that cancers caused by aberrations in apoptosis can also be treated by the methods and compositions of the disclosure. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes.

The proteins of the disclosure and compositions comprising the same are useful for many purposes, for example, as therapeutics against a wide range of chronic and acute diseases and disorders including, but not limited to, autoimmune and/or inflammatory disorders, which include Sjogren's syndrome, rheumatoid arthritis, lupus psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, Crohn's disease, reperfusion injury, septicemia, endotoxic shock, cystic fibrosis, endocarditis, psoriasis, arthritis (*e.g.,* psoriatic arthritis), anaphylactic shock, organ ischemia, reperfusion injury, spinal cord injury and allograft rejection. Other Examples of autoimmune and/or inflammatory disorders include, but are not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, Sjogren's syndrome, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, reperfusion injury, septicemia, endotoxic shock, cystic fibrosis, endocarditis, psoriasis, arthritis (*e.g*., psoriatic arthritis), anaphylactic shock, organ ischemia, reperfusion injury, spinal cord injury and allograft rejection. autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erythematosus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis. Examples of inflammatory disorders include, but are not limited to, asthma, encephilitis, inflammatory bowel disease, chronic obstructive pulmonary disease (COPD), allergic disorders, septic shock, pulmonary fibrosis, undifferentitated spondyloarthropathy, undifferentiated arthropathy, arthritis, inflammatory osteolysis, and chronic inflammation resulting from chronic viral or bacteria infections. The compositions and methods of the disclosure can be used with one or more conventional therapies that are used to prevent, manage or treat the above diseases.

The disclosure also provides methods of using the compositiosn of the disclosure to inactivate various infectious agents such as viruses, fungi, eukaryotic microbes, and bacteria. In some embodiments the compositions of the disclosure may be used to inactivate RSV, hMPV, PIV, or influenza viruses. In other embodiments, compositions of the disclosure may be used to inactivate fungal pathogens, such as, but not limited to members of *Naegleria, Aspergillus, Blastomyces, Histoplasma, Candida* or *Tinea* genera. In other embodiments, the compositions of the disclosure may be used to inactivate eukaryotic microbes, such as, but not limited to members of *Giardia, Toxoplasma, Plasmodium, Trypanosoma,* and *Entamoeba* genera. In other embodiments, compositions of the disclosure may be used to inactivate bacterial pathogens, such as but not limited to members of *Staphylococcus, Streptococcus, Pseudomonas, Clostridium, Borrelia, Vibro* and *Neiserria* genera.

The compositions of the disclosure are useful for many purposes, for example, as therapeutics against a wide range of chronic and acute diseases and disorders including, but not limited to, infectious disease, including viral, bacterial and fungal diseases. Examples of viral pathogens include but are not limited to: adenovirdiae (*e.g.,* mastadenovirus and aviadenovirus), herpesviridae (*e.g.,* herpes simplex virus 1, herpes simplex virus 2, herpes simplex virus 5, and herpes simplex virus 6), leviviridae (*e.g.,* levivirus, enterobacteria phase MS2, allolevirus), poxviridae (*e.g.,* chordopoxvirinae, parapoxvirus, avipoxvirus, capripoxvirus, leporiipoxvirus, suipoxvirus, molluscipoxvirus, and entomopoxvirinae), papovaviridae (*e.g.,* polyomavirus and papillomavirus), paramyxoviridae *(e.g.,* paramyxovirus, parainfluenza virus 1, mobillivirus (*e.g.,* measles virus), rubulavirus *(e.g.,* mumps virus), pneumonovirinae (*e.g.,* pneumovirus, human respiratory synctial virus), and metapneumovirus (*e.g.,* avian pneumovirus and human metapneumovirus)), picornaviridae (*e.g.,* enterovirus, rhinovirus, hepatovirus (*e.g.,* human hepatitis A virus), cardiovirus, and apthovirus), reoviridae (*e.g.,* orthoreovirus, orbivirus, rotavirus, cypovirus, fijivirus, phytoreovirus, and oryzavirus), retroviridae (*e.g.,* mammalian type B retroviruses, mammalian type C retroviruses, avian type C retroviruses, type D retrovirus group, BLV-HTLV retroviruses, lentivirus (*e.g.* human immunodeficiency virus 1 and human immunodeficiency virus 2), spumavirus), flaviviridae (*e.g.,* hepatitis C virus), hepadnaviridae (*e.g.,* hepatitis B virus), togaviridae (*e.g.,* alphavirus (*e.g.,* sindbis virus) and rubivirus *(e.g.,* rubella virus)), rhabdoviridae (*e.g.,* vesiculovirus, lyssavirus, ephemerovirus, cytorhabdovirus, and necleorhabdovirus), arenaviridae (*e.g.,* arenavirus, lymphocytic choriomeningitis virus, Ippy virus, and lassa virus), and coronaviridae (*e.g.,* coronavirus and torovirus). Examples of bacterial pathogens include but are not limited to: but not limited to, the *Aquaspirillum* family, *Azospirillum* family, *Azotobacteraceae* family, *Bacteroidaceae* family, *Bartonella* species, *Bdellovibrio* family, *Campylobacter* species, *Chlamydia* species (*e.g., Chlamydia pneumoniae*), clostridium, *Enterobacteriaceae* family (*e.g., Citrobacter* species, Edwardsiella, *Enterobacter aerogenes, Erwinia* species, *Escherichia coli,* Hafnia species, *Klebsiella* species, Morganella species, *Proteus vulgaris,* Providencia, *Salmonella* species, *Serratia marcescens,* and *Shigella flexneri*), *Gardinella* family, *Haemophilus influenzae, Halobacteriaceae* family, *Helicobacter* family, *Legionallaceae* family, *Listeria* species, *Methylococcaceae* family, mycobacteria (*e.g., Mycobacterium tuberculosis*), *Neisseriaceae* family, *Oceanospirillum* family, *Pasteurellaceae* family, *Pneumococcus* species, *Pseudomonas* species, *Rhizobiaceae* family, *Spirillum* family, *Spirosomaceae* family, *Staphylococcuss* (*e.g.,* methicillin resistant *Staphylococcus aureus* and *Staphylococcus pyrogenes), Streptococcus (e.g., Streptococcus enteritidis, Streptococcus fasciae,* and *Streptococcus pneumoniae*), *Vampirovibr Helicobacter* family, and *Vampirovibrio* family. Examples of fungal pathogens include, but are not limited to: *Absidia* species (*e.g., Absidia corymbifera and Absidia ramosa*), *Aspergillus* species, *(e.g., Aspergillus flavus, Aspergillus fumigatus, Aspergillus nidulans, Aspergillus niger,* and *Aspergillus terreus*), *Basidiobolus ranarum, Blastomyces dermatitidis, Candida* species (*e.g*., *Candida albicans, Candida glabrata, Candida kerr, Candida krusei, Candida parapsilosis, Candida pseudotropicalis, Candida quillermondii, Candida rugosa, Candida stellatoidea,* and *Candida tropicalis*), *Coccidioides immitis, Conidiobolus* species, *Cryptococcus neoforms, Cunninghamella* species, dermatophytes, *Histoplasma capsulatum, Microsporum gypseum, Mucor pusillus, Paracoccidioides brasiliensis, Pseudallescheria boydii, Rhinosporidium seeberi, Pneumocystis carinii, Rhizopus species (e.g., Rhizopus arrhizus, Rhizopus oryzae, and Rhizopus microsporus*), *Saccharomyces* species, *Sporothrix schenckii,* zygomycetes, and classes such as *Zygomycetes, Ascomycetes,* the *Basidiomycetes, Deuteromycetes,* and *Oomycetes.*

The disclosure also provides methods of using antibodies to deplete a cell population. In one embodiment, methods of the disclosure are useful in the depletion of the following cell types: eosinophil, basophil, neutrophil, T cell, B cell, mast cell, monocytes, endothelial cell and tumor cell. In some embodiments, antibodies of the disclosure deplete a respective cell population by at least 5%, 10%, 15%, 20%, 25%, 30%, 35%, 40%, 45%, 50%, 55%, 60%, 65%, 70%, 75%, 80%, 85%, 90%, 95%, or more as compared to a control non-engineered antibody or a conjugate thereof.

The antibodies of the disclosure and conjugates thereof may also be useful in the diagnosis and detection of diseases of symptoms thereof. In another embodiment, the compositions of the disclosure may be useful in the monitoring of disease progression. In another embodiment, the compositions of the disclosure may be useful in the monitoring of treatment regimens. In another embodiment, the compositions of the disclosure are useful for diagnosis in an *ex vivo* application, such as a diagnostic kit.

The compositions of the disclosure may be useful in the visualization of target antigens. In some embodiments, the target antigens are cell surface receptors that internalize. In other embodiments, the target antigen is an intracellular antigen. In other embodiments the target is an intranuclear antigen.

In one embodiment, the antibodies or antibody-drug conjugates of the disclosure once bound, internalize into cells wherein internalization is at least about 10%, at least about 20%, at least about 30%, at least about 40%, at least about 50%, at least about 60%, at least about 70%, at least about 80%, or at least about 90%, at least about 100%, at least about 110%, at least about 130%, at least about 140%, at least about 150%, at least about 160%, or at least about 170% more than control antibodies as described herein.

In another embodiment, the antibodies of the disclosure once bound, internalize into cells wherein internalization is 1-10%, 10-20%, 20 - 30%, 30- 40%,40- 50%, 50-60%, 60-70%, 70-80%, 80-90%, 90-100%, 100-110%, 110-120%, 120-130%, 130-140%, 140-150%, 150-160%, or 160-170% more than control antibodies as described herein.

In another embodiment, the antibodies of the disclosure once bound, internalize into cells wherein internalization is 1-10%, 10-20%, 20-30%, 30-40%,40-50%, 50-60%, 60-70%, 70-80%, 80-90%, 90-100%, 100-110%, 110-120%, 120-130%, 130-140%, 140-150%, 150-160%, or 160-170% more than control antibodies as determined by the internalization assay using a secondary antibody.

### Pharmaceutical Compositions

In another aspect, the present disclosure provides a composition, for example, but not limited to, a pharmaceutical composition, containing one or a combination of antibodies, or antibody conjugates of the present disclosure, formulated together with a pharmaceutically acceptable carrier. Such compositions may include one or a combination of, for example, but not limited to two or more different antibodies of the disclosure. For example, a pharmaceutical composition of the disclosure may comprise a combination of antibodies that bind to different epitopes on the target antigen or that have complementary activities.

Pharmaceutical compositions of the disclosure also can be administered in combination therapy, such as, combined with other agents. For example, the combination therapy can include an antibody of the present disclosure combined with at least one other therapy wherein the therapy may be surgery, immunotherapy, chemotherapy, radiation treatment, or drug therapy.

The pharmaceutical compounds of the disclosure may include one or more pharmaceutically acceptable salts. Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the disclosure also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and non-aqueous carriers that may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be suitable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

In one embodiment the compositions of the disclosure are pyrogen-free formulations which are substantially free of endotoxins and/or related pyrogenic substances. Endotoxins include toxins that are confined inside a microorganism and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight it is advantageous to remove even trace amounts of endotoxin. In one embodiment, endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg. In another embodiment, endotoxin and pyrogen levels in the composition are less then about 10 EU/mg, or less then about 5 EU/mg, or less then about 1 EU/mg, or less then about 0.1 EU/mg, or less then about 0.01 EU/mg, or less then about 0.001 EU/mg.

In one embodiment, the disclosure comprises administering a composition wherein said administration is oral, parenteral, intramuscular, intranasal, vaginal, rectal, lingual, sublingual, buccal, intrabuccal, intravenous, cutaneous, subcutaneous or transdermal.

In another embodiment the disclosure further comprises administering a composition in combination with other therapies, such as surgery, chemotherapy, hormonal therapy, biological therapy, immunotherapy or radiation therapy.

### Dosing/Administration

To prepare pharmaceutical or sterile compositions including an antibody or antibody conjugate of the disclosure, the antibody/antibody conjugate is mixed with a pharmaceutically acceptable carrier or excipient. Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, et al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present disclosure may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Compositions comprising antibodies or antibody conjugates of the disclosure can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, or at least 50 mg/kg (see, e.g., Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (20003) Cancer Immunol. Immunother. 52:133-144). The dose may be at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For antibodies or antibody conjugates of the disclosure, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the antibodies or antibody conjugates of the disclosure may be calculated using the patient's weight in kilograms (kg) multiplied by the dose to be administered in mg/kg. The dosage of the antibodies of the disclosure may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the antibodies or antibody conjugates of the disclosure may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the antibodies or antibody conjugates of the disclosure may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the antibodies of the disclosure may achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of antibodies or antibody conjugates of the disclosure may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side affects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350466 and 6,316,024). Where necessary, the composition may also include a solubilizing agent and a local anesthetic such as lidocaine to ease pain at the site of the injection. In addition, pulmonary administration can also be employed, *e.g.,* by use of an inhaler or nebulizer, and formulation with an aerosolizing agent. See, *e.g*., U.S. Pat. Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety. In one embodiment, an antibody, combination therapy, or a composition of the disclosure is administered using Alkermes AIR™ pulmonary drug delivery technology (Alkermes, Inc., Cambridge, Mass.).

A composition of the present disclosure may also be administered via one or more routes of administration using one or more of a variety of methods known in the art. As will be appreciated by the skilled artisan, the route and/or mode of administration will vary depending upon the desired results. Selected routes of administration for antibodies of the disclosure include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the disclosure can be administered via a non-parenteral route, such as a topical, epidermal or mucosal route of administration, for example, intranasally, orally, vaginally, rectally, sublingually or topically.

If the antibodies of the disclosure or conjugates thereof are administered in a controlled release or sustained release system, a pump may be used to achieve controlled or sustained release (see Langer, supra; Sefton, 1987, CRC Crit. Ref. Biomed. Eng. 14:20; Buchwald et al., 1980, Surgery 88:507; Saudek et al., 1989, N. Engl. J. Med. 321:574). Polymeric materials can be used to achieve controlled or sustained release of the therapies of the disclosure (see *e.g*., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more antibodies of the disclosure or conjugates thereof. See, *e.g.,* U.S. Pat. No. 4,526,938, PCT publication WO 91/05548, PCT publication WO 96/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760, each of which is incorporated herein by reference in their entirety.

If the antibody or antibody conjugate of the disclosure is administered topically, it can be formulated in the form of an ointment, cream, transdermal patch, lotion, gel, shampoo, spray, aerosol, solution, emulsion, or other form well-known to one of skill in the art. See, *e.g.,* Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (*e.g.,* preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (*e.g.,* a gaseous propellant, such as freon) or in a squeeze bottle. Moisturizers or humectants can also be added to pharmaceutical compositions and dosage forms if desired. Examples of such additional ingredients are well-known in the art.

If the compositions comprising antibodies or antibody conjugates are administered intranasally, it can be formulated in an aerosol form, spray, mist or in the form of drops. In particular, prophylactic or therapeutic agents for use according to the present disclosure can be conveniently delivered in the form of an aerosol spray presentation from pressurized packs or a nebuliser, with the use of a suitable propellant (*e.g.,* dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are well known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (*e.g.,* prophylactic or therapeutic agents), which can be administered in combination with the antibodies of the disclosure or conjugates thereof, may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the antibodies of the disclosure. The two or more therapies may be administered within one same patient visit.

The antibodies or antibody conjugates of the disclosure and the other therapies may be cyclically administered. Cycling therapy involves the administration of a first therapy (*e.g.,* a first prophylactic or therapeutic agent) for a period of time, followed by the administration of a second therapy (*e.g.,* a second prophylactic or therapeutic agent) for a period of time, optionally, followed by the administration of a third therapy (*e.g.,* prophylactic or therapeutic agent) for a period of time and so forth, and repeating this sequential administration, *i.e*., the cycle in order to reduce the development of resistance to one of the therapies, to avoid or reduce the side effects of one of the therapies, and/or to improve the efficacy of the therapies.

In certain embodiments, the antibodies and antibody conjugates of the disclosure can be formulated to ensure proper distribution *in vivo.* For example, the blood-brain barrier (BBB) excludes many highly hydrophilic compounds. To ensure that the therapeutic compounds of the disclosure cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273.

The disclosure provides protocols for the administration of pharmaceutical composition comprising antibodies or antibody conjugates of the disclosure alone or in combination with other therapies to a subject in need thereof. The therapies (e.g., prophylactic or therapeutic agents) of the combination therapies of the present disclosure can be administered concomitantly or sequentially to a subject. The therapy (e.g., prophylactic or therapeutic agents) of the combination therapies of the present disclosure can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the disclosure can be administered to a subject concurrently. The term "concurrently" is not limited to the administration of therapies (e.g., prophylactic or therapeutic agents) at exactly the same time, but rather it is meant that a pharmaceutical composition comprising antibodies or antibody conjugates of the disclosure are administered to a subject in a sequence and within a time interval such that the antibodies of the disclosure or conjugates thereof can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

### Equivalents

The foregoing written specification is considered to be sufficient to enable one skilled in the art to practice the disclosure. The foregoing description and Examples detail certain exemplary embodiments of the disclosure. It will be appreciated, however, that no matter how detailed the foregoing may appear in text, the disclosure may be practiced in many ways and the disclosure should be construed in accordance with the appended claims and any equivalents thereof.

All references cited herein, including patents, patent applications, papers, text books, and the like, and the references cited therein, to the extent that they are not already, are hereby incorporated herein by reference in their entirety.

### Exemplary Embodiments

1. A cysteine engineered antibody, wherein the cysteine engineered antibody comprises a substitution of one or more amino acids to a cysteine residue in at least one surface amino acid residue in the constant region of an antibody heavy chain, wherein the cysteine engineered antibody comprises at least one thiol group.
2. The cysteine engineered antibody of embodiment 1, wherein said antibody comprises 2 or more thiol groups.
3. The cysteine engineered antibody of embodiment 1, wherein said antibody comprises 4 or more thiol groups.
4. The cysteine engineered antibody of embodiment 1, wherein said antibody comprises 6 or more thiol groups.
5. The cysteine engineered antibody of embodiment 1, wherein said antibody comprises 8 or more thiol groups.
6. The cysteine engineered antibody of embodiment 1, wherein said antibody comprises 10 or more thiol groups.
7. The cysteine engineered antibody of embodiment 1, wherein said antibody comprises 12 or more thiol groups.
8. The cysteine engineered antibody of embodiment 1, wherein said antibody comprises 14 or more thiol groups.
9. The cysteine engineered antibody of embodiment 1, wherein said antibody comprises 16 or more thiol groups.
10. The cysteine engineered antibody of embodiment 1, wherein said antibody comprises 18 or more thiol groups.
11. The cysteine engineered antibody of embodiment 1, wherein said antibody comprises 20 or more thiol groups.
12. The cysteine engineered antibody of embodiment 1, wherein said antibody comprises 22 or more thiol groups.
13. The cysteine engineered antibody of embodiment 1, wherein said antibody comprises 24 or more thiol groups.
14. The cysteine engineered antibody of embodiment 1, wherein said antibody comprises 26 or more thiol groups.
15. The cysteine engineered antibody of embodiment 1, wherein said antibody comprises 28 or more thiol groups.
16. The cysteine engineered antibody of embodiment 1, wherein said antibody comprises 30 or more thiol groups.
17. The cysteine engineered antibody of embodiment 1, wherein said antibody comprises 32 or more thiol groups.
18. The cysteine engineered antibody of embodiment 1, wherein said antibody comprises 34 or more thiol groups.
19. The cysteine engineered antibody of embodiment 1, wherein said antibody comprises 36 or more thiol groups.
20. The cysteine engineered antibody of embodiment 1, wherein said antibody comprises 38 or more thiol groups.
21. The cysteine engineered antibody of embodiment 1, wherein said antibody comprises 40 or more thiol groups.
22. The cysteine engineered antibody of any of embodiments 1-21, wherein said surface amino acid residue is chosen from positions 239, 282, 289, 297, 312, 324, 330, 335, 337, 339, 356, 359, 361, 383, 384, 398, 400, 440, 422, and 442.
23. The cysteine engineered antibody of any of embodiments 1-22, wherein said antibody further comprises a substitution to a cysteine residue at a position chosen from positions 131, 132, 133, 134, 135, 136, 137, 138, and 139 of the CH1 domain of the antibody.
24. The cysteine engineered antibody of any of embodiments 1-23, wherein said cysteine engineered antibody exhibits the same or greater binding affinity for a specific target as the antibody prior to cysteine engineering.
25. The cysteine engineered antibody of any of embodiments 1-4, wherein said cysteine engineered antibody exhibits a lower binding affinity for a specific target as the antibody prior to cysteine engineering.
26. The cysteine engineered antibody of any of embodiments 1-25, wherein said cysteine engineered antibody exhibits the same, lower, or greater binding affinity for one or more IgG Fc receptor as the antibody prior to cysteine engineering.
27. The cysteine engineered antibody of any of embodiments 1-26, wherein said cysteine engineered antibody induces the same or greater level of antibody dependent cellular cytotoxicity (ADCC) as the antibody prior to cysteine engineering.
28. The cysteine engineered antibody of any of embodiments 1-26, wherein said cysteine engineered antibody induces a lower level of antibody dependent cellular cytotoxicity (ADCC) as the antibody prior to cysteine engineering.
29. The cysteine engineered antibody of any of embodiments 1-27, wherein said cysteine engineered antibody induces the same or greater level of antibody dependent complement dependent cytotoxicity (CDC) as the antibody prior to cysteine engineering.
30. The cysteine engineered antibody of any of embodiments 1-27, wherein said cysteine engineered antibody induces the same or a lower level of antibody dependent complement dependent cytotoxicity (CDC) as the antibody prior to cysteine engineering.
31. The cysteine engineered antibody of any of embodiments 1-30, wherein said cysteine engineered antibody exhibits the same or greater level of stability measured by fragmentation, Tm, and/or aggregation profile as the antibody prior to cysteine engineering.
32. The cysteine engineered antibody of any of embodiments 1-30, wherein said cysteine engineered antibody exhibits a lower level of stability measured by fragmentation, Tm, and/or aggregation profile as the antibody prior to cysteine engineering.
33. The cysteine engineered antibody of any of embodiments 1-32, wherein said engineered antibody exhibits improved or reduced half-life as compared to the antibody prior to cysteine engineering.
34. The cysteine engineered antibody of any of embodiments 1-33, wherein said thiol group is capable of chemical conjugation to a cytotoxic agent, chemotherapeutic agent, toxin, radionuclide, DNA, RNA, siRNA, microRNA, peptide nucleic acid, non-natural amino acid, peptide, enzyme, fluorescent tag, or biotin.
35. The cysteine engineered antibody of embodiment 34, wherein said cytotoxic agent is chosen from an anti-tubulin agent, a DNA minor groove binder, an anti-mitmaytansanoid, and an auristatin.
36. The cysteine engineered antibody of embodiment 34, wherein said chemotherapeutic agent is chosen from taxol, paclitaxel, doxorubicin, methotrexate, dolastatin, vinka alkaloids, methotrexate, and duocarmycin.
37. The cysteine engineered antibody of embodiment 34, wherein said toxin is chosen from abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
38. The cysteine engineered antibody of embodiment 34, wherein said radionuclide is chosen from chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
39. The cysteine engineered antibody of embodiment 34, wherein said antibody is an internalizing antibody.
40. The cysteine engineered antibody of any of embodiments 1-39, wherein said antibody is a monoclonal, chimeric, humanized, fully human, bispecific, multispecific antibody, or antibody-like molecule.
41. An isolated nucleic acid comprising a nucleotide sequence encoding a heavy chain variable domain or a light chain variable domain of cysteine engineered antibody of any of embodiments 1-40.
42. A vector comprising the nucleic acid of embodiment 41
43. A host cell comprising the vector of embodiment 42.
44. An antibody conjugate of the cysteine engineered antibodies of any of embodiments 1-40
45. A pharmaceutical composition comprising the antibody conjugate of embodiment 44.
46. A method of detecting cancer, autoimmune, inflammatory, or infectious diseases or disorders in a subject in need thereof, said method comprising administering to said subject the composition of embodiment 45.
47. The method of embodiment 46, wherein said disease or disorder comprises cells that overexpress a cell surface antigen that is bound by said antibody conjugate.
48. A method of inhibiting proliferation of a target cell, said method comprising contacting said cell with an effective amount of the antibody conjugate of embodiment 44.
49. A method of inhibiting proliferation of a target cell in a subject, said method comprising administering an effective amount of the composition of embodiment 45.
50. The method of embodiment 48 or 49, wherein said target cell overexpresses a cell surface antigen that is bound by said antibody conjugate.
51. A method of treating cancer, autoimmune, inflammatory, or infectious diseases or disorders in a subject in need thereof, said method comprising administering to said subject a therapeutically effective amount of the composition of embodiment 45.
52. The method of embodiment 51, wherein said disease or disorder comprises cells that overexpress a cell surface antigen that is bound by said antibody conjugate.
53. The method of embodiment 51, wherein said method comprises killing or reducing the growth rate of cells associated with said diseases.
54. The method of embodiment 51, wherein said method comprises depleting B cells or T cells.
55. The method of embodiment 51 comprising the administration of an additional therapy, wherein said additional therapy is chosen from chemotherapy, biological therapy, immunotherapy, radiation therapy, hormonal therapy, and surgery.
56. A method for efficiently conjugating a heterologous molecule to the cysteine engineered antibodies of any of embodiments 1-40.
57. The method of embodiment 56, wherein said method comprises conjugating said heterologous molecule to at least one position chosen from 239, 282, 289, 297, 312, 324, 330, 335, 337, 339, 356, 359, 361, 383, 384, 398, 400, 440, 422, and 442 of the Fc region of the antibody.
58. The method of embodiment 56 or 57, wherein said heterologous molecule is chosen from a cytotoxic agent, chemotherapeutic agent, toxin, radionuclide, DNA, RNA, siRNA, microRNA, peptide nucleic acid, peptide, enzyme, fluorescent tag, or biotin.
59. The method of embodiment 58, wherein said cytotoxic agent is chosen from an anti-tubulin agent, a DNA minor groove binder, an anti-mitmaytansanoid, and an auristatin.
60. The method of embodiment 58, wherein said chemotherapeutic agent is chosen from taxol, paclitaxel, doxorubicin, methotrexate, dolastatin, vinka alkaloids, and methotrexate.
61. The method of embodiment 58, wherein said toxin is chosen from abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
62. The method of embodiment 58, wherein said radionuclide is chosen from chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho) indium (¹¹⁵In, ¹¹³In, ¹¹²In, ¹¹¹In), iodine (¹³¹I, ¹²⁵I, ¹²³I, ¹²¹I) lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (^{8S}Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
63. The method of any of embodiments 56-62, wherein said conjugation efficiency is at least 5% or more as measured by residual thiol groups remaining after the conjugation reaction.
64. The method of any of embodiments 56-63, wherein said efficiency is at least 25% or more as measured by residual thiol groups remaining after the conjugation reaction.
65. The method of any of embodiments 56-64, wherein said efficiency is at least 75% or more as measured by residual thiol groups remaining after the conjugation reaction.
66. An engineered antibody, wherein the engineered antibody comprises a substitution of one or more amino acids to a non-naturally occurring residue in at least one surface amino acid residue on the CH2 and/or CH3 domain of the Fc region of an antibody,
   wherein the engineered antibody comprises at least one reactive group, and
   wherein the non-naturally occurring residue is chosen from cysteine, lysine, tyrosine, histidine, selenocysteine, selenomethionine, and non-natural amino acids.
67. The engineered antibody of embodiment 66, wherein said antibody comprises 2 or more substitutions.
68. The engineered antibody of embodiment 66, wherein said antibody comprises 4 or more substitutions.
69. The engineered antibody of embodiment 66, wherein said antibody comprises 6 or more substitutions.
70. The engineered antibody of embodiment 66, wherein said antibody comprises 8 or more substitutions.
71. The engineered antibody of embodiment 66, wherein said antibody comprises 10 or more substitutions.
72. The engineered antibody of embodiment 66, wherein said antibody comprises 12 or more substitutions.
73. The engineered antibody of embodiment 66, wherein said antibody comprises 14 or more substitutions.
74. The engineered antibody of embodiment 66, wherein said antibody comprises 16 or more substitutions.
75. The engineered antibody of embodiment 66, wherein said antibody comprises 18 or more substitutions.
76. The engineered antibody of embodiment 66, wherein said antibody comprises 20 or more substitutions.
77. The engineered antibody of embodiment 66, wherein said antibody comprises 22 or more substitutions.
78. The engineered antibody of embodiment 66, wherein said antibody comprises 24 or more substitutions.
79. The engineered antibody of embodiment 66, wherein said antibody comprises 26 or more substitutions.
80. The engineered antibody of embodiment 66, wherein said antibody comprises 28 or more substitutions.
81. The engineered antibody of embodiment 66, wherein said antibody comprises 30 or more substitutions.
82. The engineered antibody of embodiment 66, wherein said antibody comprises 32 or more substitutions.
83. The engineered antibody of embodiment 66, wherein said antibody comprises 34 or more substitutions.
84. The engineered antibody of embodiment 66, wherein said antibody comprises 36 or more substitutions.
85. The engineered antibody of embodiment 66, wherein said antibody comprises 38 or more substitutions.
86. The engineered antibody of embodiment 66, wherein said antibody comprises 40 or more substitutions.
87. The engineered antibody of any of embodiments 66-86, wherein said surface amino acid residue is chosen from 239, 282, 289, 297, 312, 324, 330, 335, 337, 339, 356, 359, 361, 383, 384, 398, 400, 440, 422, and 442.
88. The engineered antibody of any of embodiments 66-87, wherein said antibody further comprises a substitution to cysteine at position chosen from 131, 132, 133, 134, 135, 136, 137, 138, and 139 of the CH1 domain of the antibody.
89. The engineered antibody of any of embodiments 66-88, wherein said engineered antibody exhibits the same or greater binding affinity for a specific target as the antibody prior to engineering.
90. The engineered antibody of any of embodiments 66-89, wherein said engineered antibody exhibits the lower affinity for a specific target as the antibody prior to engineering.
91. The engineered antibody of any of embodiments 66-90, wherein said engineered antibody exhibits the same, reduced, or greater binding affinity for one or more IgG Fc receptor as the antibody prior to engineering.
92. The engineered antibody of any of embodiments 66-91, wherein said engineered antibody induces the same or greater level of antibody dependent cellular cytotoxicity (ADCC) as the antibody prior to engineering.
93. The engineered antibody of any of embodiments 66-91, wherein said engineered antibody induces a lower level of antibody dependent cellular cytotoxicity (ADCC) as the antibody prior to engineering.
94. The engineered antibody of any of embodiments 66-91, wherein said engineered antibody induces the same or greater level of antibody dependent complement dependent cytotoxicity (CDC) as the antibody prior to engineering.
95. The engineered antibody of any of embodiments 66-91, wherein said engineered antibody induces a lower level of antibody dependent complement dependent cytotoxicity (CDC) as the antibody prior to engineering.
96. The engineered antibody of any of embodiments 66-95, wherein said engineered antibody exhibits the same or greater level of stability measured by fragmentation, Tm, and/or aggregation profile as the antibody prior to engineering.
97. The engineered antibody of any of embodiments 66-95, wherein said engineered antibody exhibits a lower level of stability measured by fragmentation, Tm, and/or aggregation profile as the antibody prior to engineering.
98. The engineered antibody of any of embodiments 66-97, wherein said engineered antibody exhibits increased or reduced half-life as compared to the antibody prior to engineering.
99. The engineered antibody of any of embodiments 66-98, wherein said thiol group is capable of chemical conjugation to a cytotoxic agent, chemotherapeutic agent, toxin, radionuclide, DNA, RNA, siRNA, microRNA, peptide nucleic acid, non-natural amino acid, peptide, enzyme, fluorescent tag, or biotin.
100. The engineered antibody of embodiment 99, wherein said cytotoxic agent is chosen from an anti-tubulin agent, a DNA minor groove binder, an anti-mitmaytansanoid, and an auristatin.
101. The engineered antibody of embodiment 99, wherein said chemotherapeutic agent is chosen from taxol, paclitaxel, doxorubicin, methotrexate, dolastatin, vinka alkaloids, methotrexate, and duocarmycin.
102. The engineered antibody of embodiment 99, wherein said toxin is chosen from abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
103. The engineered antibody of embodiment 99, wherein said radionuclide is chosen from chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I) lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (^{8S}Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
104. The engineered antibody of embodiment 99, wherein said antibody is an internalizing antibody.
105. The engineered antibody of any of embodiments 66-104, wherein said antibody is a monoclonal, chimeric, humanized, fully human, bispecific, multispecific antibody, or antibody-like molecule.
106. An isolated nucleic acid comprising a nucleotide sequence encoding a heavy chain variable domain or a light chain variable domain of engineered antibody of any of embodiments 66-105.
107. A vector comprising the nucleic acid of embodiment 106
108. A host cell comprising the vector of embodiment 107.
109. An antibody conjugate of the engineered antibodies of any of embodiments 66-105.
110. A pharmaceutical composition comprising the antibody conjugate of embodiment 109.
111. A method of detecting cancer, autoimmune, inflammatory, or infectious diseases or disorders in a subject in need thereof, said method comprising administering to said subject the composition of embodiment 110.
112. The method of embodiment 111, wherein said disease or disorder comprises cells that overexpress a cell surface antigen that is bound by said antibody conjugate.
113. A method of inhibiting proliferation of a target cell, said method comprising contacting said cell with an effective amount of the antibody conjugate of embodiment 109.
114. A method of inhibiting proliferation of a target cell in a subject, said method comprising administering an effective amount of the composition of embodiment 110.
115. The method of embodiment 113 or 114, wherein said target cell overexpresses a cell surface antigen that is bound by said antibody conjugate.
116. A method of treating cancer, autoimmune, inflammatory, or infectious diseases or disorders in a subject in need thereof, said method comprising administering to said subject a therapeutically effective amount of the composition of embodiment 110.
117. The method of embodiment 116, wherein said disease or disorder comprises cells that overexpress a cell surface antigen that is bound by said antibody conjugate.
118. The method of embodiment 116, wherein said method comprises killing or reducing the growth rate of cells associated with said diseases.
119. The method of embodiment 116, wherein said method comprises depleting B cells or T cells.
120. The method of embodiment 116 comprising the administration of an additional therapy, wherein said additional therapy is chosen from chemotherapy, biological therapy, immunotherapy, radiation therapy, hormonal therapy, and surgery.
121. A method for efficiently conjugating a heterologous molecule to the engineered antibodies of any of embodiments 66-105.
122. The method of embodiment 121, wherein said method comprises conjugating said heterologous molecule to at least one position chosen from 239, 282, 289, 297, 312, 324, 330, 335, 337, 339, 356, 359, 361, 383, 384, 398, 400, 440, 422, and 442 of the Fc region of the antibody.
123. The method of embodiment 121 or 122, wherein said heterologous molecule is chosen from a cytotoxic agent, chemotherapeutic agent, toxin, radionuclide, DNA, RNA, siRNA, microRNA, peptide nucleic acid, peptide, enzyme, fluorescent tag, or biotin.
124. The method of embodiment 123, wherein said cytotoxic agent is chosen from an anti-tubulin agent, a DNA minor groove binder, an anti-mitmaytansanoid, and an auristatin.
125. The method of embodiment 123, wherein said chemotherapeutic agent is chosen from taxol, paclitaxel, doxorubicin, methotrexate, dolastatin, vinka alkaloids, and methotrexate.
126. The method of embodiment 123, wherein said toxin is chosen from abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
127. The method of embodiment 123, wherein said radionuclide is chosen from chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I) lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (^{8S}Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
128. The method of any of embodiments 121-127, wherein said efficiency is at least 5% or more as measured by residual thiol groups remaining after the conjugation reaction.
129. The method of any of embodiments 121-128, wherein said efficiency is at least 25% or more as measured by residual thiol groups remaining after the conjugation reaction.
130. The method of any of embodiments 121-129, wherein said efficiency is at least 75% or more as measured by residual thiol groups remaining after the conjugation reaction.
131. An Fc region of an antibody, wherein the Fc region comprises a substitution of one or more amino acids chosen from positions 239, 282, 289, 297, 312, 324, 330, 335, 337, 339, 356, 359, 361, 383, 384, 398, 400, 440, 422, and 442.
132. The Fc region of embodiment 131, wherein the Fc region comprises one or more of the following pairs of substitutions:
   a) 289 and 440;
   b) 330 and 440;
   c) 339 and 440;
   d) 359 and 440;
   e) 289 and 359;
   f) 330 and 359;
   g) 339 and 359;
   h) 289 and 339;
   i) 330 and 339;
   j) 289 and 330; and
   k) 339 and 442.
133. The Fc region of any of embodiments 131 and 132, wherein the Fc region comprises one or more of the following groups of substitutions:
   a) 289, 339, and 442;
   b) 289, 330, and 339;
   c) 330, 339, and 442; and
   d) 289, 330, and 442.
134. The Fc region of any of embodiments 131-133, wherein the Fc region is chosen from an IgG1, IgG2, IgG3, or an IgG4 isotype.
135. The Fc region any of embodiments 131-134, wherein the substitution comprises a substitution to an amino acid chosen from cysteine, lysine, tyrosine, histidine, selenocysteine, and selenomethionine.
136. The Fc region of any of embodiments 1-134, wherein the substitution comprises cysteine.
137. The Fc region of embodiment 136, wherein the cysteine comprises a thiol group.
138. The Fc region of embodiment 137, wherein the thiol group is capable of chemical conjugation.
139. The Fc region of claim 138, wherein the Fc region is conjugated to one or more of a cytotoxic agent, chemotherapeutic agent, toxin, radionuclide, DNA, RNA, siRNA, microRNA, peptide nucleic acid, non-natural amino acid, peptide, enzyme, fluorescent tag, and biotin.
140. An antibody or antigen binding fragment thereof comprising the Fc region of any of embodiments 1-139.
141. The antibody or antigen binding fragment thereof of embodiment 140, wherein the antibody is a monoclonal, chimeric, humanized, fully human, bispecific, multispecific antibody, or antibody-like molecule.
142. The antibody or antigen binding fragment thereof of embodiment 140, wherein the antibody further comprises a substitution of one or more amino acids chosen from positions 131, 132, 133, 134, 135, 136, 137, 138, and 139 of the CH1 domain of the antibody.
143. A pharmaceutical composition comprising the antibody or antigen binding fragment thereof of any of embodiments 131-142.
144. A method of detecting cancer, autoimmune, inflammatory, or infectious diseases or disorders in a subject in need thereof, said method comprising administering to said subject an antibody or an antigen binding fragment thereof, wherein the engineered antibody or antigen binding fragment thereof comprises a substitution of one or more amino acids chosen from positions 239, 282, 289, 297, 312, 324, 330, 335, 337, 339, 356, 359, 361, 383, 384, 398, 400, 440, 422, and 442 of the heavy chain.
145. A method of treating cancer, autoimmune, inflammatory, or infectious diseases or disorders in a subject in need thereof, said method comprising administering to said subject a therapeutically effective amount of an antibody or an antigen binding fragment thereof, wherein the antibody or binding fragment thereof comprises a substitution of one or more amino acids chosen from positions 239, 282, 289, 297, 312, 324, 330, 335, 337, 339, 356, 359, 361, 383, 384, 398, 400, 440, 422, and 442 of the heavy chain.
146. A nucleic acid comprising a sequence encoding the Fc region of any of embodiments 1-145.
147. The nucleic acid of embodiment 146, wherein the nucleic acid comprises a sequence encoding an amino acid sequence chosen from the sequence of SEQ ID NOs: 1-24.
148. A host cell comprising the nucleic acid of any of embodiments 146 and 147.
149. The host cell of embodiment 148, wherein the cell is a mammalian cell.
150. A method of producing an antibody or an antigen binding fragment thereof comprising incubating the host cell of embodiment 148 under suitable conditions for expressing the antibody or antigen binding fragment, and isolating the antibody or antigen binding fragment.
151. A composition comprising the Fc region of an antibody, wherein the Fc region comprises a substitution of one or more amino acids chosen from positions 239, 282, 289, 297, 312, 324, 330, 335, 337, 339, 356, 359, 361, 383, 384, 398, 400, 440, 422, and 442.
152. The Fc region of embodiment 151, wherein the Fc region comprises one or more of the following pairs of substitutions:
   a) 289 and 440;
   b) 330 and 440;
   c) 339 and 440;
   d) 359 and 440;
   e) 289 and 359;
   f) 330 and 359;
   g) 339 and 359;
   h) 289 and 339;
   i) 330 and 339;
   j) 289 and 330; and
   k) 339 and 442.
153. The Fc region of any of embodiments 151 and 152, wherein the Fc region comprises one or more of the following groups of substitutions:
   a) 289, 339, and 442;
   b) 289, 330, and 339;
   c) 330, 339, and 442; and
   d) 289, 330, and 442.
154. The Fc region of any of embodiments 151-153, wherein the Fc region is chosen from an IgGI, IgG2, IgG3, or an IgG4 isotype.
155. The Fc region any of embodiments 151-154, wherein the substitution comprises a substitution to an amino acid chosen from cysteine, lysine, tyrosine, histidine, selenocysteine, and selenomethionine.
156. The Fc region of any of embodiments 1-154, wherein the substitution comprises cysteine.
157. The Fc region of embodiment 156, wherein the cysteine comprises a thiol group.
158. The Fc region of embodiment 157, wherein the thiol group is capable of chemical conjugation.
159. The Fc region of claim 158, wherein the Fc region is conjugated to one or more of a cytotoxic agent, chemotherapeutic agent, toxin, radionuclide, DNA, RNA, siRNA, microRNA, peptide nucleic acid, non-natural amino acid, peptide, enzyme, fluorescent tag, and biotin.
160. An antibody or antigen binding fragment thereof comprising the Fc region of any of embodiments 1-159.
161. The antibody or antigen binding fragment thereof of embodiment 160, wherein the antibody is a monoclonal, chimeric, humanized, fully human, bispecific, multispecific, antibody, or antibody-like molecule.
162. The antibody or antigen binding fragment thereof of embodiment 160, wherein the antibody further comprises a substitution of one or more amino acids chosen from positions 131, 132, 133, 134, 135, 136, 137, 138, and 139 of the CH1 domain of the antibody.
163. A pharmaceutical composition comprising the antibody or antigen binding fragment thereof of any of embodiments 151-162.
164. A method of detecting cancer, autoimmune, inflammatory, or infectious diseases or disorders in a subject in need thereof, said method comprising administering to said subject an antibody or an antigen binding fragment thereof, wherein the engineered antibody or antigen binding fragment thereof comprises a substitution of one or more amino acids chosen from positions 239, 282, 289, 297, 312, 324, 330, 335, 337, 339, 356, 359, 361, 383, 384, 398, 400, 440, 422, and 442 of the heavy chain.
165. A method of treating cancer, autoimmune, inflammatory, or infectious diseases or disorders in a subject in need thereof, said method comprising administering to said subject a therapeutically effective amount of an antibody or an antigen binding fragment thereof, wherein the antibody or binding fragment thereof comprises a substitution of one or more amino acids chosen from positions 239, 282, 289, 297, 312, 324, 330, 335, 337, 339, 356, 359, 361, 383, 384, 398, 400, 440, 422, and 442 of the heavy chain.
166. A nucleic acid comprising a sequence encoding the Fc region of any of embodiment 131-165.
167. The nucleic acid of embodiment 166, wherein the nucleic acid comprises a sequence encoding an amino acid sequence chosen from the sequence of SEQ ID NOs: 1-24.
168. A host cell comprising the nucleic acid of any of embodiments 166 and 167.
169. The host cell of embodiment 168, wherein the cell is a mammalian cell.
170. A method of producing an antibody or an antigen binding fragment thereof comprising incubating the host cell of embodiment 168 under suitable conditions for expressing the antibody or antigen binding fragment, and isolating the antibody or antigen binding fragment.
171. A composition comprising an amino acid sequence chosen from SEQ ID NOs: 1-24.

### 8. EXAMPLES

The disclosure is now described with reference to the following examples. These examples are provided for the purpose of illustration only and the disclosure should in no way be construed as being limited to these examples but rather should be construed to encompass any and all variations which become evident as a result of the teachings provided herein.

### 8.1 Example 1. Expression and characterization of single variant cysteine engineered antibodies

A series of cysteine substitutions were made to the surface regions of the CH2 and CH3 domain of an IgG 1 molecule (see Figure 1) and the surface-exposed regions of the Fc cavity (e.g., Leu398). The cysteine engineered IgG1 molecules were generated using standard DNA recombinant technologies (See, e.g. Sambrook et al. Molecular Cloning-A Laboratory Manual, December 2000, Cold Spring Harbor Lab Press). The surface regions of the CH2 and CH3 domains present in an IgG1 molecule represents regions which are solvent exposed. The exposure to solvent that these regions display permits the conjugation of reagents to the specific residues within the regions. Various amino acids that are predicted to be on the surface of the CH2 and/or the CH3 domain are particular candidate amino acids to be substituted with cysteine residues.

In Figure 2, the ND1 wild type and cysteine engineered derivatives thereof were expressed, purified and subjected to PAGE analysis. The ND1 native antibody (Lane 1) and various cysteine engineered variants thereof (Lanes 2-21, corresponding to clones ND2-21) exhibited very similar molecular weight profiles under reducing conditions. These results suggest that the recombinant cysteine engineered antibodies express well and retain their predicted molecular weight compared to control lanes.

In Figure 3, all recombinant cysteine engineered proteins were purified by protein A, dialyzed into PBS 1X, 10 mM EDTA, pH 7.2, and analyzed on SEC-HPLC. The identity of the samples is schematically labeled on the figure. Peak position of standard proteins (dotted chromatogram) from right to left are thyroglobulin 670 KDa; 2, bovine gamma-globulin 158 KDa; 3, chicken ovalbumin 44 KDa; 4, equine myoglobin 17 KDa; 5, vitamin B12 13.5 KDa. The mutant antibodies retention time is about 8.6 minutes, which correspond to a molecular weight of about 150 KDa. The percentage of monomeric content for each mutant is shown. Each variant (ND2-21) displayed a similar SEC elution profile as compared with the native antibody (ND-1). This analysis confirms that the recombinant proteins in addition of retaining their expected molecular weight, do not form disulfide-linked homodimers due to the introduced cysteines.

Figure 4 shows the sequence of the heavy chain constant domain sequence of ND 1 (native). Fig. 4a shows the sequences of variants ND2 through ND6. Fig. 4b shows the sequences of variants ND7 through ND 11. Fig. 4c shows the sequences of variants ND12 through ND16. Fig. 4d shows the sequences of variants ND 17 through ND21. The introduced point mutations of ND2 to ND21 are shown underlined and in bold.

ND1 to ND21 have SEQ ID NOs: 1 to 21, respectively. Each is derived from the HC Fc for human IgG1.

Figure 5 shows recombinant expression of Fc cysteine variants. Each of the variants had an expression level comparable to the native ND 1. Furthermore, each variant except ND5 was expressed at or greater than 93% monomer (determined by SEC-HPLC). They also had molecular weight and hydrodynamic radius similar to the native ND 1 (determined by MALLS) (data not shown).

Figure 6 shows FcRn binding of the cysteine Fc-engineered variants as determined by enzyme-linked immunoabsorbent assay (ELISA). Apart from variant ND5 (D312C) that is 70 % monomeric, and the non-glycosylated variant ND20 (N297C), the variants retain binding signal to FcRn similar to the native antibody ND1.

Differential scanning calorimetry (DSC) was used to determine the temperature of melting (Tm) for native and cysteine engineered antibodies as shown in Figure 7. DSC experiments measured the heat capacity of the antibodies of the disclosure as a function of temperature in a range from 10°C to 110°C. DSC measurements were carried out using a Microcal VP-DSC ultrasensitive scanning microcalorimeter. DSC experiments were carried out in 25 mM Histidine-HCl pH6, 5 mM EDTA. The solutions and samples used for DSC were filtered using a 0.22 micron-filter and degassed just prior to loading into the calorimeter. For each set of measurements, buffer-versus baseline runs were first obtained. Immediately after this, the buffer solution was removed from the sample cell. The sample cells were loaded with 0.5 ml of an antibody (native and cysteine engineered) solution at concentrations ranging from 0.5 to 1 mg/ml. During measurement the reference cell was filled with the sample buffer. From each sample-versus-buffer experiment, the corresponding buffer-versus-buffer baseline run was subtracted. The raw data were normalized for concentration and scan rate. The data were fitted using the Origin DSC software provided by Microcal.

The DSC analysis of ND1-21 shown in Fig. 7 was conducted using a buffer of 25 mM His, pH 6.0, a range from 15°C to 110°C, and a rate of 1°C / min. Except variants ND5 (D312C), ND11 (E356C), and ND20 (N297C), the variants have similar DSC profiles to the native ND1.

Figure 8 shows various protocols for the conjugation of biotin-PEG2-maleimide to engineered antibodies and the control ND 1 at 1 mg scale. The biotin moiety in these experiments can be replaced with a drug and biotin serves as a model for conjugation. PEG2 is a linker, and the maleimide moiety reacts with the cysteine. "TCEP" represents tris-(2-carboxyethyl)phosphine. Indicated molar excess is with respect to moles antibody unless specified otherwise.

Figure 9 shows a Western blot of reducing protein gels on samples from conjugation protocol D. The variant and native proteins were purified by Protein A, dialyzed into PBS 1X, 10 mM EDTA, pH 7.2. The numbers 1-21 correspond to ND1-21, respectively. M is a molecular weight marker. C is a control antibody conjugated with biotin at lysine residues in both the light and heavy chains. The samples were first resolved by SDS-PAGE on NuPage 10% gels. The blots with transferred protein were blocked in TBST 2% BSA, incubated with avidin-HRP, and visualized with a chromogenic substrate for HRP. HC are the protein bands corresponding to the antibody heavy chains, and LC are the protein bands corresponding to the antibody light chains. There is pronounced conjugation on the heavy chain of a number of variants (especially ND2, 3, 4, 6, 7, 8, 9, 10, 11, 12, 18, 19), while there is no detectable conjugation on the HC of ND1 (native). There is some conjugation on the LC of all variants, including ND1.

Figure 10 shows Coomassie-stained non-reducing protein gels on samples from conjugation protocol D. All variant and native proteins were purified by Protein A, dialyzed into PBS 1X, 10 mM EDTA, pH 7.2, and analyzed on a 10% homogeneous SDS-PAGE under reducing and non-reducing conditions. SeeBlue Plus2 (Invitrogen) molecular weight standards (in kDa as schematically indicated) were used. 3 µg of each sample was loaded and the gel was stained using SimplyBlue™ SafeStain (Invitrogen). The numbers 1-21 correspond to ND1-21, respectively. M is a molecular weight marker. Most conjugated variants show eletrophoretic mobility pattern similar to native ND 1. Several conjugated variants contain some apparent dimer (ND 3, 5, 13, 15, 17, 18, 19), and one variant shows some apparent fragmentation (ND 20).

Figure 11 contains results of intact mass and peptide mapping of the ND variants, conjugated by protocol D. Variants ND4, 7, 10, 12, 18 have close to or greater than 90% conjugation at the engineered cysteines, and show no or little oligomerization. DAR is Drug to Antibody molar Ratio of the conjugated sample.

### 8.2 Example 2. Expression and characterization of double-variant cysteine engineered antibodies

Based on select variants, eleven double variants were constructed as depicted Figure 12, numbered DM1 to DM 11. Ten of the double variants were based on select variants, ND4, ND7, ND10, ND12, ND 18, and another double variant was based on ND10 and ND19.

Figure 13 provides data on the expression of the double variants. All double variants expressed well by transient transfection. Only four of these double variants, DM8, DM9, DM10, DM11 are expressed and purified at greater than 93% monomer levels (determined by size exclusion chromatography with ultraviolet detection (SEC-UV)). These four double variants have MW and Rh similar to the native ND 1 (data not shown).

The double ND variants were analyzed on non-reducing SDS-PAGE as seen in Figure 14. "C" is native ND1 used as a control. "M" is a molecular weight standard. The numbers 1-10 correspond to DM1-10. The SDS-PAGE results are consistent with the SEC-UV results. Variants DM1-7 contain a large percent of aggregates. Variant DM4 has the least amount of monomer, and DM8 contains the largest percent of monomer.

Figure 15 shows results from the DSC analysis of double variants. DSC was performed as described above except that the His buffer was 10 mM. The four mostly monomeric (>93%) double variants show DSC profiles very similar to native and to the single variant ND10.

Figure 16 provides results of Western blotting and Coomassie stained gels analyzing the conjugation of double variants. Conjugation of double variants DM8 (ND 4 and ND10) and DM11 (ND10 and ND19) using protocol D (see Figure 6) a 10:1 biotin-maleimide:antibody ratio and a 20:1 biotin-maleimide:antibody ratios.

The double variants, conjugated as described in Fig. 16, were subjected to intact mass and peptide mapping, and that data is provided in Figure 17. Two of double variants. DM8 (ND4 and ND10) and DM11 (ND10 and ND 19) have close to or greater than 90% conjugation at the engineered cysteines. DAR is Drug to Antibody molar Ratio of the conjugated sample.

### 8.3 Example 3. Modified conjugation protocol

An experiment was conducted in scaling up the conjugation reaction. Figure 18 shows the results of conjugation performed on single and double ND variants. Conjugation protocol D (Figure 8) was used except for the employment of a more extensive overnight dialysis after the TCEP treatment, and the conjugation itself was conducted for 1 hr at room temperature. The drug:mAb molar ratio was 10:1 1 for single variants and 20:1 for double variants.

Figure 19 shows results of peptide mapping analysis of single and double variants conjugated samples as shown on the previous Figure 18. This table summarizes the conjugation efficiencies at the engineered cysteines, as well as the overall drug-to-antibody ratio (DAR). DM9" is a second preparation of DM9 using the same mutations.

### 8.4 Example 4. Scaled-up conjugation

Figure 20 shows results of a scaled-up conjugation experiment. Conjugation was performed at (a) 2.5, (b) 5, (c) 10, (d) 20, and (e) 40 mg/ml of antibody. The conjugation protocol as described for Fig. 18 was used. The drug:antibody molar ratio was 10:1. In the protein gels, "C" is native ND 1.

Figure 21 shows results of peptide mapping of the scaled-up conjugation. The conjugated samples from the previous Figure 20 were subjected to peptide mapping analysis. The conjugation efficiency results are shown. The concentrations correspond to the antibody concentrations during TCEP pretreatment, dhAA-stimulated refolding, and conjugation.

### 8.5 Example 5. Expression and characterization of triple-variant cysteine engineered antibodies

Four triple variants were developed based on select variants. Figure 22 illustrates the residues selected for mutation in the triple variants, termed T1 to T4.

Figure 23 provides expression and monomer levels of the 4 triple variants. Protein was purified protein from 0.5 L culture. Monomer levels were determined on SEC-HPLC. For comparison, native antibody ND1 is 99% monomeric.

Figure 24 contains results of conjugation of triple variants with biotin maleimide. The TCEP/dhAA protocol was used with conjugation for 1 hr at room temperature and 1:20 antibody:drug molar ratio. Two different protein preps of T1 were conjugated. The native ND1 and the double variant DM11 were included as controls for conjugation specificity and efficiency.

Figure 25 shows results of peptide mapping of the triple variants. The conjugated triple variant samples from the previous Figure 24 were subjected to peptide mapping analysis. The conjugation efficiencies at the engineered cysteines are presented.

Figure 26 shows DSC analysis of the triple variants, conducted as described above with 10 mM His buffer. T 1 shows a few degrees lower melting temperature for the first transition, CH2. T2 shows some precipitation at 85°C. T3 precipitates at high temperatures. T4 is nearly identical to native.

## Claims

1. An Fc region of an antibody, wherein the Fc region comprises a substitution at position 289, wherein the substitution comprises a substitution to an amino acid chosen from cysteine, lysine, tyrosine, histidine, selenocysteine, and selenomethionine, and wherein a heterologous agent is coupled to the substutited amino acid.

2. The Fc region of claim 1, wherein the Fc region further comprises a substitution of one or more positions chosen from 239, 282, 297, 312, 324, 330, 335,337, 339, 356, 359, 361,383,384, 398, 400, 440, 422, and 442, wherein the substitution comprises a substitution to an amino acid chosen from cysteine, lysine, tyrosine, histidine, selenocysteine, and selenomethionine.

3. The Fc region of claim 2, wherein the Fc region comprises one or more of the following groups of substitutions:
a) 289 and 440;
b) 330 and 440;
c) 339 and 440;
d) 359 and 440;
e) 289 and 359;
f) 330 and 359;
g) 339 and 359;
h) 289 and 339;
i) 330 and 339;
j) 289 and 330; and
k) 339 and 442.

4. The Fc region of claim 2, wherein the Fc region comprises one or more of the following groups of substitutions:
a) 289, 339, and 442;
b) 289, 330, and 339;
c) 330, 339, and 442; and
d) 289, 330, and 442.

5. The Fc region of claim 1, 2, 3 or 4, wherein the heterologous agent is chosen from a cytotoxic agent, a chemotherapeutic agent, a toxin, a radionuclide, DNA, RNA, siRNA, microRNA, a peptidonucleic acid, a non-natural amino acid, a peptide, an enzyme, a fluorescent tag, or biotin.

6. The Fc region of claim 1-5, wherein the Fc region further comprises a substitution at one or more positions chosen from 234, 235 and 331.

7. The Fc region of claim 5, wherein the substitions are chosen from 234F, 235F, 235Y, and 331S.

8. The Fc region of claim 1-7, wherein the Fc region is chosen from an IgG1, IgG2, IgG3, or an IgG4 isotype.

9. The Fc region of claim 1-8, wherein the substitution comprises cysteine.

10. An Fc fusion protein comprising the Fc region of claim 1-9.

11. An antibody or antigen binding fragment thereof comprising the Fc region of claim 1-9.

12. The antibody or antigen binding fragment thereof of claim 11, wherein said antibody further comprises a substitution to a cysteine residue at a position chosen from positions 131, 132, 133, 134, 135, 136, 137, 138, and 139 of the CH1 domain of the antibody.

13. A composition comprising the the Fc region of claim 1-9.

14. A pharmaceutical composition comprising the antibody or antigen binding fragment thereof of claim 11 or 12.


SEQUENCE LISTING

<110>  MedImmune LLC

<120>  Engineered Antibodies for Site-Specific Conjugation

<130>  MED0455

<150>  US 61/219,255
<151>  2009-06-22

<160>  24

<170>  PatentIn version 3.5

<210>  1
<211>  329
<212>  PRT
<213>  Homo sapiens

<400>  1

Ser Thr Lys Gly Pro Ser Val Phe Pro Leu Ala Pro Ser Ser Lys Ser
1               5                   10                  15

Thr Ser Gly Gly Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu

50                  55                  60

Ser Ser Val Val Thr Val Pro Ser Ser Ser Leu Gly Thr Gln Thr Tyr
65                  70                  75                  80

Ile Cys Asn Val Asn His Lys Pro Ser Asn Thr Lys Val Asp Lys Arg
                85                  90                  95

Val Glu Pro Lys Ser Cys Asp Lys Thr His Thr Cys Pro Pro Cys Pro
            100                 105                 110

Ala Pro Glu Leu Leu Gly Gly Pro Ser Val Phe Leu Phe Pro Pro Lys
        115                 120                 125

Pro Lys Asp Thr Leu Met Ile Ser Arg Thr Pro Glu Val Thr Cys Val
    130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                165                 170                 175

Gln Tyr Asn Ser Thr Tyr Arg Val Val Ser Val Leu Thr Val Leu His
            180                 185                 190

Gln Asp Trp Leu Asn Gly Lys Glu Tyr Lys Cys Lys Val Ser Asn Lys
        195                 200                 205

Ala Leu Pro Ala Pro Ile Glu Lys Thr Ile Ser Lys Ala Lys Gly Gln
    210                 215                 220

Pro Arg Glu Pro Gln Val Tyr Thr Leu Pro Pro Ser Arg Glu Glu Met
225                 230                 235                 240

Thr Lys Asn Gln Val Ser Leu Thr Cys Leu Val Lys Gly Phe Tyr Pro
                245                 250                 255

Ser Asp Ile Ala Val Glu Trp Glu Ser Asn Gly Gln Pro Glu Asn Asn
            260                 265                 270

Tyr Lys Thr Thr Pro Pro Val Leu Asp Ser Asp Gly Ser Phe Phe Leu
        275                 280                 285

Tyr Ser Lys Leu Thr Val Asp Lys Ser Arg Trp Gln Gln Gly Asn Val
    290                 295                 300

Phe Ser Cys Ser Val Met His Glu Ala Leu His Asn His Tyr Thr Gln
305                 310                 315                 320

Lys Ser Leu Ser Leu Ser Pro Gly Lys
                325

<210>  2
<211>  330
<212>  PRT
<213>  Artificial Sequence

<220>
<223>  ND2-Fc-Ser239Cys

<400>  2

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Cys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  3
<211>  330
<212>  PRT
<213>  Artificial Sequence

<220>
<223>  ND3-Fc-Val282Cys

<400>  3

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Cys Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  4
<211>  330
<212>  PRT
<213>  Artificial Sequence

<220>
<223>  ND4-FC-Thr289Cys

<400>  4

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Cys Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn

260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  5
<211>  330
<212>  PRT
<213>  Artificial Sequence

<220>
<223>  ND5-FC-Asp312Cys

<400>  5

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Cys Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 6
<211> 330
<212> PRT
<213> Artificial Sequence

<220>
<223> ND6-FC-Ser324Cys

<400> 6

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Cys Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  7
<211>  330
<212>  PRT

<213>  Artificial Sequence

<220>
<223>  ND7-FC-Ala330Cys

<400>  7

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Cys Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 8
<211> 330
<212> PRT
<213> Artificial Sequence

<220>
<223> ND8-FC-Thr335Cys

<400> 8

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Cys Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  9
<211>  330
<212>  PRT
<213>  Artificial Sequence

<220>
<223>  ND9-FC-Ser337Cys

<400>  9

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Cys Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  10

<211>  330
<212>  PRT
<213>  Artificial Sequence

<220>
<223>  ND10-FC-Ala339Cys

<400>  10

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Cys Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr

245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  11
<211>  330
<212>  PRT
<213>  Artificial Sequence

<220>
<223>  ND11-Fc-Glu356Cys

<400>  11

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Cys Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  12
<211>  330
<212>  PRT
<213>  Artificial Sequence

<220>
<223>  ND12-FC-Thr359Cys

<400>  12

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Cys Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  13

<211>  330
<212>  PRT
<213>  Artificial Sequence

<220>
<223>  ND13-FC-Asn361Cys

<400>  13

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Cys Gln Val Ser Leu Thr Cys Leu Val Lys Gly Phe Tyr

245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  14
<211>  330
<212>  PRT
<213>  Artificial Sequence

<220>
<223>  ND14-FC-Ser383Cys

<400>  14

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Cys Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  15
<211>  330
<212>  PRT
<213>  Artificial Sequence

<220>
<223>  ND15-Fc-Asn384Cys

<400>  15

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Cys Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  16

<211>  330
<212>  PRT
<213>  Artificial Sequence

<220>
<223>  ND16-FC-Leu398Cys

<400>  16

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr

245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Cys Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  17
<211>  330
<212>  PRT
<213>  Artificial Sequence

<220>
<223>  ND17-FC-Val422Cys

<400>  17

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp

145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Cys Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  18
<211>  330
<212>  PRT
<213>  Artificial Sequence

<220>
<223>  ND18-FC-Ser440Cys

<400>  18

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Cys Leu Ser Leu Ser Pro Gly Lys
                325                 330

<210>  19
<211>  330
<212>  PRT
<213>  Artificial Sequence

<220>
<223>  ND19-FC-Ser442Cys

<400>  19

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Cys Leu Ser Pro Gly Lys
                325                 330

<210> 20
<211> 330
<212> PRT
<213> Artificial Sequence

<220>
<223> ND20-Fc-Asn297Cys

<400> 20

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Cys Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  21
<211>  330
<212>  PRT
<213>  Artificial Sequence

<220>
<223>  ND21-Fc-Ser400Cys

<400>  21

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Cys Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  22
<211>  326
<212>  PRT
<213>  Homo sapiens

<400>  22

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
        260                 265                 270

Thr Pro Pro Met Leu Asp Ser Asp Gly Ser Phe Phe Leu Tyr Ser Lys
    275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  23
<211>  377
<212>  PRT
<213>  Homo sapiens

<400>  23

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210> 24
<211> 327
<212> PRT
<213> Homo sapiens

<400> 24

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325